# EUROPEAN PATENT APPLICATION

(11) **EP 1 333 029 A1**
(43) Date of publication of application: **06.08.2003**
(21) Application number: 01993391.0
(22) Date of filing: 07.11.2001
(51) Int. Cl.: C07D 239/26, C07D 239/30, C07D 239/34, C07D 413/04, C07D 405/04, A01N 43/54, A01N 43/84

(54) **PYRIMIDINE DERIVATIVES AND HERBICIDES CONTAINING THE SAME**

(30) Priority: 08.11.2000 JP 2000340413; 05.11.2001 JP 2001339893
(71) Applicant: Sumitomo Chemical Takeda Agro Company, Limited, Tokyo 103-0027 (JP)
(72) Inventor: KURAGANO, Takashi, Tsukuba-shi, Ibaraki 305-0821 (JP); TANAKA, Yasushi, Tsukuba-shi, Ibaraki 305-0821 (JP)
(74) Representative: Teipel, Stephan, Dr.
(86) International application number: JP0109754
(87) International publication number: WO02038550

(57) **Abstract**

The present invention provides a pyrimidine derivative represented by the formula: 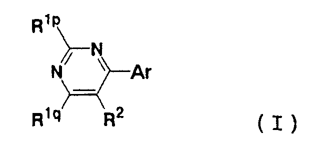 wherein R^{1p} and R^{1q} are the same or different, and each represents (1)hydrogen, (2)halogen, (3) a C₁₋₆alkyl group which may be substituted or (4) a C₁₋₆alkoxy group, and so on,
R² is halogen, a C₁₋₆alkyl group, cyano group, and so on, Ar is a phenyl group which may be substituted or is a condensed hetero ring which may be substituted, which has excellent selective herbicidal activity, and a herbicide containing the derivative.

## Description

### Technical Field

This invention relates to a pyrimidine derivative and a herbicide containing the pyrimidine derivative. The pyrimidine derivative in this invention has excellent herbicidal activity against weeds in paddy fields or plow lands without inducing any harm in crop plants, such as rice plant, wheat, barley, maize, cotton, soybeans and other plants, and is useful as an excellent selective herbicide to be used in paddy fields or plow lands.

### Background Art

Heretofore, several pyrimidine derivatives have ever been reported. However, the established herbicides containing pyrimidine derivatives are not fully satisfactory due to insufficient herbicidal effects on weeds, phytotoxicity on crop plants , toxicity to mammals, fish and shellfish, risk of environmental pollution and so on, and the development of selective herbicides which are more improved in the above aspects has been awaited in earnest.

### Disclosure of Invention

Taking these actual situations into consideration, an object of this invention is to provide herbicides containing pyrimidine derivatives which show excellent selective herbicidal activity.

The present inventors have made intensive efforts in order to develop selective herbicides which have excellent herbicidal activity and are not phytotoxic on crops. As a result, they have found that the pyrimidine derivatives represented by the formula (I) or salts thereof have a strong herbicidal activity with remarkably reduced phytotoxicity on crop plants such as rice plant, wheat, barley, maize, cotton, soybeans and so on, and show high selective herbicidal effect. The present inventors further carried out intensive researches based on these findings and have completed the present invention. No report has been reported regarding the pyrimidine derivative of the present invention and its herbicidal activity, thus the compound represented by the formula (I) is novel.

Thus, the present invention provides:
[1] A pyrimidine derivative or a salt thereof represented by Formula (I): 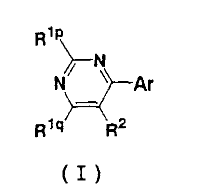 [wherein each of R^{1p} and R^{1q} may be the same or differently (1) hydrogen, (2) halogen, (3) a C₁₋₆alkyl group which may be substituted by a substituent or substituents selected from the group consisting of halogen, a C₁₋₆alkoxy group, a C₂₋₆alkenyloxy group, a C₂₋₆alkynyloxy group, C₁₋₆alkylthio group, C₂₋₆alkenylthio group, a C₂₋₆alkynylthio group, a C₁₋₆haloalkoxy group, a C₂₋₆haloalkenyloxy group, a C₁₋₆haloalkylthio group, a C₂₋₆haloalkenylthio group, hydroxy group, mercapto group and cyano group, the number of the substituent being a number falling within substitutable number, preferably from 1 to 6, (4) a C₁₋₆alkoxy group, (5) a C₂₋₆alkenyloxy group, (6) a C₂₋₆alkynyloxy group, (7) a C₁₋₆haloalkoxy group, (8) a C₂₋₆haloalkenyloxy group , (9) a C₁₋₆alkylthio group, (10) a C₂₋₆alkenylthio group, (11) a C₂₋₆alkynylthio group, (12) a C₁₋₆haloalkylthio group, (13) a C₂₋₆haloalkenylthio group, (14) an amino group which may be mono- or di-substituted by a substituent or substituents selected from a C₁₋₆alkyl group, a C₂₋₆alkenyl group, a C₂₋₆alkynyl group, a C₁₋₇ alkanoyl group and a cyclic amino which may contain one or two atoms selected from oxygen atom, sulfur atom and nitrogen atom, (15) a C₂₋₇alkoxycarbonyl group, (16) a C₃₋₇alkenyloxycarbonyl group, (17) a C₃₋₇ haloalkenyloxycarbonyl group, (18) a C₃₋₇alkynyloxycarbonyl group, (19) carbamoyl group which may be substituted on its nitrogen atom by one or two C₁₋₄alkyl groups, (20) thiocarbamoyl group, (21) cyano group, or (22) formyl group, R² is halogen, a C₁₋₆alkyl group, a carbamoyl group which may be substituted on its nitrogen atom by one or two C₁₋₄alkyl groups, thiocarbamoyl group, cyano group or formyl group,
   Ar is represented by one of the formulas: 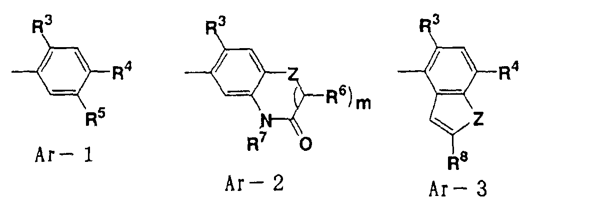 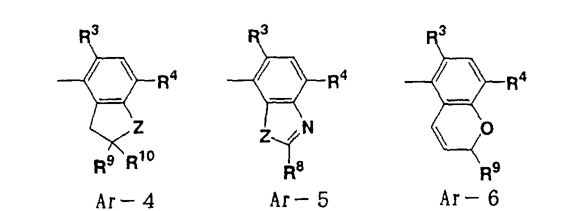 (wherein R³ is hydrogen, halogen, a C₁₋₆alkyl group, a C₁₋₆haloalkyl group, a C₁₋₆alkoxy group or a C₁₋₆haloalkoxy group,
   R⁴ is (1) halogen, (2) nitro group, (3) cyano group, (4) carbamoyl group, (5) thiocarbamoyl group, (6) a hydroxyC₁₋₄alkyl group, (7) a C₁₋₄haloalkyl group, (8) a C₁₋₆alkoxy-C₁₋₄alkyl group, (9) a C₂₋₇alkoxycarbonyl group, (10) a C₃₋₇alkenyloxycarbonyl group, (11) a C₃₋₇alkynyloxycarbonyl group, (12) a C₁₋₆alkoxy group, (13) a C₂₋₆alkenyloxy group, (14) a C₂₋₆alkynyloxy group or (15) a C₇₋₁₂aralkyloxy group which may be substituted by halogen, a C₁₋₆alkyl group, a C₁₋₆haloalkyl group, a C₁₋₆alkoxy group, a C₁₋₆haloalkoxy group, a C₂₋₆alkenyloxy group, a C₂₋₆alkynyloxy group, a C₂₋₇alkoxycarbonyl group, a C₃₋₇alkenyloxycarbonyl group, a C₃₋₇alkynyloxycarbonyl group, a C₂₋₇alkoxycarbonyl-C₁₋₄alkoxy group, a C₃₋₇alkenyloxycarbonyl-C₁₋₄alkoxy group or a C₃₋₇alkynyloxycarbonyl-C₁₋₄alkoxy group, the number of the substituents being from one to the maximum substitutable number,
   R⁵ is hydrogen, cyano group, a C₁₋₆alkyl group, a C₃₋₆cycloalkyl group, hydroxy group, mercapto group, a C₁₋₆alkoxy group, a C₁₋₆alkoxy-C₁₋₄alkoxy group, a C₃₋₆cycloalkyloxy group, a C₁₋₆haloalkoxy group, a C₂₋₆alkenyloxy group, a C₂₋₆haloalkenyloxy group, a C₂₋₆alkynyloxy group, a C₆₋₁₀aryloxy group, a C₇₋₁₂aralkyloxy group, a C₁₋₆alkythio group, a C₁₋₆alkoxyC₁₋₄alkylthio group, a C₃₋₆cycloalkylthio group, a C₁₋₆haloalkylthio group, a C₂₋₆alkenylthio group, a C₂₋₆haloalkenylthio group, a C₂₋₆alkynylthio group, a C₆₋₁₀arylthio group, a C₇₋₁₂aralkylthio group, a C₁₋₆alkylsulfonyl group, a C₃₋₆cycloalkylsulfonyl group, a C₁₋₆haloalkylsulfonyl group, a C₂₋₆alkenylsulfonyl group, a C₂₋₆haloalkenylsulfonyl group, a C₂₋₆alkynylsulfonyl group, or a cyclic amino group which may contain one or two oxygen atoms, sulfur atoms and nitrogen atoms, or alternatively
   R⁵ is one of the groups represented by formulas: 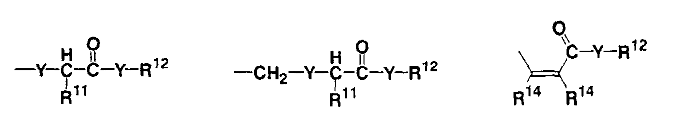 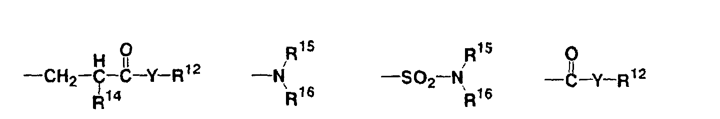 (wherein Y is oxygen, sulfur or -N-R¹³,
   R¹¹ is hydrogen or a C₁₋₆alkyl group,
   R¹² is hydrogen, a C₁₋₆alkyl group, a C₁₋₆haloalkyl group, a C₃₋₆cycloalkyl group, a C₂₋₆alkenyl group, C₂₋₆haloalkenyl group, a C₂₋₆alkynyl group, a C₆₋₁₀aryl group, a C₇₋₁₂aralkyl group, a C₁₋₆alkoxy-C₁₋₄alkyl group, a C₂₋₆alkenyloxy-C₁₋₄alkyl group, a C₂₋₆ alkynyloxy-C₁₋₄alkyl group, a C₃₋₆cycloalkoxy-C₁₋₄alkyl group, a C₂₋₇alkoxycarbonyl-C₁₋₄alkyl group, a C₃₋₇alkenyoxycarbonyl-C₁₋₄alkyl group, a C₃₋₇alkynyloxycarbonyl-C₁₋₄alkyl group, a C₄₋₇cycloalkoxycarbonyl-C₁₋₄alkyl group, a C₂₋₇haloalkoxycarbonyl-C₁₋₄alkyl group, a C₃₋₇haloalkenyloxycarbonyl-C₁₋₄alkyl group or a C₇₋₁₂aralkyloxycarbonyl-C₁₋₄alkyl group,
   R¹³ is hydrogen, a C₁₋₆alkyl group, a C₁₋₆alkoxy-C₁₋₄alkyl group, a C₁₋₇alkanoyl group, a C₇₋₁₁arylcarbonyl group, a C₂₋₇alkoxycarbonyl group, a C₂₋₇haloalkoxycarbonyl group, or a C₃₋₇haloalkenyloxycarbonyl group,
   R¹⁴ is hydrogen, halogen or a C₁₋₆alkyl group,
   each of R¹⁵ and R¹⁶ is same or differently hydrogen, a C₁₋₆alkyl group, a C₂₋₆alkenyl group, a C₂₋₆alkynyl group, a C₃₋₆cycloalkyl group, a C₁₋₆alkoxy-C₁₋₄alkyl group, a C₆₋₁₂aralkyl group, a C₁₋₄alkyl group substituted by a 5-or 6-membered heterocyclic ring which may contain one or two atoms selected from nitrogen, oxygen and sulfur, a C₁₋₇alkanoyl group, a C₆₋₁₂arylcarbonyl group, a C₂₋₇haloalkylcarbonyl group, a C₂₋₇alkoxycarbonyl group, a C₃₋₇alkenyloxycarbonyl group, a C₃₋₇alkynyloxycarbonyl group, a C₄₋₇cycloalkyloxycarbonyl group, a C₂₋₇haloalkoxycarbonyl group, a C₁₋₆alkylsulfonyl group, a C₂₋₆alkenylsulfonyl group, a C₂₋₆haloalkenylsulfonyl group, a C₂₋₆alkynylsulfonyl group, a C₃₋₆cycloalkylsulfonyl group, a C₁₋₆haloalkylsulfonyl group, a C₆₋₁₀arylsulfonyl group, a C₇₋₁₂aralkylsulfonyl group or alternatively each of R¹⁵ and R¹⁶ is same or differently a group represented by formula: 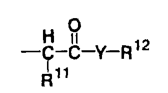 (wherein each of R¹¹ and R¹² has the same meaning as mentioned above)),
   R⁶ is hydrogen or a C₁₋₆alkyl group, and m represents 0 or 1,
   R⁷ is hydrogen, a C₁₋₆alkyl group, a C₁₋₆haloalkyl group, a C₂₋₆alkenyl group, a C₂₋₆alkynyl group, a C₁₋₆alkoxy-C₁₋₄alkyl group, a C₁₋₇alkanoyl group, a C₇₋₁₁arylcarbonyl group, a C₂₋₇alkoxycarbonyl group, a C₃₋₇alkenyloxycarbonyl group, a C₃₋₇ alkynyloxycarbonyl group, a C₄₋₇cycloalkyloxycarbonyl group, a C₂₋₇alkoxycarbonyl-C₁₋₄alkyl group, a C₃₋₇alkenyloxycarbonyl-C₁₋₄alkyl group, a C₃₋₇haloalkenyoxycarbonyl-C₁₋₄alkyl group or a C₃₋₇alkynyloxycarbonyl-C₁₋₄alkyl group,
   R⁸ is hydrogen, halogen, a C₁₋₆ alkyl group, a C₁₋₆alkoxy group, a C₁₋₆haloalkoxy group, a C₂₋₆ alkenyloxy group, a C₂₋₆alkynyloxy group, a C₁₋₆alkylthio group, a C₁₋₆haloalkylthio group, a C₂₋₆alkenylthio group, a C₂₋₆alkynylthio group, a C₁₋₆haloalkyl group, a C₁₋₆alkoxy -C₁₋₄alkyl group, a C₁₋₆alkylthio-C₁₋₄alkyl group or a C₁₋₆alkylsulfonyl-C₁₋₄alkyl group,
   R⁹ is hydrogen or a C₁₋₆alkyl group,
   R¹⁰ is a C₁₋₆alkyl group,
   Z is oxygen atom or sulfur atom)].
[2] A pyrimidine derivative or a salt thereof as described in the above (1), which is represented by formula (Ia): 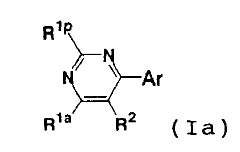 (wherein R^{1p}, R² and Ar have each the same meaning as described above, and R^{1a} is a C₁₋₆alkyl group which is substituted by the substituent or the substituents described above).
[3] A pyrimidine derivative or a salt thereof as described in the above (1), which is represented by the formula (Ia): 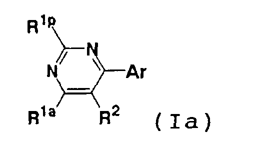 (wherein R^{1p}, R² and Ar have each the same meaning as described above, and R^{1a} is a C₁₋₆alkyl group).
[4] A pyrimidine derivative or a salt thereof as described in any one of the above (1) to (3), wherein Ar is the group represented by formula Ar-1 in the formula (I).
[5] A pyrimidine derivative or a salt thereof as described in any one of the above (1) to (3), wherein Ar is the group represented by formula Ar-2 in the formula (I).
[6] A pyrimidine derivative or a salt thereof as described in any one of the above (1) to (3), wherein Ar is the group represented by formula Ar-3 in the formula (I).
[7] A pyrimidine derivative or a salt thereof as described in any one of the above (1) to (3), wherein Ar is the group represented by formula Ar-4 in the formula (I).
[8] A pyrimidine derivative or a salt thereof as described in any one of the above (1) to (3), wherein Ar is the group represented by formula Ar-5 in the formula (I).
[9] A pyrimidine derivatives or a salt thereof as described in any one of the above (1) to (3), wherein Ar is the group represented by formula Ar-6 in the formula (I).
[10] A herbicide which comprises a pyrimidine derivative or a salt thereof as described in any one of the above (1) to (9).

### Best Mode for Carrying Out the Invention

In the compound represented by the formula (I), each of R^{1p} and R^{1q} independently represents (1) hydrogen, (2) halogen (for example, fluorine, chlorine, bromine, iodine), (3) a straight or branched C₁₋₆alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl, which may be substituted by a substituent or substituents including halogen (for example, fluorine, chlorine, bromine, iodine), a C₁₋₆alkoxy group (for example, methoxy, ethoxy, n-propoxy, isopropoxy, n-butoxy, isobutoxy and so on), a C₂₋₆alkenyloxy group (for example, allyloxy, 1-buten-3-yloxy, 3-buten-1-yloxy and so on), a C₂₋₆alkynyloxy group (for example, propargyloxy, 2-butyn-1-yloxy, 3-butyn-2-yloxy and so on), a C₁₋₆alkylthio group (for example, methylthio, ethylthio, n-propylthio, isopropylthio, sec-butylthio, n-pentylthio and so on), a C₂₋₆alkenylthio group (for example, allylthio, 1-buten-3-ylthio, 3-buten-2-ylthio), a C₂₋₆alkynylthio group (for example, propargylthio, 2-butyn-1-ylthio, 3-butyn-2-ylthio and so on), a C₁₋₆haloalkoxy group (for example, trifluoromethoxy and so on), a C₂₋₆haloalkenyloxy group (for example, 2-chloro-2-propen-1-yloxy), a C₁₋₆haloalkylthio group (for example, trifluoromethylthio and so on), a C₂₋₆haloalkenylthio group (for example, 2-chloro-2-propen-1-ylthio and so on), hydroxy group, mercapto group, and cyano group, the number of the substituent being a number being within the range of the substitutable number, preferably 1 to 6, (4) a C₁₋₆alkoxy group (for example, methoxy, ethoxy, n-propoxy, isopropoxy, n-butoxy, isobutoxy), (5) a C₂₋₆alkenyloxy group (for example, allyloxy, 1-buten-3-yloxy, 3-buten-1-yloxy), (6) a C₂₋₆alkynyloxy group (for example, propargyloxy, 2-butyn-1-yloxy, 3-butyn-2-yloxy), (7) a C₁₋₆haloalkoxy group (for example, trifluoromethoxy), (8) a C₂₋₆haloalkenyloxy group (for example, 2-chloro-2-propen-1-yloxy), (9) a C₁₋₆alkylthio group (for example, methylthio, ethylthio, n-propylthio, isopropylthio, sec-butylthio, n-pentylthio), (10) a C₂₋₆alkenylthio group (for example, allylthio, 1-buten-3-ylthio, 3-buten-1-ylthio), (11) a C₂₋₆alkynylthio group (for example, propargylthio, 2-butyn-1-ylthio, 3-butyn-2-ylthio), (12) a C₁₋₆haloalkylthio group (for example, trifluoromethylthio), (13) a C₂₋₆haloalkenylthio group (for example, 2-chloro-2-propen-1-ylthio), (14) amino group which may be substituted, wherein the substitution may be mono- or di- substitution by a substituent or substituents selected from C₁₋₆alkyl group (for example, methyl, ethyl, n-propyl, isopropyl, n-butyl, isobutyl, sec-butyl, tert-butyl, n-pentyl, sec-pentyl, isopentyl, neopentyl, n-hexyl, isohexyl), a C₂₋₆alkenyl group (for example, allyl, 1-buten-3-yl, 3-buten-1-yl), a C₂₋₆alkynyl group (for example, propargyl, 2-butyn-1-yl, 3-butyn-2-yl), a C₁₋₇alkanoyl group (for example, formyl, acetyl, propionyl, n-butyryl) ,or cyclic amino group which may contain one or two atoms selected from oxygen atom, sulfur atom and nitrogen atom (for example, morpholino, pyrrolidino, piperidino), (15) a C₂₋₇alkoxycarbonyl group (for example, methoxycarbonyl, ethoxycarbonyl, n-propoxycarbonyl, isopropoxycarbonyl, n-butoxycarbonyl, isobutoxycarbonyl), (16) a C₃₋₇alkenyloxycarbonyl group (for example, allyloxycarbonyl, 1-buten-3-yloxycarbonyl, 3-buten-1-yloxycarbonyl), (17) a C₃₋₇haloalkenyloxycarbonyl group (for example, 2-chloro-2-propen-1-yloxycarbonyl), (18)a C₃₋₇alkynyloxycarbonyl group (for example, propargyloxycarbonyl, 2-butyn-1-yloxycarbonyl, 3-butyn-2-yloxycarbonyl), (19) a carbamoyl group which may be mono- or di-substituted on its nitrogen atom, by one or two C₁₋₄alkyl groups (for example, methyl, ethyl, n-propyl, isopropyl, n-butyl, isobutyl), (20) thiocarbamoyl group, (21) cyano group, or (22) formyl group.
R² is halogen (for example, fluorine, chlorine, bromine, iodine), a C₁₋₄alkyl group (for example, methyl, ethyl, n-propyl, isopropyl, n-butyl, isobutyl, sec-butyl); a carbamoyl group which may be mono- or di-substituted on its nitrogen atom by one or two a C₁₋₄alkyl groups (for example, methyl, ethyl, n-propyl, isopropyl, n-butyl, isobutyl); thiocarbamoyl group; cyano group; or formyl group.
R³ is hydrogen, halogen (for example, fluorine, chlorine, bromine, iodine), a C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl), a C₁₋₆haloalkyl group (for example, chloromethyl, 2-chloroethyl, trifluoromethyl), a C₁₋₆alkoxy group (for example, methoxy, ethoxy, n-propoxy, isopropoxy, n-butoxy, isobutoxy, sec-butoxy, tert-butoxy, n-pentyloxy), or a C₁₋₆haloalkoxy group (for example, trifluoromethoxy).
R⁴ is (1) halogen (for example, fluorine, chlorine, bromine, iodine), (2) nitro group, (3) cyano group, (4) carbamoyl group, (5) thiocarbamoyl group, (6) a hydroxy-C₁₋₆alkyl group (for example, hydroxymethyl, 1-(hydroxy)ethyl), (7) a C₁₋₄haloalkyl group (for example, trifluoromethyl), (8) a C₁₋₆alkoxy-C₁₋₄alkyl group (for example, methoxymethyl, ethoxymethyl, n-propoxymethyl, isopropoxymethyl), (9) a C₂₋₇alkoxycarbonyl group (for example, methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl, n-pentyloxycarbonyl), (10) a C₃₋₇alkenyloxycarbonyl group (for example, allyloxycarbonyl, 1-buten-3-yloxycarbonyl, 3-buten-1-yloxycarbonyl), (11) a C₃₋₇alkynyloxycarbonyl group (for example, propargyloxycarbonyl, 2-butyn-1-yloxycarbonyl, 3-butyn-2-yloxycarbonyl), (12) a C₁₋₆alkoxy group (for example, methoxy, ethoxy, n-propoxy, isopropoxy, n-butoxy, isobutoxy, sec-butoxy), (13) a C₂₋₆alkenyloxy group (for example, allyloxy, 1-buten-3-yloxy, 3-buten-1-yloxy), (14) a C₂₋₆alkynyloxy group (for example, propargyloxy, 2-butyn-1-yloxy, 3-butyn-2-yloxy), (15) a C₇₋₁₂aralkyloxy group which may be substituted, and the number of the substitutes is one to substitutable maximum number, preferably 1 to 3 wherein the substituent includes halogen (for example, fluorine, chlorine, bromine, iodine), a C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl), a C₁₋₆haloalkyl group (for example, chloromethyl, bromomethyl 1-chloroethyl, trifluoromethyl),
a C₁₋₆alkoxy group (for example, methoxy, ethoxy, n-propoxy, isopropoxy, n-butoxy, isobutoxy, sec-butoxy, tert-butoxy, n-pentyloxy), a C₁₋₆haloalkoxy group (for example, trifluoromethoxy), a C₂₋₆alkenyloxy group (for example, allyloxy, 1-buten-3-yloxy, 3-buten-1-yloxy), a C₂₋₆alkynyloxy group (for example, propargyloxy, 2-butyn-1-yloxy, 3-butyn-2-yloxy), a C₂₋₇alkoxycarbonyl group (for example, methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl, n-pentyloxycarbonyl), a C₃₋₇alkenyloxycarbonyl group (for example, allyloxycarbonyl, 1-buten-3-yloxycarbonyl, 3-buten-1-yloxycarbonyl),
a C₃₋₇alkynyloxycarbonyl group (for example, propargyloxycarbonyl, 2-butyn-1-yloxycarbonyl, 3-butyn-1-yloxycarbonyl),
a C₂₋₆alkoxycarbonyl-C₁₋₄alkoxy group (for example, methoxycarbonylmethoxy, ethoxycarbonylmethoxy, n-propoxycarbonylmethoxy, isopropoxycarbonylmethoxy, n-butoxycarbonylmethoxy, isobutoxycarbonylmethoxy, sec-butoxycarbonylmethoxy, tert-butoxycarbonylmethoxy, n-pentyloxycarbonylmethoxy, 1-(methoxycarbonyl)ethoxy, 1-(ethoxycarbonyl)ethoxy),
a C₃₋₇alkenyloxycarbonyl-C₁₋₄alkoxy group (for example, allyloxycarbonylmethoxy, 1-buten-3-yloxycarbonylmethoxy, 3-buten-1-yloxycarbonylmethoxy),
a C₃₋₇alkynyloxycarbonyl-C₁₋₄alkoxy group (for example, propargyloxycarbonylmethoxy, 2-butyn-1-yloxycarbonylmethoxy, 3-butyn-2-yloxycarbonylmethoxy).
R⁵ is hydrogen, a C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl), a C₃₋₆cycloalkyl group (for example, cyclopropyl, cyclobutyl, cyclohexyl), hydroxyl group, cyano group, mercapto group, C₁₋₆alkoxy group (for example, methoxy, ethoxy, n-propoxy, isopropoxy, n-butoxy, isobutoxy, sec-butoxy, tert-butoxy, n-pentyloxy),
a C₁₋₆alkoxy-C₁₋₄alkoxy group (for example, methoxymethoxy, ethoxymethoxy, n-propoxymethoxy, isopropoxymethoxy, n-butoxymethoxy), a C₃₋₆cycloalkyloxy group (for example, cyclopropyloxy, cyclobutyloxy, cyclopentyloxy),
a C₁₋₆haloalkoxy group (for example, trifluoromethoxy), a C₂₋₆alkenyloxy group (for example, allyloxy, 1-buten-3-yloxy, 3-buten-1-yloxy), a C₂₋₆haloalkenyloxy group (for example, 2-chloro-2-penten-1-yloxy),
a C₂₋₆alkynyloxy group (for example, propargyloxy, 2-butyn-1-yloxy, 3-butyn-2-yloxy), a C₆₋₁₀aryloxy group (for example, phenoxy, naphthyloxy),
a C₇₋₁₂aralkyloxy group (for example, benzyloxy, phenethyloxy), a C₁₋₆alkylthio group (for example, methylthio, ethylthio, n-propylthio, isopropylthio, n-butylthio, isobutylthio, sec-butylthio, tert-butylthio, n-pentylthio, sec-pentylthio, isopentylthio, neopentylthio, n-hexylthio, isohexylthio), a C₁₋₆alkoxy-C₁₋₄alkylthio group (for example, methoxymethylthio, ethoxymethylthio, methoxyethylthio), a C₃₋₆cycloalkylthio group (for example, cyclopropylthio, cyclobutylthio, cyclopentylthio), a C₁₋₆haloalkylthio group (for example, trifluoromethylthio), a C₂₋₆alkenylthio (for example, allylthio, 1-buten-3-ylthio, 3-buten-1-ylthio),
a C₂₋₆haloalkenylthio group (for example, 2-chloro-2-penpen-1-ylthio), a C₂₋₆alkynylthio group (for example, propargylthio, 2-butyn-1-ylthio, 3-butyn-2-ylthio), a C₆₋₁₀arylthio group (for example, phenylthio, naphthylthio), a C₇₋₁₂aralkylthio group (for example, benzylthio, phenethylthio), a C₁₋₆alkylsulfonyl group (for example, methanesulfonyl, ethanesulfonyl, n-propylsulfonyl, isopropylsulfonyl, n-butylsulfonyl, isobutylsulfonyl, sec-butylsulfonyl, tert-butylsulfonyl, n-pentylsulfonyl, sec-pentylsulfonyl, isopentylsulfonyl, neopentylsulfonyl, n-hexylsulfonyl, isohexylsulfonyl), a C₃₋₆cycloalkylsulfonyl group (for example, cyclopropylsulfonyl, cyclobutylsulfonyl, cyclopentylsulfonyl), a C₁₋₆haloalkylsulfonyl group (for example, chloromethylsulfonyl, trifluoromethylsulfonyl), a C₂₋₆alkenylsulfonyl group (for example, allylsulfonyl, methallylsulfonyl),
a C₂₋₆haloalkenylsulfonyl group (for example, 2-chloro-2-propen-1-ylsulfonyl), a C₂₋₆alkynylsulfonyl group (for example, propargylsulfonyl),or cyclic amino group which contains 1 or 2 atoms selected from oxygen atom, sulfur atom and nitrogen atom (for example, morpholino, pyrrolidino, piperidino).
Y is oxygen atom , sulfur atom, or -N-R¹³,
R¹¹ is hydrogen or a C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl).
R¹² is hydrogen, a C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl), a C₃₋₆cycloalkyl group (for example, cyclopropyl, cyclobutyl, cyclohexyl), a C₁₋₆haloalkyl group (for example, chloromethyl, 2-chloroethyl, trifluoromethyl), a C₂₋₆alkenyl group (for example, allyl, 1-buten-3-yl, 3-buten-1-yl), a C₂₋₆haloalkenyl group (for example, 2-chloro-2-propen-1-yl), a C₂₋₆alkynyl group (for example, propargyl, 2-butyn-1-yl, 3-butyn-2-yl), a C₆₋₁₀aryl group (for example, phenyl, naphthyl), a C₇₋₁₂aralkyl group (for example, benzyl, phenethyl), a C₁₋₆alkoxy-C₁₋₄alkyl group (for example, methoxymethyl, ethoxymethyl, 2-methoxyethyl, 2-ethoxyethyl), a C₂₋₆alkenyloxy-C₁₋₄alkyl group (for example, allyloxymethyl, 1-buten-3-yloxymethyl, 3-buten-1-yloxymethyl), a C₂₋₆alkynyloxyC₁₋₄alkyl group (for example, propargyloxymethyl, 2-butyn-1-yloxymethyl, 3-butyn-1-yloxymethyl),
a C₃₋₆cycloalkoxyC₁₋₄alkyl group (for example, cyclopropyloxymethyl, cyclobutyloxymethyl),
a C₂₋₇alkoxycarbonyl-C₁₋₄alkyl group (for example, methoxycarbonylmethyl, ethoxycarbonylmethyl, isopropoxycarbonylmethyl, sec-butoxycarbonylmethyl, 1-(methoxycarbonyl)ethyl),
a C₃₋₇alkenyloxycarbonyl-C₁₋₄alkyl group (for example, allyloxycarbonylmethyl, 1-buten-3-yloxycarbonylmethyl, 3-buten-1-yloxycarbonylmethyl),
a C₃₋₇alkynyloxycarbonyl-C₁₋₄alkyl group (for example, propargyloxycarbonylmethyl, 2-butyn-1-yloxycarbonylmethyl, 3-butyn-2-yloxycarbonylmethyl),
a C₄₋₇cycloalkoxycarbonyl-C₁₋₄alkyl group (for example, cyclopropyloxycarbonylmethyl, cyclohexyloxycarbonylmethyl, 1-(cyclopropyloxycarbonyl)ethyl),
a C₂₋₇haloalkoxycarbonyl-C₁₋₄alkyl group (for example, chloromethoxycarbonylmethyl, 2-chloroethoxycarbonylmethyl, 2-(chloromethoxycarbonyl)ethyl),
a C₃₋₇haloalkenyloxycarbonyl-C₁₋₄alkyl group (for example, 2-chloro-2-propen-1-yloxycarbonylmethyl), or a C₇₋₁₂aralkyloxycarbonyl-C₁₋₄alkyl group (for example, benzyloxycarbonylmethyl, 2-(benzyloxycarbonyl)ethyl, phenethyloxycarbonylmethyl).
R¹³ is hydrogen, a C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl), a C₁₋₆alkoxy-C₁₋₄alkyl group (for example, methoxymethyl, ethoxymethyl, 2-methoxyethyl, 2-ethoxyethyl), a C₁₋₇alkanoyl group (for example, formyl, acetyl, propionyl, butyryl, isobutyryl, pentanoyl, hexanoyl), a C₇₋₁₁arylcarbonyl group (for example, benzoyl, naphthalenecarbonyl), a C₂₋₇alkoxycarbonyl group (for example, methoxycarbonyl, ethoxycarbonyl, n-propoxycarbonyl, isopropoxycarbonyl), a C₂₋₇haloalkoxycarbonyl group (for example, chloromethoxycarbonyl, bromomethoxycarbonyl, (1-chloro)ethoxycarbonyl), or a C₃₋₇haloalkenyloxycarbonyl group (for example, 2-chloro-2-propen-1-yloxycarbonyl).
R¹⁴ is hydrogen, halogen (for example, fluorine, chlorine, bromine, iodine), or a C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl).
R¹⁵ and R¹⁶ are the same or different, and each represents hydrogen, a C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl), a C₂₋₆alkenyl group (for example, allyl, 1-buten-3-yl, 3-buten-1-yl), a C₂₋₆alkynyl group (for example, propargyl, 2-butyn-1-yl, 3-butyn-2-yl), a C₃₋₆cycloalkyl group (for example, cyclopropyl, cyclobutyl, cyclopentyl, cyclohexyl), C₁₋₆alkoxy-C₁₋₄alkyl group (for example, methoxymethyl, ethoxymethyl, 2-methoxyethyl, 2-ethoxyethyl), a C₆₋₁₂aralkyl group (for example, benzyl, phenethyl), a C₁₋₄alkyl group which is substituted by a 5- or 6- membered hetero cyclic ring containing one or two atoms selected from nitrogen atom , oxygen atom and sulfur atom (for example, 4-pyridylmethyl, 2-furylmethyl, 2-thiophenemethyl),a C₁₋₇alkanoyl group (for example, formyl, acetyl, propionyl, butyryl, isobutyryl, pentanoyl, hexanoyl), a C₆₋₁₂arylcarbonyl group (for example, benzoyl, naphthalenecarbonyl), a C₂₋₇haloalkylcarbonyl group (for example, chloroacetyl, trifluoroacetyl), a C₂₋₇alkoxycarbonyl group (for example, methoxycarbonyl, ethoxycarbonyl, n-propoxycarbonyl, isopropoxycarbonyl), a C₃₋₇alkenyloxycarbonyl group (for example, allyloxycarbonyl, 1-buten-3-yloxycarbonyl, 3-buten-1-yloxycarbonyl), a C₃₋₇alkynyloxycarbonyl group (for example, propargyloxycarbonyl, 2-butyn-1-yloxycarbonyl,3-butyn-2-yloxycarbonyl),
a C₄₋₇cycloalkyloxycarbonyl group (for example, cyclopropyloxycarbonyl, cyclobutyloxycarbonyl, cyclohexyloxycarbonyl), a C₂₋₇haloalkoxycarbonyl group (for example, chloromethoxycarbonyl, (1-chloroethoxy)carbonyl), a C₁₋₆alkylsulfonyl group (for example, methylsulfonyl, ethylsulfonyl, n-propylsulfonyl, isopropylsulfonyl, n-butylsulfonyl, isobutylsulfonyl), a C₂₋₆alkenylsulfonyl group (for example, allylsulfonyl), a C₂₋₆haloalkenylsulfonyl group (for example, 2-chloro-2-propen-1-ylsulfonyl),
a C₂₋₆alkynylsulfonyl group (for example, propargylsulfonyl),a C₃₋₆cycloalkylsulfonyl group (for example, cyclopropylsulfonyl, cyclobutylsulfonyl, cyclohexylsulfonyl), a C₁₋₆haloalkylsulfonyl group (for example, chloromethylsulfonyl), a C₆₋₁₀arylsulfonyl group (for example, phenylsulfonyl, naphthylsulfonyl), C₇₋₁₂aralkylsulfonyl group (for example, benzylsulfonyl, phenethylsulfonyl), or a group represented by the formula: 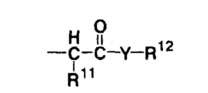 (wherein each of R¹¹, R¹² and Y has the same meaning as described above)
R⁶ is hydrogen or a C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl),
m is 0 or 1,
R⁷ is hydrogen, a C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl), a C₂₋₆alkenyl group (for example, allyl, 1-buten-3-yl, 3-buten-1-yl), a C₂₋₆alkynyl group (for example, propargyl, 2-butyn-1-yl, 3-butyn-2-yl),
a C₁₋₆haloalkyl group (for example, chloromethyl, chloroethyl), a C₁₋₆alkoxy-C₁₋₄alkyl group (for example, methoxymethyl, ethoxymethyl, 2-methoxyethyl, 2-ethoxyethyl), a C₁₋₇alkanoyl group (for example, formyl, acetyl, propionyl, butyryl, isobutyryl, pentanoyl, hexanoyl), a C₇₋₁₁arylcarbonyl group (for example, benzoyl, naphthalenecarbonyl), a C₂₋₇alkoxycarbonyl group (for example, methoxycarbonyl, ethoxycarbonyl, n-propoxycarbonyl, isopropoxycarbonyl),
a C₃₋₇alkenyloxycarbonyl group (for example, allyloxycarbonyl, 1-buten-3-yloxycarbonyl, 3-buten-1-yloxycarbonyl), a C₃₋₇alkynyloxycarbonyl group (for example, propargyloxycarbonyl, 2-butyn-1-yloxycarbonyl,3-butyn-2-yloxycarbonyl),
a C₄₋₇cycloalkyloxycarbonyl group (for example, cyclopropyloxycarbonyl, cyclopentyloxycarbonyl, cyclohexyloxycarbonyl), a C₂₋₇alkoxycarbonyl-C₁₋₄alkyl group (for example, methoxycarbonylmethyl, 1-(methoxycarbonyl)ethyl, ethoxycarbonylmethyl, n-propoxycarbonylmethyl, isopropoxycarbonylmethyl), a C₃₋₇alkenyloxycarbonyl-C₁₋₄alkyl group (for example, allyloxycarbonylmethyl, 1-(allyloxycarbonyl)ethyl, 1-buten-3-yloxycarbonylmethyl,
3-buten-1-yloxycarbonylmethyl), a C₃₋₇haloalkenyloxycarbonyl-C₁₋₄alkyl group (for example, 2-chloro-2-propen-1-yloxycarbonylmethyl), or a C₃₋₇alkynyloxycarbonyl-C₁₋₄alkyl group (for example, 2-butyn-1-yloxycarbonylmethyl, 1-(2-butyn-1-yloxycarbonyl)ethyl, 3-butyn-2-yloxycarbonylmethyl).
R⁸ is hydrogen, halogen (for example, fluorine, chlorine, bromine, iodine), a C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl), a C₁₋₆alkoxy group (for example, methoxy, ethoxy, n-propoxy, isopropoxy, n-butoxy, isobutoxy, sec-butoxy, tert-butoxy, n-pentyloxy, sec-pentyloxy, isopentyloxy, neopentyloxy, n-hexyloxy, isohexyloxy), a C₁₋₆haloalkoxy group (for example, chloromethoxy, 2-chloroethoxy), C₂₋₆alkenyloxy group (for example, allyloxy, 1-buten-3-yloxy, 3-buten-1-yloxy), a C₂₋₆alkynyloxy group (for example, propargyloxy, 2-butyn-1-yloxy, 3-butyn-2-yloxy), C₁₋₆alkylthio group (straight or branched alkyl group , for example, methylthio, ethylthio, n-propylthio, isopropylthio, n-butylthio, isobutylthio, sec-butylthio, tert-butylthio, n-pentylthio, sec-pentylthio, isopentylthio, neopentylthio, n-hexylthio, isohexylthio), a C₁₋₆haloalkylthio group (for example, chloromethylthio, 2-chloroethoxy), C₂₋₆alkenylthio group (for example, allylthio, 1-buten-3-ylthio, 3-buten-1-ylthio), a C₂₋₆alkynylthio group (for example, propargylthio, 2-butyn-1-ylthio, 3-butyn-2-ylthio), a C₁₋₆haloalkyl group (for example, chloromethyl, bromomethyl), a C₁₋₆alkoxyC₁₋₄alkyl group (for example, methoxymethyl, ethoxymethyl), a C₁₋₆alkylthio-C₁₋₄alkyl group (for example, methylthiomethyl, ethylthiomethyl), or a C₁₋₆alkylsulfonyl-C₁₋₄alkyl group (for example, methylsulfonylmethyl, ethylsulfonylmethyl).
R⁹ is hydrogen or a C₁₋₆alkyl group (straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl).
R¹⁰ is a C₁₋₆alkyl group (e.g. straight or branched alkyl group, for example, methyl, ethyl, n-propyl, isopropyl, n-butyl, isobutyl, sec-butyl, tert-butyl, n-pentyl, sec-pentyl, isopentyl, neopentyl, n-hexyl, isohexyl). Z is oxygen atom or sulfur atom.

The compound represented by the formula (I) (hereinafter, it may be referred to as compound (I)) includes any ones as combined with the groups selected optionally from the above-mentioned symbols, and the especially preferable compounds are shown below.
(1) The compound represented by the formula (Ia), wherein R^{1p} and R^{1a} are each the same or different, and each represents hydrogen, halogen, a C₁₋₆alkyl group, a C₁₋₆alkoxy group, a C₂₋₆alkenyloxy group, C₂₋₆alkynyloxy group or a C₁₋₃haloalkyl group and so on, among which is especially preferred, hydrogen, chlorine, bromine, methyl, ethyl, difluoromethyl or trifluoromethyl, and R² is halogen, a C₁₋₆alkyl group or cyano group, among which is especially preferred, chlorine, bromine, iodine, methyl or cyano group.
(2) The compound represented by the formula (I), wherein Ar is a group represented by Ar-1,
   R³ is hydrogen or halogen, among which is especially preferred, hydrogen, fluorine or chlorine,
   R⁴ is halogen, cyano group or nitro group, among which is especially preferred chlorine, cyano group or nitro group.
   R⁵ is a C₁₋₄alkoxy group, a C₂₋₅alkenyloxy group, a C₂₋₅haloalkenyl group, a C₂₋₅alkynyloxy group, a C₁₋₄alkanesulfonylamino group which may have a substituent on its nitrogen atom, a C₁₋₆alkoxycarbonyl-C₁₋₄alkoxy group, a C₁₋₄haloalkoxycarbonyl-C₁₋₄alkoxy group, or a C₁₋₆alkoxycarbonyl-C₁₋₄alkylthio group, among which is especially preferred, isopropoxy, isobutoxy, allyloxy, propargyloxy, 3-buten-2-yloxy, methanesulfonylamino, ethanesulfonylamino, isopropylsulfonylamino, methoxycarbonylmethoxy, ethoxycarbonylmethoxy, 1-(methoxycarbonyl)ethoxy, 2-chloro-2-propen-1-yloxy or methoxycarbonylmethylthio.
(3) The compound represented by the formula (I), wherein Ar is a group represented by Ar-2,
   R³ is hydrogen or halogen, among which is especially preferred, fluorine or chlorine,
   R⁶ is hydrogen or a C₁₋₃alkyl group, among which is especially preferred, methyl or ethyl, m is 1, R⁷ is a C₁₋₃alkyl group, a C₂₋₅alkenyl group, a C₂₋₅alkynyl group or a C₁₋₃alkoxy-C₁₋₃alkyl group, among which is especially preferred, ethyl, n-propyl, propargyl or ethoxymethoxy, Z is oxygen or sulfur.
(4) The compound represented by the formula (I), wherein Ar is a group represented by Ar-3, R³ is hydrogen or halogen, among which is especially preferred, fluorine or chlorine,
   R⁴ is halogen, cyano group or nitro group, among which is especially preferred chlorine, cyano group or nitro group,
   R⁸ is a C₁₋₃alkyl group, a C₁₋₃haloalkyl group or a C₁₋₃alkoxy-C₁₋₃alkyl group, among which is especially preferred, methyl, chloromethyl or methoxymethyl,
   Z is oxygen or sulfur.
(5) The compound represented by the formula (I), wherein Ar is a group represented by Ar-4, R³ is hydrogen or halogen, among which is especially preferred, fluorine or chlorine,
   R⁴ is halogen, cyano group or nitro group, among which is especially preferred chlorine, cyano group or nitro group,
   each of R⁹ and R¹⁰ is a C₁₋₄alkyl group, and methyl is especially preferred.
(6) The compound represented by the formula (I), wherein Ar is a group represented by Ar-5, R³ is hydrogen or halogen, among which is especially preferred, fluorine or chlorine, R⁸ is a C₁₋₃alkyl group, a C₂₋₅alkenyl group, a C₂₋₅alkynyl group, a C₁₋₃alkoxy group or a C₁₋₃alkylthio group, among which is especially preferred, ethyl, n-propyl, propargyl, methoxy, ethoxy or methylthio.
(7) The compound represented by the formula (I), wherein Ar is a group represented by Ar-6, R³ is hydrogen or halogen, among which is especially preferred, fluorine or chlorine,
   R⁴ is halogen, cyano group or nitro group, among which is especially preferred, chlorine, cyano group or nitro group,
   R⁹ is a C₁₋₃alkyl group, a C₃₋₅alkenyl group, a C₃₋₅alkynyl group or a C₁₋₃alkoxy-C₁₋₃alkyl group, among which is especially preferred, methyl, ethyl, n-propyl or propargyl.

The acid group such as sulfo group, carboxyl group and so on in the compound of this invention can form agrochemically acceptable basic salts with inorganic bases or organic bases, while, the basic group such as basic nitrogen atom in the molecule or basic amino group in substituent can form agrochemically acceptable acid addition salts with inorganic acids or organic acids. The salts with an inorganic base are those with alkali metals (for example, sodium, potassium and so on), alkaline earth metals (for example, calcium and so on), on ammonia and so on. And, the salt with an organic base includes, salts with, for example, dimethylamine, triethylamine, N,N-dimethylaniline, piperazine, pyrrolidine, piperidine, pyridine, 2-phenylethylamine, benzylamine, ethanolamine, diethanolamine, 1,8-diazabicyclo[5,4,0]undecene(hereafter, mentioned as DBU) and so on. The inorganic acid addtition sals of the compound (I) include salts with, for example, hydrochloric acid, hydrobromic acid, hydroiodic acid, sulfuric acid, nitric acid, phosphoric acid, perchloric acid and so on. The organic acid addition salt of the compound (I) includes salts with, for example, formic acid, acetic acid, propionic acid, oxalic acid, succinic acid, benzoic acid, p-toluenesulfonic acid, methanesulfonic acid, trifluoroacetic acid and so on.

The compound of this invention or a salt thereof can be used as agrochemicals such as herbicide, which are excellent in safety. The compound or a salt thereof of this invention is especially useful as herbicide, and even with a low dose, it has a very strong herbicidal activity, and exhibits a broad spectrum against weeds, for example, weed in paddy fields such as early watergrass, smallflower umbrella sedge, ducksalad, needle spikerush, arrowhead, common falsepimpernel, Indian toothcup and so on, weed in field such as southern crabgrass, green foxtail, slender amaranth, velvetleaf, goosefoot, tufted knotweed, common purslane, jimsonweed, tall morning-glory, common cocklebur, fall panicum, johnsongrass, purple nutsedge, wild oat, downy brome, common chickweed, Indian mustard, sicklepod, wild chamomile, Asiatic dayflower and so on. Moreover, it has little toxicity to crop such as rice, wheat, barley, corn, cotton and so on, and shows high safety. The compound or a salt thereof shows excellent selective herbicidal activity between crops and various kinds of weeds, has a low toxicity to mammals, fish and shellfish, and can be used very safely as herbicides for paddy field, plow land, orchard, and non-crop land without polluting the environment.

When a compound or a salt thereof of the present invention is used as agrochemicals, particularly compositions, they can be applied in the per se known forms for general use of agrochemical compositions. Namely, depending on the objects, one or more than two kinds of compounds or salts thereof are dissolved or dispersed in a suitable liquid carrier, or admixed with or adsorbed on a suitable solid carrier to get various forms of compositions, for example, emulsifiable liquids, oil-solubles, sprays, wettable powders, dusts, DL (driftless) powders, granules, fine granules, fine granules F, flowables, dry-flowables, Jumbo granules, tablets and so on. These preparations may be further supplemented with emulsifiers, dispersants, spreaders, penetrating agents, wetting agents, mucilages, stabilizers, and can be manufactured by the per se known methods. The liquid carriers (solvents) that can be used includes, for example, water, alcohols (for example, methanol, ethanol, 1-propanol, 2-propanol, ethylene glycol and so on), ketones (for example, acetone, methyl ethyl ketone and so on), ethers (for example, dioxane, tetrahydrofuran, ethylene glycol monomethyl ether, diethylene glycol monomethyl ether, propylene glycol monomethyl ether and so on), aliphatic hydrocarbons (for example, kerosene, fuel oil, machine oil and so on), aromatic hydrocarbons (for example, benzene, toluene, xylene, solvent naphtha, methylnaphthalene and so on), halogenated hydrocarbons (for example, dichloromethane, chloroform, carbon tetrachloride and so on) , acid amides (for example, N,N-dimethylformamide, N,N-dimethylacetamide and so on), esters (for example, ethyl acetate, butyl acetate, fatty acid glycerol esters and so on), and nitriles (for example, acetonitrile, propionitrile and so on). These solvents can be used by mixing one or more than two of them in appropriate ratios. The solid carriers (diluents, fillers) include, for example, vegetable powders (for example, soybean powder, tobacco powder, wheat flour, wood flour, and so on), mineral powders (for example, clays such as kaolin, bentonite, acid clay, clay and so on, talcs such as talcum powder, agalmatolite powder, and so on, silicas such as diatomaceous earth, mica powder, and so on, alumina, sulfur powder, activated carbon, and so on. These fillers can be used by mixing one or more than two of them in appropriate ratios. The liquid carrier or solid carrier can be used in a proportion of generally about 1 to 99 wt% and, preferably about 1 to 80 wt% based on the whole composition. The surfactants which may be used as emulsifiers, spreaders, penetrants, dispersants include, nonionic or anionic surfactants such as soaps, polyoxyethylene alkylarylethers (for example, NOIGEN™, E A142™ (TM means a trade mark and the same applies below; Dai-ichi Kogyo Seiyaku Co., Ltd.), polyoxyethylene aryl esters (for example, NONAL™; manufactured by Toho Chemical Industry Co., Ltd.),
alkylsulfates (for example, EMAL10™, EMAL40™; manufactured by KAO Corp.), alkylslufonates (for example, NEOGEN™, NEOGEN T™; manufactured by Dai-ichi Kogyo Seiyaku Co., Ltd., NEOPELEX ; manufactured by KAO Corp.), polyethylene glycol ethers (for example, Nonipol 85™, Nonipol 100™, Nonipol 160™ ; Sanyo Chemical Industries, Ltd.), polyol esters (for example, Tween 20™, Tween 80™; manufactured by KAO Corp.). The surfactants can be used in a proportion of generally about 0.1 to about 50%, and preferably about 0.1 to 25% with respect to the total amount of the compositions. The preferred concentration of the compound or a salt thereof of the present invention in herbicidal compositions is about 1 to 90 wt% for emulsifiable liquids and wettable powders, about 0.01 to 10 wt% for oil-solubles, dusts and DL(driftless) powders, and about 0.05 to 10 wt% for fine granules F and granules, although the concentration may be varied depending on the objective. In the practical use, it is preferable to dilute emulsifiable liquids or wettable powders and so on to an appropriate volume (for example, to about 100 to 100,000 fold of volume), for example, with water and so on before use, and to spray it (them).

The amount of the compound (I) or a salt thereof for use as an herbicide may be, in general, 0.05g to 50g, preferably 0.1g to 5g per are of paddy fields, or 0.04g to 10g. preferably 0.08 to 5g per are of plow land in terms of the active ingredient, i.e. compound (I) or a salt thereof, although it can vary with the type of field, time season, method of applying, target weed to be controlled and crops and so on. For weed in plow land, it is preferable to use the compound (I) or salts thereof as soil treating agent before emergence or soil treating agent for leaf and stem. For example, the herbicide of the present invention can be used even 2 or 3 weeks later after the emergence without exhibiting phytotoxicity.

The herbicide comprising the compound (I) of the present invention or a salt thereof can be used, in necessary, in combination with one or two (preferably one to three) kinds of other agrochemicals, for example, herbicides, plant growth regulating agents, microbicides, insecticides, acaricides, nematicides and so on.

The other herbicide (herbicidal active ingredient), includes, for example, (1) sulfonyl urea herbicide (chlorsulfuron, sulfometuron-methyl, chlorimuron-ethyl, triasulfuron, amidosulfuron, oxasulfuron, tribenuron-methyl, prosulfuron, ethametsulfuron-methyl, triflusulfuron-methyl, thifensulfuron-methyl, flazasulfuron, rimsulfuron, nicosulfuron, flupyrsulfuron, bensulfuron-methyl, pyrazosulfuron-ethyl, imazosulfuron, sulfosulfuron, cinosulfuron, azimsulfuron, metsulfuron-methyl, halosulfuron-methyl, ethoxysulfuron, cyclosulfamuron, and so on), (2) pyrazole herbicides (pyraflufen-ethyl, pyrazolate, pyrazoxyfen, benzofenap and so on), (3) carbamate herbicides (di-allate, butylate, tri-allate, phenmedipham, chlorpropham, asulam, phenisopham, benthiocarb, molinate, esprocarb, pyributicarb, dimepiperate, swep and so on), (4) chloroacetanilide herbicides (propachlor, metazachlor, alachlor, acetochlor, metolachlor, butachlor, pretilachlor, thenylchlor and so on), (5) diphenylether herbicide (acifluorfen, oxyfluorfen, lactofen, fomesafen, aclonifen, chlomethoxynil, bifenox, CNP and so on), (6) triazine herbicides (simazine, atrazine, propazine, cyanazine, ametoryn, simetryn, dimethametryn, prometryn and so on), (7) phenoxy acid or benzoic acid herbicides (2,3,6-TBA, dicamba, quinclorac, quinmerac, clopyralid, picloram, triclopyr, fluroxypyr, benazolin, diclofop-methyl, fluazifop-butyl, haloxyfop-methyl, quizalofop-ethyl, cyhalohop-butyl, 2,4-PA, MCP, MCPB, phenothio1 and so on), (8) acid amide or urea herbicides (Isoxaben, diflufenican, diuron, linuron, fluometuron, difenoxuron, methyl-daimuron, isoproturon, isouron, tebuthiuron, methabenzthiazuron, propanil, mefenacet, clomeprop, naproanilide, bromobutide, daimuron, cumyluron, etobenzanid, 3-(1-(3,5-dichlorophenyl)-1-methylethyl)-2,3-dihydro -6-methyl-5-phenyl-4H-1,3-oxazin-4-one) and so on), (9) organic phosphoric herbicides (glyphosate, bialaphos, amiprofos-methyl, anilofos, bensulide, piperophos, butamifos, anilofos and so on), (10) dinitroaniline herbicides (bromoxynil, ioxynil, dinoseb, trifluralin, prodiamine and so on), (11) cyclohexanedione herbicides (alloxydim, sethoxydim, cloproxydim, clethodim, cycloxydim, tralkoxydim and so on), (12) imidazoline herbicides (imazamethabenz, imazapyr, imazamethapyr imazethapyr, imazamox, imazaquin and so on), (13) bipyridium herbicides (paraquat, diquat and so on), (14) other herbicides (bentazon, tridiphane, indanofan, amitrole, carfentrazon-ethyl, sulfentrazon, fenchlorazole-ethyl, fentrazamide, isoxaflutole, clomazone, maleic hydrazide, pyridate, chloridazon, norflurazon, pyrithiobac, bromacil, terbacil, metribuzin, oxaziclomefone, cinmethylin, flumiclorac-pentyl, cinidon-ethyl, flumioxazin, fluthiacet-methyl, azafenidin, benfuresate, oxadiazon, oxadiargyl, pentoxazone, cyhalofop-butyl, cafenstrole, pyriminobac-methyl, bispyribac-sodium, pyribenzoxim, 7-(4,6-dimethoxypyrimidin-2-ylthio)-3-methylphthalide,1-(2-chlorophenyl)-4-(N-cyclohexyl-N-ethylcarbamoyl)-5(4H)-tetrazolinone, 2-(2-(3-chlorophenyl)-2,3-epoxypropyl)-2-ethylindane-1,3-dione), ACN, 3-(2-chloro-4-methylsulfonylbenzoyl)-4-phenylthiobicyclo[3.2.1]oct-3-en-2-one, dithiopyr, dalapon, chlorthiamid and so on) and so on.

The plant growth regulating agent (plant growth regulating active ingredients) includes, for example, hymexazol, paclobutrazol, uniconazole-P, inabenfide, prohexadione-calcium and so on. The microbicide (microbicidal active ingredients) includes, for example, (1) polyhaloalkylthio fungicides (captan and so on), (2) organophospate fungicides (IBP, EDDP, tolclofos-methyl and so on), (3) benzimidazol fungicides (benomyl, carbendazim, thiophanate-methyl and so on), (4) carboxyamide fungicides (meproni1, flutolanil, thifluzamid, furametpyr, teclofthalam, pencycuron, carpropamid, diclocymet and so on), (5) acylalanine fungicides (metalaxyl and so on), (6) azole fungicides (triflumizole, ipconazole, pefurazoate, prochloraz and so on), (7) methoxyacryl fungicides (azoxystrobin, metominostrobin and so on), (8) antibiotic fungicides (validamycin A, blasticidin S, kasugamycin, polyoxin and so on), (9) other fungicides (fthalide, probenazole, isoprothiolane, tricyclazole, pyroquiln, ferimzone, acibenzolar S-methyl, diclomezine, oxolinic acid, phenazine oxide, TPN, iprodione and so on) and so on.

The insecticide (insecticidal active ingredients) includes, for example, (1) organic phosphate insecticides (fenthion, fenitrothion, pirimiphos-methyl, diazinon, quinalphos, isoxathion, pyridafenthion, chlorpyrifos-methyl, vamidothion, malathion, phenthoate, dimethoate, disulfoton, monocrotophos, tetrachlorvinphos, chlorfenvinphos, propaphos, acephate, trichlorphon, EPN, pyraclofos and so on), (2) carbamate insecticides (carbaryl, metolcarb, isoprocarb, BPMC, propoxur, XMC, carbofuran, carbosulfan, benfuracarb, furathiocarb, methomyl, thiodicarb and so on), (3) synthetic pyrethroide insecticides (cycloprothrin, ethofenprox and so on), (4) neristoxin insecticides (cartap, bensultap, thiocyclam and so on), (5) neonicotinoide insecticides (imidacloprid, nitenpyram, acetamiprid, thiamethoxam), 3-(6-chloro-3-pyridylmethyl)-1,3-thiazolidin-2-ylidenecyanamide, 1-methy-2-nitro-3-(tetrahydrofuran-3-ylmethyl)-guanidine, (E)-1-(2-chloro-1,3-thiazol-5-ylmethyl)-3-methy-2-nitroguanidine and so on), (6) other insecticide (buprofezin, tebufenozide, fipronil and so on) and so on. The acaricide (acaricidal active ingredients) includes, for example, hexythiazox, pyridaben, fenpyroximate, tebufenpyrad, chlorfenapyr, etoxazole, pyrimidifen and so on. The nematicide (nematicidal active ingredients) includes, for example, fosthiazate and so on. The other agrochemical active ingredients (for example, herbicidal active ingredients, plant growth regulating active ingredients, fungicidal active ingredients, insecticidal active ingredients, acaricidal active ingredients, nematicidal active ingredients and so on) can be used in an amount of about 0.1 to 20 wt%, preferably about 0.1 to 10 wt% to the total amount of the compositons.

Moreover, the herbicide comprising the compound (I) of the present invention or a salt thereof can be, in necessary, mixed with a synergist (for example, piperonyl butoxide and so on), an attractant (for example, eugenol and so on), a repellent (for example, creosote and so on), a coloring agent ( for example, edible blue No.1 and so on), a fertilizer (for example, urea and so on) and so on.

Although the compound (I) of the present invention or the salt thereof is a novel compound, it can be manufactured by the per se known methods or the similar methods. The compound (I) or the salt thereof can be manufactured by the manufacturing methods 1 to 13 described hereinafter, but, the manufacturing method will not be limited to them.

### MANUFACTURING METHOD

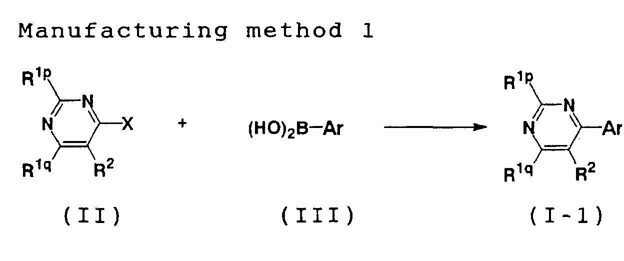

wherein each of R^{1p}, R^{1q}, R² and Ar has the same meaning as mentioned above, and X is halogen.

Arylboronic acid (III) can be manufactured by the known method (for example, Org. Synth..39,3(1959), J.Org.

Chem.,56(12), 3763 (1991) and so on) or the similar way. In this reaction, the compound (III) is usually used in a proportion of about 0.8 to 2 equivalents, preferably about 0.9 to 1.5 equivalents to the amount of the compound (II). This reaction is carried out in a solvent, which does not interfere with the reaction. The preferable solvent contains, for example, aromatic hydrocarbons such as benzene, toluene, and so on, alcohols such as methanol, ethanol, propanol, and so on, ethers such as diethyl ether, diisopropyl ether, dioxane, tetrahydrofuran (THF), dimetoxyethane, and so on, ketones such as acetone, methyl ethyl ketone, and so on, nitriles such as acetonitrile, and so on, aliphatic amides such as dimethylformamide (DMF), dimethylacetamide, and so on, sulfoxides such as dimethylsulfoxide (DMSO), and so on, phosphoric amides such as hexamethyl phosphoric triamide (HMPA) and so on, sulfones such as sulfolane and so on. These solvents can be mixed for use together with two or more solvents at appropriate ratio. The preferable base used in this reaction includes, for example, organic bases such as triethylamine, tri-n-propylamine, pyridine, dimethylaniline, dimethylaminopyridine, 1,8-diazabicyclo[5,4,0]-7-undecene(DBU), 1,4-diazabicyclo[2,2,2]octane (DBO) and so on, inorganic bases, for example, alkali metal hydroxides such as sodium hydroxide, potassium hydroxide, and so on, alkaline-earth metal hydroxides such as calcium hydroxide, and so on, alkali metal hydrogencarbonates such as sodium hydrogencarbonate, and so on, alkaline-earth metal carbonates such as calcium carbonate, and so on, metal hydrides such as sodium hydride, potassium hydride, and so on. The amount of the base is about 0.7 to 4.0 equivalents, preferably about 0.9 to 2.0 equivalents to the amount of the compound (II). As the catalyst, tetrakis(triphenylphosphine)palladium (0), dichlorobis(triphenylphosphine)palladium, dichloro[1,2-bis(diphenylphosphino)ethane]palladium( II), dichloro[1,3-bis(diphenylphosphino)propane]nickel(II ), or dichlorobis(triphenylphosphine)nickel(0) can be used. The amount of the catalyst is about 0.01 to 0.3 equivalents, preferably about 0.02 to 0.1 equivalents.

The reaction temperature variess depending on the solvent, the base and so on to be used, but is usually about 20 to 180°C, preferably about 50 to 110°C. The reaction time differs depending on the reaction temperature, but is about 30 minutes to 20 hours, preferably about 1 hour to 8 hours to complete the reaction. The completion of the reaction can be confirmed by thin-layer chromatography or high-performance liquid chromatography and so on. 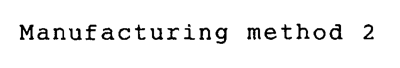 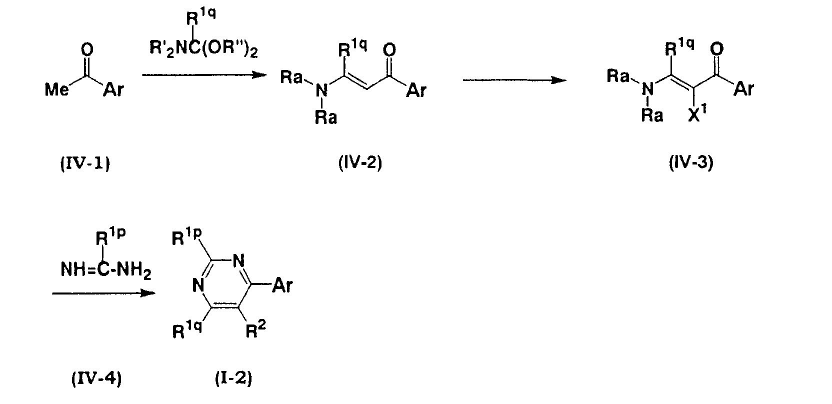 wherein each of R^{1p}, R^{1q}, R² and Ar has the same meaning as mentioned above, X¹ is halogen, and R',R"and Ra are each a C₁₋₄alkyl group.

Enaminone (IV-2) can be manufactured by the known method (for example, Heterocycles, 43(1), 221, (1996), J.Org.Chem., 45(22), 4522 (1980) and so on) or the similar way. The compound (IV-3) can be produced by halogenating the compound (IV-2). As the halogenating agent, chlorine, bromine, N-chlorosuccinimide, tert-butyl hypochlorite and so on. This reaction is carried out in a solvent, which does not interefere with the reaction. The preferable solvent includes, for example, aromatic hydrocarbons such as benzene, toluene, and so on, halogenated hydrocarbons such as chloroform, carbon tetrachloride, dichloromethane, and so on, ethers such as diethyl ether, diisopropyl ether, dioxane, tetrahydrofuran (THF), and so on, ketones such as acetone, methyl ethyl ketone, and so on, nitriles such as acetonitrile, and so on, aliphatic amides such as dimethylformamide, dimethylacetamide, and so on, sulfoxides such as dimethylsulfoxide(DMSO), and so on, phosphoric amides such as hexamethylphosphoric triamide(HMPA), and so on, sulfones such as sulfolane and so on. These solvents may be mixed for use at appropriate ratio. The amount of halogenating agent is usually about 0.8 to 3.0 equivalents, preferably about 1.0 to 1.5 equivalents to the amount of the compound (IV-2). The reaction temperature varies depending on the solvent, but is usually about -10 to 100°C, preferably about 0 to 50°C. The reaction time differs depending on the reaction temperature, but is about 10 minutes to 10 hours, preferably about 30 minutes to 5 hours to complete the reaction. In the method for producing the compound (I-2), the amidine (IV-4) can be used usually in a proportion of about 0.8 to 2.0 equivalents, preferably about 1.0 to 1.5 equivalents to the amount of the compound (IV-3). This reaction is carried out in a solvent, which does not interfere with the reaction. The preferable solvent includes, for example, aromatic hydrocarbons such as benzene, toluene, and so on, halogenated hydrocarbons such as chloroform, carbon tetrachloride, dichloromethane, and so on, ethers such as diethyl ether, diisopropyl ether, dioxane, tetrahydrofuran (THF), and so on, ketones such as acetone, methyl ethyl ketone, and so on, nitriles such as acetonitrile, and so on, alcohols such as methanol, ethanol, and so on, aliphatic amides such as dimethylformamide(DMF),dimethylacetamide, and so on, sulfoxides such as dimethylsulfoxide(DMSO) and so on, phosphoric amides such as hexamethylphosphoric triamide(HMPA), and so on, sulfones such as sulfolane and so on. These solvents may be mixed for use at appropriate ratio. Bases used in this reaction include, for example, organic bases such as triethylamine, tri-n-propylamine, pyridine, dimethylaniline, dimethylaminopyridine, 1,8-diazabicyclo[5,4,0]-7-undecene(DBU), 1,4-diazabicyclo[2,2,2]octane(DBO), and so on; inorganic bases, for example, alkali metal hydroxides such as sodium hydroxide, potassium hydroxide, and so on, alkaline-earth metal hydroxides such as calcium hydroxide, and so on, alkali metal hydrogencarbonates such as sodium hydrogencarbonate, and so on, alkaline-earth metal carbonates such as calcium carbonate, and so on, metal hydrides such as potassium hydride, sodium hydride, and so on, sodium methoxide, and so on. The amount of the base is about 1.0 to 2.0 equivalents, preferably about 1.1 to 1.5 equivalents to the amount of the compound (IV-4). The reaction temperature differs depending on the solvents and the bases to be used, but is usually from room temperature to 150°C, preferably about 50 to 100°C. The reaction time differs depending on the reaction temperature, but is about 10 minutes to 24 hours, preferably about 30 minutes to 15 hours to complete the reaction. The completion of the reaction can be confirmed by thin-layer chromatography or high-performance liquid chromatography and so on. 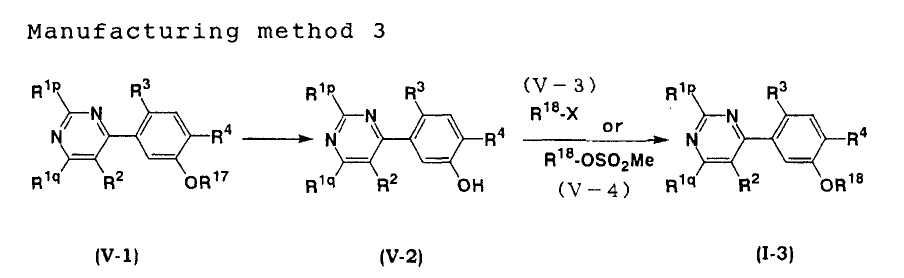 wherein each of R^{1p}, R^{1q}, R², R³, R⁴ and X has the same meaning as mentioned above, R¹⁷ is a C₁₋₄alkyl group, R¹⁸ is a C₁₋₆alkyl group, a C₃₋₆alkenyl group, a C₃₋₆alkynyl group, a C₃₋₆cycloalkyl group, a C₁₋₆haloalkyl group, a C₇₋₁₂aralkyl group, a C₆₋₁₀aryl group, a C₁₋₆alkoxy-C₁₋₄alkyl group, or a group of the formula; 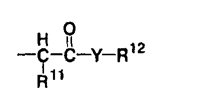 wherein each of R^{1p}, R^{1q} and R¹² has the same meaning as described above.
The compound (V-1) can be manufactured by the manufacturing method 1 or 2. In this reaction, the compound (V-1) can be deprotected in hydrobromic acid, hydroiodic acid or acetic acid solution thereof. In this reaction, hydrobromic acid or hydroiodic acid is used usually in a proportion of about 5 to 50 equivalents, preferably about 10 to 30 equivalents to the amount of the compound (V-1). The reaction temperature is usually about 10 to 180°C, preferably about 50 to 150°C. The reaction time differs depending on the reaction temperature, but is usually about 10 minutes to 24 hours, preferably about 1 to 12 hours to complete the reaction. Also, in this reaction, the compound (V-1) can be deprotected by reacting with a Lewis acid. As Lewis acid, boron tribromide, aluminum chloride and so on can be used. In this reaction, the Lewis acid is usually used in a proportion of about 1 to 10 equivalents, preferably about 2 to 5 equivalents to the amount of the compound (V-1). This reaction can be carried out in a solvent, which does not interfere with the reaction. The preferable solvent includes, for example, aromatic hydrocarbons such as benzene, toluene, and so on, halogenated hydrocarbons such as chloroform, carbon tetrachloride, dichloromethane, and so on, ethers such as diethyl ether, diisopropyl ether, dioxane, tetrahydrofuran (THF), and so on. These solvents may be mixed for use at appropriate ratio. The reaction temperature is usually about -10 to 150°C, preferably about 10 to 120°C. The reaction time differs depending on the reaction temperature, but is usually about 10 minutes to 24 hours, preferably about 1 to 12 hours to complete the reaction. In the method for producing the compound (I-3), the compound (V-3) or (V-4) is used usually in a proportion of about 0.8 to 5 equivalents, preferably 0.9 to 2.0 equivalents to the amount of the compound (V-2). This reaction may be carried out in a solvent, which does not interfere with the reaction. The preferable solvent includes, for example, aromatic hydrocarbons such as benzene, toluene, and so on, halogenated hydrocarbons such as chloroform, carbon tetrachloride, dichloromethane, and so on, ethers such as diethyl ether, diisopropyl ether, dioxane, tetrahydrofuran (THF), and so on, ketones such as acetone, methyl ethyl ketone, and so on, nitriles such as acetonitrile, aliphatic amides such as dimethylformamide(DMF),dimethylacetamide, and so on, sulfoxides such as dimethylsulfoxide(DMSO) and so on, phosphoric amides such as hexamethylphosphoric triamide(HMPA), and so on, sulfones such as sulfolane and so on. These solvents may be mixed for use at appropriate ratio. The preferable bases used in this reaction include, for example, organic bases such as triethylamine, tri-n-propylamine, pyridine, dimethylaniline, dimethylaminopyridine, 1,8-diazabicyclo[5,4,0]-7-undecene(DBU), 1,4-diazabicyclo[2,2,2]octane(DBO); inorganic bases, for example, alkali metal hydroxides such as sodium hydroxide, potassium hydroxide, and so on, alkaline-earth metal hydroxides such as calcium hydroxide, and so on, alkali metal hydrogencarbonates such as sodium hydrogencarbonate, and so on, alkaline-earth metal carbonates such as calcium carbonate, and so on, metal hydrides such as potassium hydride, sodium hydride, and so on, and potassium fluoride, and so on. The amount of the bases is about 0.8 to 4.0 equivalents, preferably about 1.0 to 1.5 equivalents to the amount of the compound (V-2). The reaction temperature differs depending on the solvent, base and so on, but is usually about -20 to 100°C, preferably about 0 to 50°C. The reaction time differ depending on the reaction temperature, but is about 10 minutes to 10 hours, preferably about 30 minutes to 3 hours to complete the reaction. The completion of this reaction can be confirmed by thin-layer chromatography or high-performance liquid chromatography and so on. 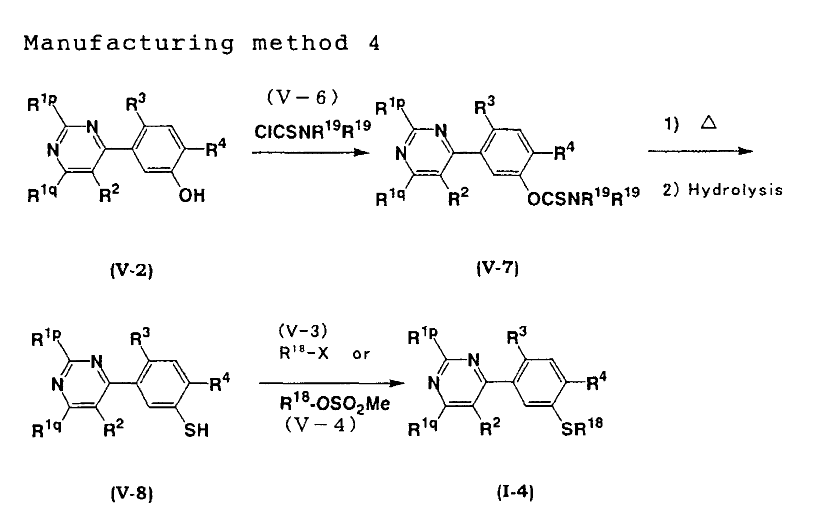 wherein each of R^{1p}, R^{1q}, R², R³, R¹⁸ and X has the same meaning as mentioned above, R¹⁹ is a C₁₋₄alkyl group.
The compound (V-8) can be manufactured from the compound (V-2) by the known method described in the papers, for example, J. Org, Chem, 31, 3980 (1996) and so on. In the reaction for producing the compound (V-7) from the compound (V-2), the compound (V-6) is usually used in a proportion of about 0.8 to 3.0 equivalents, preferably about 0.9 to 1.5 equivalents to the amount of the compound (V-2). This reaction may be carried out in a solvent, which does not interfere with the reaction. The solvent, may be any one of the solvents as mentioned for the reaction in the manufacturing method 3 (manufacturing of I-3). As the preferable bases used in this reaction, the same bases used in the manufacturing method 3 (manufacturing of I-3) can be used. The amount of the bases is about 0.8 to 3 equivalents, preferably about 1.0 to 1.5 equivalents to the amount of the compound (V-2). The reaction temperature differs depending on the solvent and the base to be used, but is generally about -20 to 150°C,preferably about 0 to 100°C. The reaction time differs depending on the reaction temperature, but is about 10 minutes to 10 hours, preferably about 30 minutes to 5 hours to complete the reaction. The reaction for producing the compound (V-8) from the compound (V-7) is carried out in the absence or presence of a solvent, which does not interfere with the reaction under heating. The preferable solvent includes, for example, aromatic hydrocarbons such as benzene, toluene, p-dichlorobenzene, and so on, halogenated hydrocarbons such as chloroform, carbon tetrachloride, dichloromethane, and so on, ethers such as diethyl ether, diisopropyl ether, dioxane, tetrahydrofuran (THF), and so on, ketones such as acetone, methyl ethyl ketone, and so on, nitriles such as acetonitrile, and so on. The reaction temperature differs depending on the solvent and the base to be used, but is usually about 50 to 200°C, preferably about 70 to 150°C. The reaction time differs depending on the reaction temperature, but is usually about 10 minutes to 10 hours, preferably about 30 minutes to 5 hours to complete the reaction. Then, the resulting product can be subjected to the hydrolysis reaction to provide the mercapto compound (V-8). The hydrolysis can be conducted by using inorganic bases such as alkali metal hydroxides such as sodium hydroxide, potassium hydroxide, and so on, alkaline-earth metal hydroxides such as calcium hydroxide, and so on, alkali metal hydrogencarbonates such as sodium hydrogencarbonate, and so on, alkaline-earth metal carbonates such as calcium carbonate, and so on. The amount of the base is about 0.8 to 10 equivalents, preferably about 1.0 to 5 equivalents. The reaction temperature differs depending on the base to be used, but is generally about -10 to 150°C, preferably about 0 to 100°C. The reaction time differs depending on the reaction temperature, but is about 10 minutes to 5 hours, preferably about 30 minutes to 2 hours to complete the reaction. In the method for producing the compound (I-4), the compound (V-3) or (V-4) is generally used in a proportion of about 0.8 to 5 equivalents, preferably about 0.9 to 2.0 equivalents to the amount of compound (V-8). This reaction may be carried out in a solvent, which does not interfere with the reaction. Such solvent may be any one of the solvents as mentioned for the reaction in the manufacturing method 3 (the manufacturing method of I-3). The preferable base includes the same one used in the manufacturing method 3 (the manufacturing method of I-3) can be used. The amount of the base is about 0.8 to 4.0 equivalents, preferably about 1.0 to 1.5 equivalents to the amount of the compound (V-8). The reaction temperature differs depending on the solvent and the base to be used, but is generally about -20 to 100°C, preferably about 0 to 50°C. The reaction time differs depending on the reaction temperature, but is about 10 minutes to 10 hours, preferably about 30 minutes to 3 hours to complete the reaction. The completion of the reaction can be confirmed by thin-layer chromatography or high-performance liquid chromatography and so on. 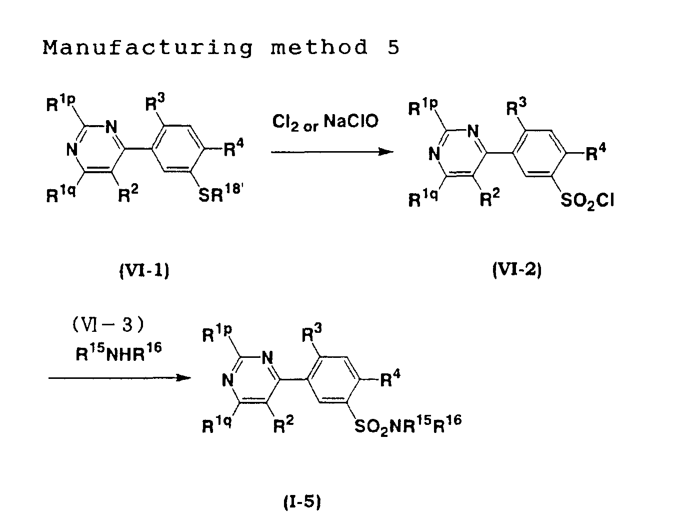 wherein each of R^{1p}, R^{1q}, R², R³, R⁴, R¹⁵ and R¹⁶ has the same meaning as mentioned above, R¹⁸ is benzyl group. In the reaction for producing the compound (VI-2) from the compound (VI-1), sulfonyl chloride(VI-2) can be obtained by reacting the compound (VI-1) with the excessive amount of chlorine gas or sodium hypochlorite, preferably in a proportion of about 5 to 10 equivalent amount, in a solvent, which dose not interfere with the reaction. As the solvent, for example, water, acetic acid, halogenated hydrocarbons such as dichloromethane, and chloroform and so on can be used. These solvents may be mixed for use at appropriate ratio. The reaction temperature differs depending on the solvent to be used, but is usually about -50 to 60°C, preferably about -20 to 30°C. The reaction time differs depending on the reaction temperature, but is usually about 5 minutes to 2 hours, preferably about 10 minutes to 1 hour to complete the reaction. Then, in the method for producing the compound (I-5), the compound (VI-3) is usually used in a proportion of about 0.8 to 3 equivalents, preferably about 0.9 to 1.3 equivalents to the amount of the compound (VI-2). This reaction can be carried out in a solvent which does not interfere with the reaction. The solvent may be any one of the solvents as mentioned for the reaction in the manufacturing method 1. As the preferable bases used in this reaction, the same base as mentioned for the reaction in the manufacturing method 1 can be used, or the compound (I-3) may concurrently serve as a base. The amount of the base is about 0.8 to 4.0 equivalents, preferably about 1.0 to 1.5 equivalents to the amount of the compound (VI-3). When the compound (VI-3) acts as a base concurrently, about 1.0 to 1.5 equivalents of the compound (VI-3) is further required. The reaction temperature differs depending on the solvents and the bases to be used, but is generally about -20 to 100°C, preferably about 0 to 50°C. The reaction time differs depending on the reaction temperature, but is about 10 minutes to 14 hours, preferably about 30 minutes to 5 hours to complete the reaction. The completion of the reaction can be confirmed by thin-layer chromatography or high-performance liquid chromatography and so on. 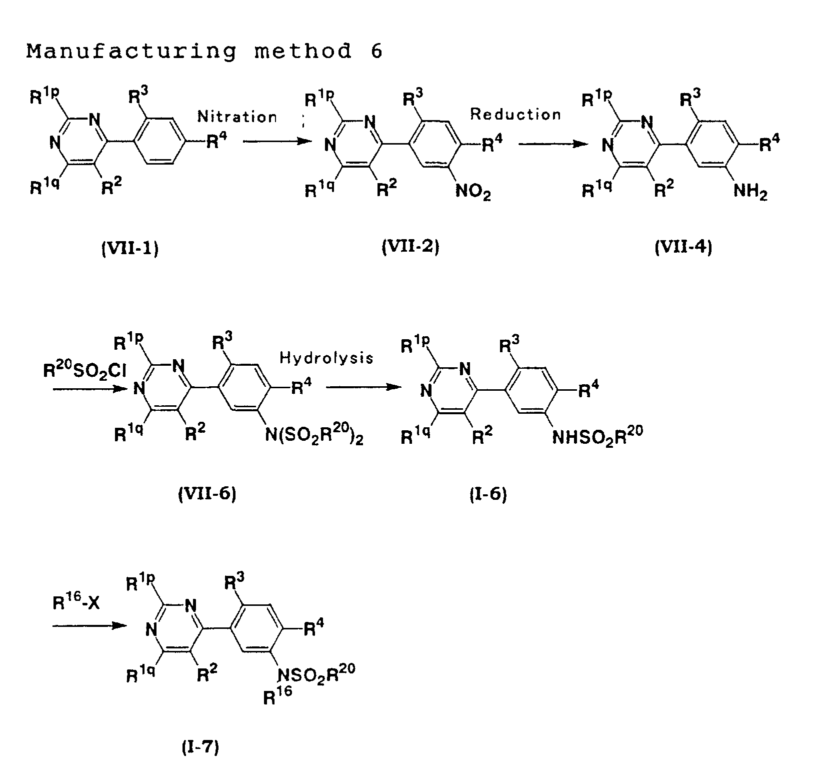 wherein each of R^{1p}, R^{1q}, R², R³, R⁴, R¹⁶ and X has the same meaning as mentioned above, and R²⁰ is a C₁₋₆alkyl group, a C₂₋₆alkenyl group, a C₂₋₆alkynyl group, a C₆₋₁₀aryl group or a C₇₋₁₂aralkyl group.
The compound (VII-2) can be manufactured by the known method (for example, Org.Synth., Coll., Vol.III, 337 and so on) or by the similar method. Namely, the reaction can be conducted under the condition wherein the concentrated nitric acid or fuming nitric acid is used as a nitrating agent, and acetic acid or sulfuric acid is used as a solvent. The nitrating agent is used in a proportion of about 0.8 to 2.0 equivalents, preferably about 1.0 to 1.2 equivalents to the amount of the compound (VII-1). The reaction temperature is usually about 0 to 100°C, preferably about 10 to 50°C. The reaction time is usually about 10 minutes to 12 hours, preferably about 30 minutes to 6 hours to complete the reaction. The compound (VII-4) can be manufactured by the known method (for example, Org.Synth.,Coll.,Vol.II,160,Org. Synth.,Coll.,Vol.V,829, and so on) or the similar method. Namely, the compound (VII-4) can be manufactured with a reductant such as iron or tin under the acid condition by acetic acid, hydrochloric acid and so on. As the solvent, for example, aliphatic carboxylic acids such as acetic acid, and so on, alcohols such as methanol, ethanol, and so on, water and so on can be used. The reaction temperature differs depending on the solvents to be used, but is generally about 0 to 100°C, preferably about 10 to 50°C. The reaction time is about 30 minutes to 12 hours, preferably about 1 to 6 hours. Also, the compound (VII-4) can be produced by catalytic hydrogenation with a palladium-carbon catalyst. As the solvent, for example, aliphatic carboxylic acids such as acetic acid, and so on, aliphatic carboxylates such as ethyl acetate, and so on, alcohols such as methanol, ethanol, and so on can be used. The reaction temperature is generally about 0 to 50°C, preferably about 10 to 25°C, and the point of where the theoretical amount of hydrogen is consumed will be regarded as the termination of the reaction. In the reaction for producing the compound (VII-6) from the compound (VII-4), sulfonyl chloride (VII-3) is usually used in a proportion of about 1.5 to 4.5 equivalents, preferably about 1.8 to 3.0 equivalents to the amount of the amino compound (VII-4). This reaction can be carried out in a solvent, which does not interfere with the reaction. As the solvent, the same solvent as mentioned for the reaction in the manufacturing method 3(manufacturing of I-3) can be used. As the preferable bases used in this reaction, the same base as mentioned for the reaction in the manufacturing method 3 (manufacturing of I-3) can be used. The amount of the bases is about 1.8 to 5.0 equivalents, preferably about 2.0 to 3.5 equivalents to the amount of the compound (VII-4). The reaction temperature differs depending on the solvents and the bases to be used, but is generally about -20 to 150°C, preferably about 0 to 100°C. The reaction time differs depending on the reaction temperature, but is about 10 minutes to 14 hours, preferably about 30 minutes to 8 hours to complete the reaction. The compound (I-6) can be produced by hydrolysis of the compound (VII-6) under basic condition. The base used in this reaction includes, for example, alkali metal hydroxides such as sodium hydroxide, potassium hydroxide, and so on, alkaline-earth metal hydroxides such as calcium hydroxide, and so on, alkali metal hydrogencarbonates such as sodium hydrogencarbonate, and so on, alkaline-earth metal carbonates such as calcium carbonate, and so on. The amount of base is about 0.8 to 3.0 equivalents, preferably about 0.9 to 1.5 equivalents to the amount of the compound (VII-6). The reaction solvent includes, for example, water, ethers such as dioxane, tetrahydrofuran(THF), and so on, ketones such as acetone, methyl ethyl ketone, and so on, nitriles such as acetonitrile, and so on, aliphatic amides such as dimethylformamide(DMF), dimethylacetamide, and so on. These solvents may be mixed for use in appropriate ratio. The reaction temperature differs depending on the solvents and bases to be used, but is usually about -10 to 100°C, preferably about 0 to 50°C. The reaction time differs depending on the reaction temperature, but is usually about 10 minutes to 10 hours, preferably about 30 minutes to 5 hours to complete the reaction. The compound (I-7) can be manufactured by alkylating and acylating the compound (I-6) under basic condition. The alkylating or acylating agent (R⁷-X) is usually used in a proportion of about 0.8 to 3.0 equivalents, preferably about 0.9 to 2.0 equivalents to the compound (I-6). This reaction can be carried out in the solvent, which does not interfere with the reaction. As the solvent, the same solvent mentioned for the reaction in the manufacturing method 3 (the manufacturing method of I-3) can be used. As the preferable base, the same base as mentioned for the reaction in the manufacturing method 3 (the manufacturing method of I-3) can be used. The amount of the base is about 0.8 to 4.0 equivalents, preferably about 1.0 to 1.5 equivalents to the amount of the compound (I-6). The reaction temperature differs depending on the solvents and the bases to be used, is generally about -20 to 150°C, preferably about 0 to 80°C. The reaction time differs depending on the reaction temperature, but is about 10 minutes to 14 hours, preferably about 30 minutes to 8 hours to complete the reaction. The completion of the reaction can be confirmed by thin-layer chromatography or high-performance liquid chromatography and so on. 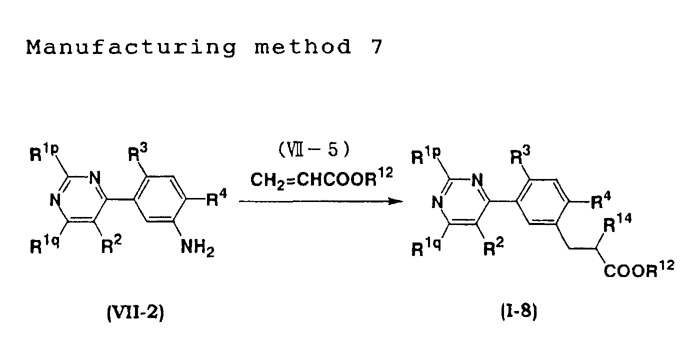 wherein each of R^{1p}, R^{1q}, R², R³, R⁴, R¹² and R¹⁴ has the same meaning as mentioned above.
The compound (I-8) can be manufactured by reacting the compound (VII-2) with a nitrite, and so on, to give a diazonium salt, then with an acrylate (VII-5) in the presence of cupric halide. The solvent used in this reaction includes, for example, aromatic hydrocarbons such as benzene, toluene, and so on, ethers such as diethyl ether, diisopropyl ether, dioxane, tetrahydrofuran (THF), and so on, ketones such as acetone, methyl ethyl ketone, and so on, nitriles such as acetonitrile, and so on. The nitride can be used usually in a proportion of about 0.9 to 2.0 equivalents, preferably about 1.1 to 1.5 equivalents. Also, as cupric halide, cupric chloride or cupric bromide is used in a proportion of about 0.9 to 2.0 equivalents, preferably about 1.1 to 1.5 equivalents. The acrylate is used in a proportion of about 2.0 to 20 equivalents, preferably about 5.0 to 15 equivalents. The reaction temperature differs depending on the solvents to be used, but is generally about -10 to 50°C, preferably about 0 to 30°C. The reaction time differs depending on the reaction temperature, but is about 1 to 48 hours, preferably about 5 to 20 hours to complete the reaction. The completion of the reaction can be confirmed by thin-layer chromatography or high-performance liquid chromatography and so on. 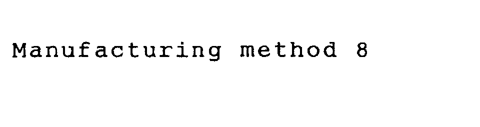 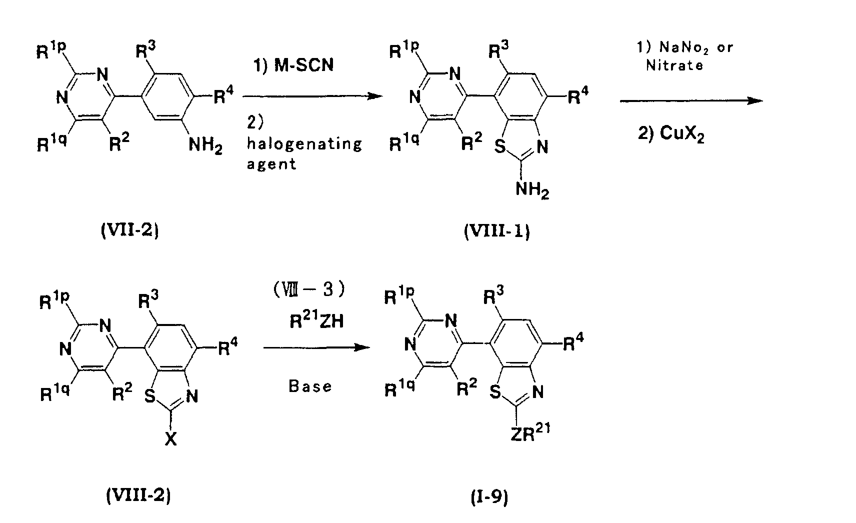 wherein each of R^{1p}, R^{1q}, R², R³, R4, Z and X has the same meaning as mentioned above, R²¹ is a C₁₋₆alkoxy group, a C₂₋₆alkenyloxy group, a C₂₋₆alkynyloxy group, a C₁₋₆alkylthio group, a C₂₋₆alkenylthio group or a C₂₋₆alkynylthio group.
In the reaction of the compound (VII-2) with a thiocyanate, the thiocyanate is usually used in a proportion of about 0.8 to 3 equivalents, preferably about 0.9 to 1.3 equivalents to the amount of the compound (VII-2). The thiocyanate includes, for example, ammonium thiocyanate, sodium thiocyanate, potassium thiocyanate, and so on. This reaction can be carried out in a solvent, which does not interfere with the reaction. The solvent mentioned for the reaction in the manufacturing method 1 can preferably be used. The reaction temperature differs depending on the solvents to be used, but is generally about -20 to 120°C, preferably about 0 to 80°C. The reaction time differs depending on the reaction temperature, but is usually about 10 minutes to 14 hours, preferably about 30 minutes to 8 hours to complete the reaction. Then a halogenating agent such as chlorine, bromine, thionyl bromide, thionyl chloride, hydrogen chloride, hydrogen bromide, and so on can be used. The reaction temperature differs depending on the halogenating agent, but is generally about -20 to 100°C, preferably about 0 to 80°C. The reaction time differs depending on the reaction temperature, but is usually about 10 minutes to 8 hours, preferably about 30 minutes to 4 hours to complete the reaction. In the reaction for producing the compound (VIII-2) from the compound (VIII-1), a diazotizing agent such as sodium nitrite, and so on, is used. The diazotizing agent is usually used in a proportion of about 0.8 to 2.0 equivalents, preferably about 0.9 to 1.2 equivalents. This reaction can be carried out in a solvent, which does not interfere with the reaction. As the preferable solvent, for example, water, acetic acid, sulfuric acid, hydrochloric acid, hydrobromic acid, acetonitrile, and so on can be used. As the copper halide, cupric chloride, cupric bromide, and so on can be used generally in a proportion of about 0.8 to 2 equivalents, preferably about 0.9 to 1.2 equivalents. The reaction temperature differs depending on the solvents to be used, but is generally about -20 to 100°C, preferably about 0 to 80°C. The reaction time differs depending on the reaction temperature, but is usually about 10 minutes to 8 hours, preferably about 30 minutes to 4 hours to complete the reaction. In the reaction for producing the compound (I-9), the compound (VIII-3) is usually used in a proportion of about 0.8 to 3.0 equivalents, preferably about 0.9 to 2.0 equivalents to the amount of the compound (VIII-2). This reaction can be carried out in a solvent, which does not interfere with the reaction. As the solvent, the same solvent as mentioned for the reaction in the manufacturing method 3 (manufacturing of I-3) can be used As the preferable bases used in this reaction, the same bases as mentioned for the reaction in the manufacturing method 3 (manufacturing of I-3) can be used. The amount of the bases is about 0.8 to 4.0 equivalents, preferably about 1.0 to 1.5 equivalents to the amount of the compound (VIII-2). The reaction temperature differs depending on the solvents and the bases to be used, but is generally about -20 to 150°C, preferably about 0 to 80°C. The reaction time differs depending on the reaction temperature, but is about 10 minutes to 14 hours, preferably about 30 minutes to 8 hours to complete the reaction. The completion of the reaction can be confirmed by thin-layer chromatography or high-performance liquid chromatography and so on. 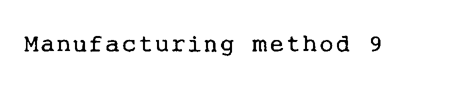 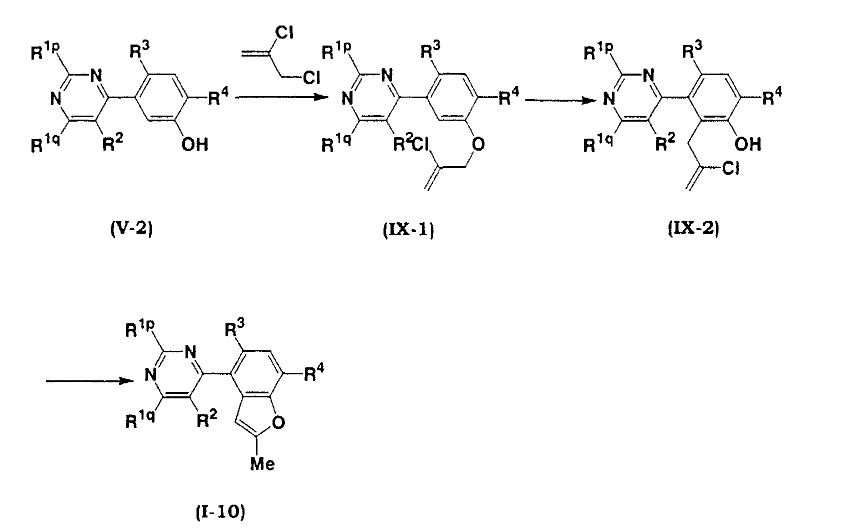 wherein each of R^{1p}, R^{1q}, R², R³ and R⁴ has the same meaning as mentioned above.
The compound (IX-1) can be manufactured by reacting the compound (V-2) with 2,3-dichloro-1-propene in presence of a base. 2,3-Dichloro-1-propene is usually used in a proportion of about 0.8 to 3 equivalents, preferably about 0.9 to 2.0 equivalents to the compound (V-2). This reaction can be carried out in a solvent, which does not interfere with the reaction. As the solvent, the same solvent as mentioned for the reaction in the manufacturing method 3 (manufacturing of I-3) can be used. As the preferable bases used in this reaction, the same bases as mentioned for the reaction in the manufacturing method 3 (manufacturing of I-3) can be used. The amount of the bases is about 0.8 to 4.0 equivalents, preferably about 1.0 to 1.5 equivalents to the amount of the compound (V-2). The reaction temperature differs depending on the solvents and the bases to be used, but is generally about -20 to 150°C, preferably about 0 to 80°C. The reaction time differs depending on the reaction temperature, but is usually about 10 minutes to 14 hours, preferably about 30 minutes to 8 hours to complete the reaction. In the reaction for producing the compound (IX-2) from the compound (IX-1), the compound (IX-2) can be manufactured by heating the compound (IX-1) in a solvent, which does not interfere with the reaction. The preferable solvent includes, for example, aromatic hydrocarbons such as benzene, toluene, xylene, and so on, aliphatic amides such as dimethylformamide(DMF), dimethylacetamide, and so on, amines such as N,N-dimethylaniline, and so on. The compound (IX-2) can be manufactured by dissolving the compound (IX-1) in the above-mentioned solvent, and at about 50 to 250°C, preferably about 100 to 200°C. The reaction time differs depending on the reaction solvent, but is usually about 30 minutes to 20 hours, preferably about 1 to 8 hours to complete the reaction. To produce the compound (I-10), the compound (IX-2) is cyclized by dissolving in organic acids such as methanesulfonic acid, trifuloromethanesulfonic acid, and so on. The reaction temperature is about 0 to 100°C,preferably about 20 to 50°C, and the reaction time differs depending on the reaction temperature, but is usually about 30 minutes to 10 hours, preferably about 1 to 5 hours to complete the reaction. The completion of the reaction can be confirmed by thin-layer chromatography or high-performance liquid chromatography and so on. 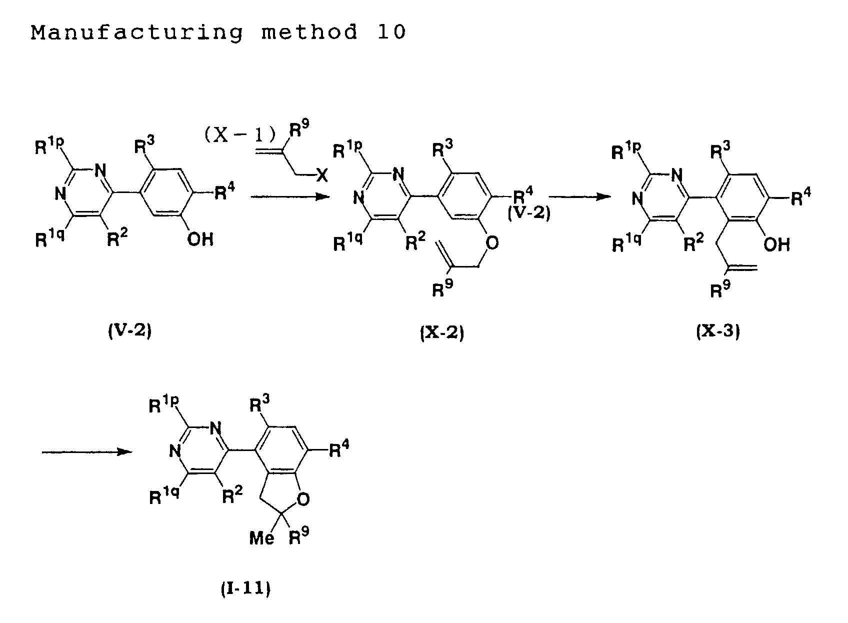 wherein each of R^{1p}, R^{1q}, R², R³, R⁴, R⁹ and X has the same meaning as mentioned above.
The compound (X-2) can be manufactured by reacting the compound (V-2) and the compound (X-1) in the presence of a base. The compound (X-1) is generally used in a proportion of about 0.8 to 3 equivalents, preferably about 0.9 to 2.0 equivalents. This reaction can be carried out in a solvent, which does not interfere with the reaction. As the solvent, the same solvent as mentioned for the reaction in the manufacturing method 3 (manufacturing of I-3) can be used. As the preferable bases used in this reaction, the same bases as mentioned for the reaction in the manufacturing method 3 (manufacturing of I-3) can be used. The amount of the bases is about 0.8 to 4.0 equivalents, preferably about 1.0 to 1.5 equivalents to the amount of the compound (V-2). The reaction temperature differs depending on the the solvents and the bases to be used, but is generally about -20 to 150°C, preferably about 0 to 80°C. The reaction time differs depending on the reaction temperature, but is usually about 10 minutes to 14 hours, preferably about 30 minutes to 8 hours to complete the reaction. The compound (X-3) can be manufactured by heating the compound (X-2) in a solvent, which does not interfere with the reaction. The preferable solvent includes, for example, aromatic hydrocarbons such as benzene, toluene, xylene, and so on, aliphatic amides such as dimethylformamide(DMF), dimethylacetamide, and so on, amines such as N,N-dimethylaniline, and so on. The reaction temperature is about 50 to 250°C, preferably about 80 to 200°C. The reaction time differs depending on the reaction solvents, but is about 30 minutes to 20 hours, preferably about 1 to 8 hours to complete the reaction. The compound (I-11) can be manufactured by reacting the compound (X-3) under acidic condition in a solvent, which does not interfere with the reaction. The preferable solvent includes, for example, aromatic hydrocarbons such as benzene, toluene, xylene, and so on. The acid used in this reaction includes, for example, organic acids such as p-tluenesulfonic acid, or inorganic acids such as sulfuric acid, and so on. The above-mentioned acid is used in a proportion of about 0.1 to 2 equivalents, preferably about 0.2 to 0.5 equivalents. The reaction temperature differs depending on the solvents to be used, but is generally about 70 to 200°C,preferably about 80 to 130°C. The reaction time differs depending on the reaction temperature, but is usually about 10 minutes to 14 hours, preferably about 30 minutes to 8 hours to complete the reaction. The completion of the reaction can be confirmed by thin-layer chromatography or high-performance liquid chromatography and so on. 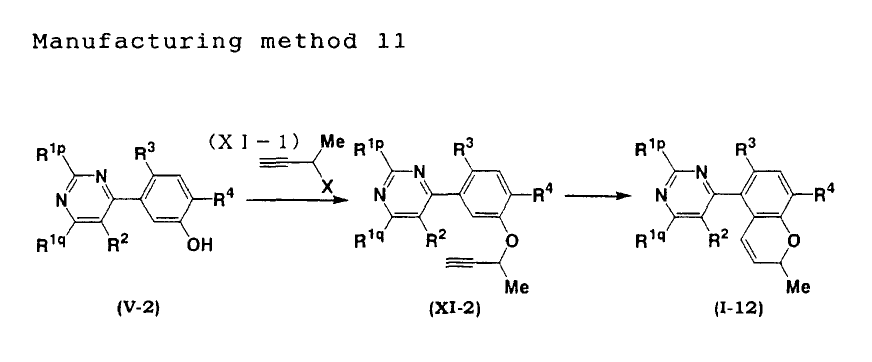 wherein each of R^{1p}, R^{1q}, R², R³, R⁴ and X has the same meaning as mentioned above.
In the reaction for producing the compound (XI-2), the compound (XI-1) is usually used in a proportion of about 0.8 to 3.0 equivalents, preferably about 0.9 to 2.0 equivalents to the amount of a compound (V-2). This reaction can be carried out in the solvent, which does not interfere with the reaction. As the solvent mentioned for the reaction in the manufacturing method 3 (manufacturing of I-3) can be used. As the preferable bases used in this reaction, the same bases as mentioned for the reaction in the manufacturing method 3 (manufacturing of I-3) can be used. The amount of the bases is about 0.8 to 4.0 equivalents, preferably about 1.0 to 1.5 equivalents to the amount of the compound (V-2). The reaction temperature differs depending on the solvents or the bases to be used, but is generally about -20 to 150°C, preferably about 0 to 80°C. The reaction time differs depending on the reaction temperature, but is usually about 10 minutes to 14 hours, preferably about 30 minutes to 8 hours to complete the reaction. The compound (I-12) can be manufactured by cyclizing the compound (XI-2) in the presence of a base. This reaction can be carried out in a solvent which does not interfere with the reaction. The preferable solvent includes, for example, aromatic hydrocarbons such as toluene, xylene, mesitylene and so on, halogenated hydrocarbons such as chloroform, carbon tetrachloride, and so on, amines such as N,N-dimethylaniline, N,N-diethylaniline, and so on, phosphoric amides such as hexamethylphosphoric triamide, and so on, sulfones such as sulfolane, and so on, polyalcohols such as diethylene glycol and so on. These solvents may be mixed for use in appropriate ratio. The preferable bases used in this reaction include for example, sodium hydrogencarbonate, potassium carbonate, cecium fluoride, potassium fluoride, calcium fluoride, cesium chloride, and so on. The amount of the base is about 0.01 to 50 equivalents, preferably about 0.1 to 20 equivalents to the amount of the compound (XI-2). The reaction temperature differs depending on the solvents and the bases to be used, but is generally about 60 to 220°C, preferably about 100 to 180°C. The reaction time differs depending on the reaction temperature, but is usually about 30 minutes to 10 hours, preferably about 1 to 5 hours to complete the reaction. The completion of the reaction can be confirmed by thin-layer chromatography or high-performance liquid chromatography and so on. 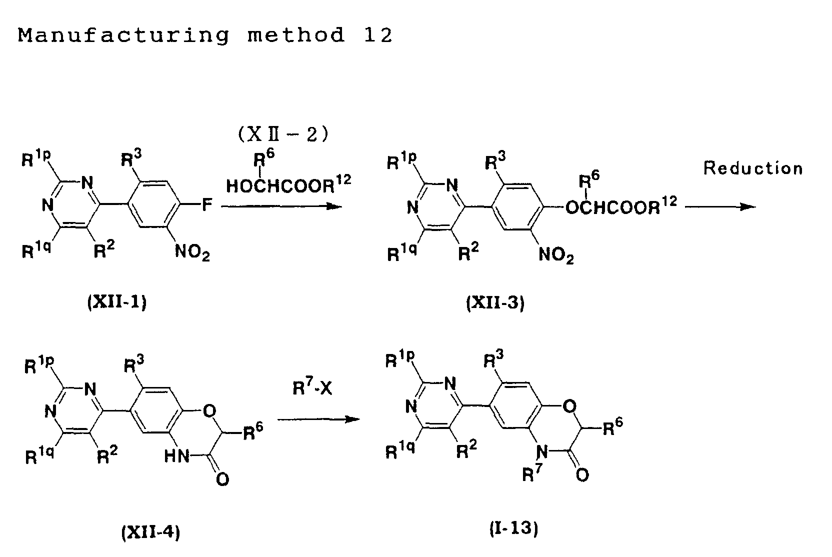 wherein each of R^{1p}, R^{1q}, R², R³, R⁶, R⁷, R¹² and X has the same meaning as mentioned above.

The compound (XII-1) can be manufactured by the manufacturing method 6 (VII-2). The compound (XII-3) can be manufactured by reacting the compound (XII-1) and the compound (XII-2) in the presence of a base. The compound (XII-2) is usually used in a proportion of about 0.8 to 2.0 equivalents, preferably about 0.9 to 1.5 equivalents to the amount of the compound (XII-1). This reaction can be carried out in a solvent, which does not interfere with the reaction. The same solvent as mentioned for the reaction in the manufacturing method 3 (the manufacturing method of I-3) can be used. As the preferable base, the same base as mentioned for the reaction in the manufacturing method 3 (the manufacturing method of I-3) can be used. The amount of the bases is about 0.8 to 4.0 equivalents, preferably about 1.0 to 1.5 equivalents to the amount of the compound (XII-1). The reaction temperature differs depending on the solvents and the bases to be used, but is generally about -20 to 150°C, preferably about 0 to 80°C. The reaction time differs depending on the reaction temperature, but is usually about 10 minutes to 14 hours, preferably about 30 minutes to 8 hours to complete the reaction. The compound (XII-4) can be manufactured by reducing the compound (XII-3) with reduced iron under acid condition such as acetic acid, hydrochloric acid, and so on. The solvent includes, for example, aliphatic carboxylates such as ethyl acetate, and so on, alcohols such as methanol, ethanol, and so on, water, and so on.
The reaction temperature differs depending on the solvents and the bases to be used, but is generally about 0 to 100°C,preferably about 10 to 50°C. The reaction time is about 30 minutes to 12 hours, preferably about 1 to 6 hours. The compound (I-13) can be manufactured by alkylating or acylating the compound (XII-4) in the presence of a base. The alkylating or acylating agent (R⁷-X) is usually used in a proportion of about 0.8 to 3 equivalents, preferably about 0.9 to 2.0 equivalents to the amount of the compound (XII-4). This reaction can be carried out in a solvent which does not interfere with the reaction. The same solvent as mentioned for the reaction in the manufacturing method 3 (manufacturing of I-3) can be used. As the preferable bases used in this reaction, the same bases as mentioned for the reaction in the manufacturing method 3 (manufacturing of I-3) can be used. The amount of the bases is about 0.8 to 4.0 equivalents, preferably about 1.0 to 1.5 equivalents to the amount of the compound (XII-4). The reaction temperature differs depending on the solvents and the bases to be used, but is generally about -20 to 150°C, preferably about 0 to 80°C. The reaction time differs depending on the reaction temperature, but is usually about 10 minutes to 14 hours, preferably about 30 minutes to 8 hours to complete the reaction. The completion of the reaction can be confirmed by thin-layer chromatography or high-performance liquid chromatography and so on. 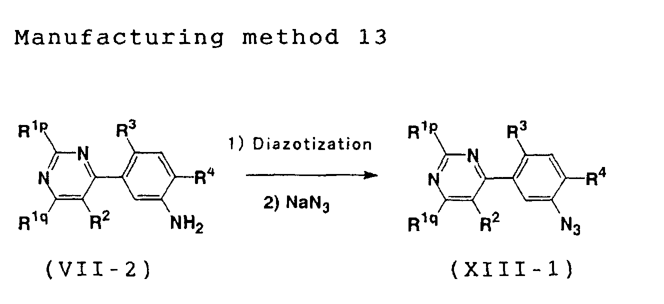 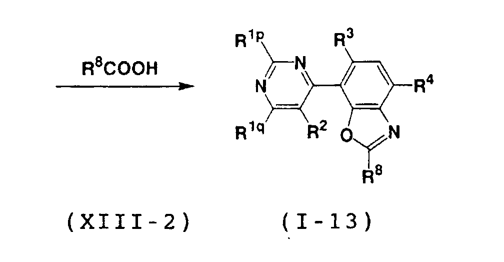 wherein each of R^{1p}, R^{1q}, R², R³ and R⁴ has the same meaning as mentioned above, R⁸ is a C₁₋₄alkyl group, a C₂₋₆alkenyl group, a C₃₋₆alkynyl group.
The compound (XIII-1) can be manufactured by reacting the compound (VII-2) with a nitrite to give diazonium salts, which is then reacted with sodium azide. This reaction can be carried out in a solvent which does not interfere with the reaction. As the solvent, there may be mentioned, for example, alcohols such as ethanol, methanol, and so on, aliphatic carboxylic acids such as acetic acid, trifluoroacetic acid, and so on, or water, and so on. As the nitrite, tert-butyl nitrite, iso-amyl nitrite, and so on can be usually used in an amount of 0.8 to 2.0 equivalents, preferably 1.1 to 1.5 equivalents. The reaction temperature differs depending on the solvents to be used, but is usually about -10 to 80°C, preferably about 0 to 30°C. The reaction time differs depending on the reaction temperature, but is usually about 0.5 to 12 hours, preferably about 1 to 6 hours. In producing the compound (I-13), the compound (XIII-2) is usually used in an amount of 0.8 to 10 equivalents. This reaction is carried out in the solvent, which does not influence on the reaction. As the preferable solvent, there may be mentioned, for example, aromatic hydrocarbons such as benzene, toluene, and so on, halogenated hydrocarbons such as chloroform, carbon tetrachloride, and so on, ketones such as acetone, methyl ethyl ketone, and so on, nitriles such as acetonitrile, and so on, or the compound (XIII-2) may serve as the solvent. The reaction temperature differs depending on the solvents and the bases to be used, but is usually about 20 to 200°C, preferably about 50 to 150°C to complete the reaction. This reaction can be confirmed by thin-layer chromatography or high-speed liquid chromatography and so on.

### EXAMPLES

Hereunder, the present invention is illustrated in more detail by the following Reference Examples and Examples. However, the scope of the present invention is not to be considered to be restricted to the present embodiments. As the eluents used in the column chromatography in the Reference Examples and Examples, the eluents monitored by means of TLC (Thin Layer Chromatography) were used. In the TLC-observation, the silica gel 60F₂₅₄ plates manufactured by Merck & Co., were used as the TLC-plate, and the UV-detector was adopted as the detection method. As silica gel for the column chromatography, silica gel 60 (0.063-0.200 mm) manufactured by Merck & Co. was used. When a mixed solvent was used as an eluent, the ratio shown in the parentheses indicates the volume to volume ratio of the mixed solvents. NMR (Nuclear Magnetic Resonance) spectrum means ¹H or ¹⁹F-NMR, and was measured by a Bruker AC-200P type (200MHz) spectrometer, using tetramethylsilane and fluorotrichloromethane as internal standard. All δ values were shown in terms of ppm. IR spectrum was measured by a Perkinelmerpalagon 100 type FT-IR spectrometer. The absorption band was shown by wave number (cm⁻¹).

Abbreviations used in the Reference Examples, Examples and Tables given below have the following meanings;
Me: methyl group, Et: ethyl group, n-Pr: normal propyl group, i-Pr: isopropyl group, tert-Bu: tertiary-butyl group, Ph: phenyl group, s: singlet, br: broad, d: doublet, t: triplet, q: quartet, m: multiplet, dd: double doublet, septet: septet, J: coupling constant, Hz: Heltz, CDCl₃: deutero-chloroform (chloroform-d), DMSO-d₆: deuterated dimethyl sulfoxide, %: wt%, mp: melting point and dec: decomposition. In addition, room temperature means a temperature within about 15-25°C.

### REFERENCE EXAMPLES

### Reference Example 1

### 4,5-dichloro-6-trifluoromethylpyrimidine (Compound No. 1-1)

(1) Ethyl trifluoroacetoacetate (29.2g, 0.16mol) and formamidine hydrochloride (12.9g, 0.16mol) were added to ethanol(150ml), and to this was dropwise added sodium methoxide (28% in methanol, 30.9g, 0.16mol) at room temperature, and the resulting mixture was stirred at 50°C for 2 hours, further refluxed for 13 hours. After cooling, the reaction mixture was evaporated, and brine was added to the residue. The resulting mixture was neutralized with concentrated hydrochloric acid (pH 4), and the crystal separated was filtered, washed with cold water, and dried. Furthermore, the filtrate was extracted with ethyl acetate (3 times), and dried, and then evaporated. The mixed solution of hexane:ethyl acetate=5:1 was added to the residue, and the crystal separated was collected by filtration, then dried to give 4-hydroxy-6-trifluoromethylpyrimidine.
   Total yield:17.0g
   ¹H-NMR(DMSO-d₆) δ : 6.83(1H, s), 8.38(1H, s), 13.15(1H, br s).
   ¹⁹F-NMR(DMSO-d₆)δ : -69.99
   m.p. :167-169 °C
(2) The compound prepared in reference example 1-(1) (1.0g, 6.09mmol) was dissolved in acetic acid (10ml), and to this solution was dropwise added antiformin(15ml) at 70-80 °C. The resulting mixture was stirred at the same temperature for 1 hour. After cooling, the reaction mixture was evaporated, and brine was added to the residue. The mixture was extracted with ethyl acetate, washed with brine, dried, and then evaporated. The crystal precipitated upon addition of hexane:ethyl acetate=10:1 was collected by filtration, and dried to give 5-chloro-4-hydroxy-6-trifluoromethyl-pyrimidine (0.5g).
   m.p.:179-183 °C
   ¹H-NMR(DMSO-d₆) δ 8.24(s), 13.69(br s).
   ¹⁹F-NMR(DMSO-d₆) δ -67.55.
(3) Thionyl chloride (13.9g, 0.11mol) and DMF (1.5g, 21mmol) were added to the compound prepared in reference example 1-(2) (9.2g, 46.3mmol), and the resulting mixture was stirred at 50 °C for 2 hours, and at 80 °C for 1 hour. After cooling, the excess amount of thionyl chloride was evaporated, and the residue was dissolved in chloroform. The solution was neutralized with aqueous sodium bicarbonate, washed with water, dried, and then evaporated. The residue was purified by silica gel column chromatography (ethyl acetate:hexane=10:1) to give 4,5-dichloro-6-trifluoromethylpyrimidine (7.6g, oil).
   ¹H-NMR(CDCl₃) δ 8.99(s).

### Reference Example 2

### 5-bromo-4-chloro-6-trifluoromethylpyrimidine (Compound No. 1-2)

(1) The compound prepared in reference example 1-(1) (3.7g, 22.5mmol) and sodium acetate (9g, 0.11mol) were added to acetic acid (36ml). Bromine (3.95g, 24.7mmol) was added to this mixture all at once at room temperature, and the resulting mixture was stirred for 68 hours at room temperature. Then, the mixture was heated at 80 °C for 1 hour, cooled, and evaporated to remove the acetic acid. After addition of water to the residue, the precipitated crystal was collected by filtration, washed with water, and dried to give 5-bromo-4-hydroxy-6-trifluoromethylpyrimidine (4.7g).
   ¹H-NMR(DMSO-d₆) δ 8.38((1H, s), 13.60(1H, br s).
(2) Thionyl chloride (2.94g, 25mmol) and DMF (0.35g, 5.2mmol) were added to the compound prepared in reference example 2-(1) (2.94g, 12mmol). The mixture was stirred at 70 °C for 1.5 hours, cooled, and evaporated to remove the excess of thionyl chloride. Aqueous sodium bicarbonate was added to the residue, and the mixture was extracted with diethyl ether. The extract was washed with brine, dried, and then evaporated to give a residue, which was purified by silica gel column chromatography (ethyl acetate:hexane=30:1), to yield 5-bromo-4-chloro-6-trifluoromethylpyrimidine (2.47g).
   ¹H-NMR(CDCl₃) δ 9.02(s).
   ¹⁹F-NMR(CDCl₃) δ-68.2.
   IR(neat) 1539, 1520, 1225, 1155, 784, 703 cm⁻¹

### Reference Example 3

### 4,5-dichloro-6-methylpyrimidine (Compound No. 1-3)

(1) Activation of Raney nickel
   Sodium hydroxide (128g) was dissolved in water (500ml), and Raney nickel (100g) was gradually added below 40 °C over 3 hours. The mixture was further stirred for 1 hour at 40-45 °C, and water (300ml) was added to the reaction mixture. The supernatant was removed by decantation (the procedure was repeated about 10 times). Then, water was continuously flowed into the bottom of the beaker through grass tube for 13 hours, so that the solution was made neutral. Finally, the supernatant was removed by decantation (the remaining suspension was used in the next step without drying).
   Activated Raney nickel (whole amount) obtained above, thiouracil (50g, 0.35mmol), 28% aqueous ammonia (75ml), and water (400ml) were mixed. The mixture was heated under reflux for 2 hours and Raney nickel was filtered off under heating and washed with warm water. The filtrate was evaporated to dryness and ethanol was added to the residue. The resultant crystal was collected by filtration (26.6g). Furthermore, the filtrate was evaporated to give 10.5g of the crystal.(these crystals were found to be the objective compound by NMR.)
   Total yield:37.1g
   m.p. :151-152 °C
   ¹H-NMR(CDCl₃ ) δ 2.16(3H, s), 6.14(1H, s), 8.05(1H, s), 12.30(1H, br s).
(2) The compound prepared in reference example 3-(1) (5.0g, 45mmol) was dissolved in acetic acid (5ml), and chlorine gas generated by potassium permanganate (11g) and concentrated hydrochloric acid (70ml) was bubbled through the reaction mixture at or below 15 °C.
   After the reaction mixture was stirred at room temperature for 1 hour, the crystal separated was collected by filtration, and dried to give 5-chloro-4-hydroxy-6-methylpyrimidine (6.8g).
   ¹H-NMR(DMSO-d₆) δ 2.33(3H, s), 8.08(1H, s).
(3) The compound prepared in reference example 3-(2) (1.0g, 7mmol) was added to phosphorus oxychloride (5ml), and the resulting mixture was stirred for 1 hour at 100 °C. After cooling, an excess of phosphorus oxychloride was evaporated, and chloroform was added to the residue. The mixture was neutralized with aqueous sodium bicarbonate. The chloroform layer was separated, and water layer was extracted with chloroform. The chloroform layers were combined, washed with water, dried, and evaporated. The residue was purified by silica gel column chromatography (hexane:ethyl acetate=1:1) to give 4,5-dichloro-6-methylpyrimidine (0.8g, oil).
   ¹H-NMR(CDCl₃) δ 2.67(3H, s), 8.73(1H, s).

### Reference Example 4

### 4,5-dichloro-6-difluoromethylpyrimidine (Compound No. 1-4)

(1) To a suspension of ethyl difluoroacetoacetate (25g, 0.15mol) and formamidine hydrochloride (15.7g, 0.195mol) in ethanol (150ml), was dropwise added 28% sodium methylate (38.6g, 0.2mol) at room temperature. The mixture was refluxed for 18 hours, cooled, and evaporated. Water (1.5L) was added to the residue, and the resulting solution was neutralized with concentrated hydrochloric acid. The crystal separated was collected by filtration, washed with water, and dried (9.9g). Furthermore, the filtrate was extracted twice with ethyl acetate, washed with brine, and then evaporated. The crystal formed upon addition of hexane/ethyl acetate=10/1 was collected by filtration, and dried to give 4-hydroxy-6-difluoromethylpyrimidine.
   Total yield:12.5g (57.0%)
   ¹H-NMR(DMSO-d₆ ) δ 6.57(1H, s), 6.70(1H, t, J=54.2Hz), 8.29(1H, s), 12.87(1H, br.s).
   ¹⁹F-NMR(DMSO-d₆) δ -121.3(d, J=57.4Hz).
(2) The compound prepared in reference example 4-(1) (10.7g, 73.2mmol) and NCS (10.75g, 80.5mmol) were dissolved in DMF (50ml), and the resulting solution was stirred at 80 °C for 2 hours. After cooling, the reaction mixture was added to ice water, extracted with ethyl acetate twice, washed with brine, dried, and then evaporated. A mixed solvent of hexane:ethyl acetate=3:1 was added to the residue, and the resultant crystal was collected by filtration and dried to give 5-chloro-4-hydroxy-6-difluoromethylpyrimidine (5.2g).
   ¹H-NMR(DMSO-d₆ ) δ 7.06(1H, t, J=52.9Hz), 8.32(1H, s), 13.45(1H, br.s).
   ¹⁹F-NMR(DMSO-d₆) δ -121.0(d, J=56.2Hz).
   m.p.:148-150 °C
(3) Thionyl chloride (8.06g, 67.7mmol) and DMF (1.0ml) were added to the compound prepared in reference example 4-(2) (4.9g, 27.1mmol) and the resulting mixture was stirred at 50 °C for 1 hour, and 80 °C for 1 hour. After cooling, the excess thionyl chloride was removed by evaporation, followed by dissolution in chloroform. The mixture was neutralized with aqueous sodium bicarbonate, washed with water, dried, and evaporated. The residue was purified by silica gel column chromatography (hexane:ethyl acetate=5:1), to give 4,5-dichloro-6-difluoromethylpyrimidine (4.4g, oil).
   ¹H-NMR(CDCl₃) δ 6.83(1H, t, J=53.1Hz), 8.97(1H, s).
   ¹⁹F-NMR(CDCl₃) δ -121.0(d, J=56.2Hz).

### Reference Example 5

### 4,5-dichloro-2-methyl-6-trifluoromethylpyrimidine (Compound No. 1-5)

(1) To a suspension of ethyl trifluoroacetoacetate (18.4g, 0.1mol) and acetamidine hydrochloride (9.5g, 0.1mol) in ethanol (100ml) was dropwise added 28% sodium methylate (19.3g, 0.1mol) under cooling with ice. The resulting mixture was stirred at room temperature for 1 hour, and further refluxed for 12 hours. After cooling, the reaction mixture was evaporated, water (150ml) was added to the residue, then the mixture was neutralized with concentrated hydrochloric acid. The crystal separated was collected by filtration, washed with water, and dried to give 4-hydroxy-2-methyl-6-trifluoromethylpyrimidine (9.8g).
   m.p.:141-143 °C
   ¹H-NMR(CDCl₃) δ 2.57(3H, s), 6.72(1H, s), 13.4(1H, br s).
   ¹H-NMR(DMSO-d₆) δ 2.36(3H, s), 6.67(1H, s).
   ¹⁹F-NMR(DMSO-d₆) δ -72.13.
(2) The compound prepared in reference example 5-(1) (5.0g, 28mmol) and NCS (4.2g, 30.8mmol) were dissolved in DMF (110ml), and the resulting solution was stirred at 80 °C for 12.5 hours. After cooling, the reaction mixture was added to ice water, extracted twice with ethyl acetate, washed with water, dried, and then evaporated. A mixed solvent of hexane:ethyl acetate=5:1 was added to the residue, and the resultant crystal was collected by filtration, and dried to give 5-chloro-4-hydroxy-2-methyl-6-trifluoromethylpyrimidine (3.6g, oil).
   ¹H-NMR(d₆-DMSO) δ 2.35(3H, s), 13.50(1H, br s).
(3) Thionyl chloride (3.41g, 29mmol) and DMF (0.48g, 6.5mmol) were added to the compound prepared in reference example 5-(2) (2.55g, 12mmol), and the resulting mixture was stirred at 70 °C for 1.5 hours. After cooling, toluene was added to the reaction mixture, and evaporated. Aqueous sodium bicarbonate was added to the residue, and the mixture was extracted with chloroform. The extract was washed with water, dried, and then evaporated. The residue was purified by silica gel column chromatography (hexane:ethyl acetate=30:1), to give 4,5-dichloro-2-methyl-6-trifluoromethylpyrimidine (1.67g, oil).
   ¹H-NMR(CDCl₃) δ 2.78(3H, s).
   ¹⁹F-NMR(CDCl₃) δ -68.1.
   IR(neat) 1555, 1530, 1302, 1199, 1154, 828, 708 cm⁻¹

### Reference Example 6

### 4-chloro-2-fluoroacetophenone (Compound No. 1-6)

(1) 4-chloro-2-fluorotoluene (25.7g, 0.177mol) and potassium permanganate (66.4g, 0.42mol) were added to water (750ml), and the mixture was refluxed for 4.5 hours. Manganese dioxide was filtered off under heating, and washed with warm water. The filtrate was concentrated to about 2/3 of volume, and neutralized with concentrated hydrochloric acid (pH 3) under cooling. The crystal separated was collected by filtration, washed with water, and dried to give 4-chloro-2-fluorobenzoic acid (11.0g).
   ¹H-NMR(DMSO-d₆) δ 7.39(1H, dd, J=8.45, 2.04Hz), 7.54(1H, dd, J=72.2, 2.03Hz), 7.88(1H, t, J=8.33Hz).
   m.p. :200-202 °C
(2) The compound prepared in reference example 6-(1) (17.4g, 0.1mol) was added to dichloroethane (80ml), and thionyl chloride (23.8g, 0.2mol) was dropwise added thereto. The reaction mixture was refluxed for 5 hours. After the reaction mixture was evaporated, toluene was added to the residue, and the insoluble materials were filtered off. The filtrate was evaporated to give acid chloride (oil, about 20g).
(3) Anhydrous magnesium chloride (6.7g, 70mmol) and triethylamine (24.2g, 0.24mol) were added to toluene (120ml), and dimethyl malonate (15.8g, 0.12mmol) was added dropwise under cooling with water. After the reaction mixture was stirred at room temperature for 3 hours, a toluene solution of the compound prepared in reference example 6-(2) (about 20g) in toluene (20ml) was added dropwise at about 20 °C. The resulting mixture was stirred for 14 hours at room temperature. After concentrated hydrochloric acid (20ml) was added to the reaction mixture under cooling with ice, the resulting mixture was added to ice water. The organic layer was separated, and the aqueous layer was further extracted with toluene. The toluene layer was combined with the former organic layer, washed with brine, dried, and then evaporated to give an oil. The oil was added to a mixture of DMSO (80ml) and water (4ml). The mixture was gradually heated, stirred at 140 °C (the temperature of oil bath) for 2 hours, and further stirred at 180 °C for 1 hour. After cooling, the reaction mixture was poured into ice water, and extracted with diethyl ether. The extract was washed with brine, dried, and then evaporated. The residue was distilled under reduced pressure to give 4-chloro-2-fluoroacetophenone (14.4g, oil).
   ¹H-NMR(CDCl₃ ) δ 2.63(3H, d, J=5.03Hz), 7.12-7.25(2H, m), 7.84(1H, t, J=9.02Hz).
   b.p.:100-104 °C/20mmHg

### Reference Example 7

### 4-chloro-2-fluorophenylboronic acid (Compound No. 1-7)

(1) Concentrated hydrochloric acid (35ml) and aniline (21.1 0.145mmol) were added to water (100ml), and to the solution was dropwise added aqueous solution (20ml) of sodium nitrite (11.2g, 0.16mol) under cooling with ice. The resulting mixture was stirred at the same temperature for 30 minutes. Then, under cooling with ice, the diazonium salt obtained above was gradually added to aqueous solution (50ml) of sodium iodide (24.0g, 0.16mol) with a dropping pipet. The resulting mixture was stirred at room temperature for 1 hour. Sodium hydrogensulfite (5g) was added to the reaction mixture, and the mixture was extracted with diethyl ether (3 times), washed with water, dried, and then evaporated. The residue was purified by silica gel column chromatography (hexane:ethyl acetate=10:1) to give 4-chloro-2-fluoro-1-iodobenzene (31.7g, oil).
   ¹H-NMR(CDCl₃) δ 6.88-6.98(1H, m), 7.09(1H, dd, J=2.28, 7.76Hz), 7.67(1H, m).
(2) Under nitrogen atmosphere, to a solution of the compound prepared in reference example 7-(1) (10.0g, 39mmol) in diethyl ether (150ml), was dropwise added n-butyl lithium (1.6M in hexane, 28.5ml, 45mmol) over 20 minutes. The mixture was stirred for 30 minutes at the same temperature. Then, a solution of trimethyl borate (4.86g, 46.8mol) in diethyl ether (25ml) was dropwise added thereto at -70 to -78 °C over 50 minutes. The mixture was stirred at the same temperature for 3 hours, after which time the reaction mixture was allowed to stand for raising the temperature up to room temperature overnight. Under cooling with water, 10% hydrochloric acid (90ml) was dropwise added to the reaction mixture, and the mixture was stirred at room temperature for 1 hour. The diethyl ether layer was separated, and the aqueous layer was extracted with diethyl ether. The ether layers were combined, washed with brine, dried, and evaporated. A small amount of petroleum ether was added to the residue, and the crystal separated was collected by filtration, and dried to give 4-chloro-2-fluorophenylboronic acid. Furthermore, the filtrate was evaporated, and the crystal was obtained by the same way.
   Yield:2.25g
   m.p.:>220 °C
   ¹H-NMR(DMSO-d₆) δ 7.10-7.35(2H, m), 7.58(1H, t, J=7.56Hz), 8.22(2H, br s)
   ¹⁹F-NMR(DMSO-d₆) δ -101.4

### Reference Example 8

### 4-Chloro-2-fluoro-5-methoxyphenylboronic acid (compound No. 1-8)

(1) 17ml of concentrated hydrochloric acid and 13.1g (74.6mmol) of aniline were added into 60ml of water. An aqueous solution (20ml) containing 5.6g (82mmol) of sodium nitrite was dropwise added to the mixture under cooling with ice, followed by stirring for 30 minutes. Thus obtained diazonium salt was portionwise added to an aqueous solution (15ml) containing 12.3g (82mmol) of sodium iodide under cooling with ice, followed by stirring at room temperature for 1 hour. 5g of sodium hydrogensulfite were added to the reaction mixture, followed by extraction with ether (3 times). The extract was washed with brine and dried. The solvent was removed off, and the residue was purified by silica gel column chromatography (hexane : ethyl acetate = 10:1) to obtain 10.6g of 4-chloro-2-fluoro-5-methoxy-1-iodobenzene. m.p.90-94°C
   ¹H-NMR(CDCl₃) δ 3.87(3H, s), 7.11(1H, d, J=7.12Hz), 7.21(1H, d, J=5.25Hz)
(2) 90ml of ether solution containing 6.01g (21mmol) of the compound obtained in the Reference Example 8(1) were stirred in the stream of nitrogen gas at -78°C, followed by dropwise addition of n-butyl lithium (1.5M in hexane, 15.0ml, 23mmol) for 15 minutes and stirring at the above temperature for 30 minutes. Then, 40ml of ether solution containing 2.6g (25mmol) of trimethyl borate were dropwise added at temperature between -70°C and -78°C for 1 hour and the mixture was stirred at the same temperature for 3 hours. The mixture was left to stand at room temperature overnight, whereby the temperature was raised to the room temperature. 60ml of 1N hydrochloric acid was dropwise added under water cooling with water and the mixture was stirred at room temperature for 1 hour. The ether layer was separated, and the aqueous layer was extracted with ether. The ether layers were combined, washed with brine, dried and concentrated by evaporation of solvent. A small amount of petroleum ether was added to the residue, and the deposited crystals were collected by filtration and dried to obtain 2.69g of 4-chloro-2-fluoro-5-methoxyphenylboronic acid.
   m.p.289-291°C
   ¹H-NMR(DMSO-d₆) δ 3.84(3H, s), 7.22-7.28(2H, m), 8.15-8.40(2H, br)
   ¹⁹F-NMR(DMSO-d₆) 6 -112.6
   IR(Nujol) 3522, 3350, 1603, 1053, 944, 878, 785 cm⁻¹

### Reference Example 9

### 4-Chloro-2-fluoro-5-methoxycarbonylphenylboronic acid (compound No. 1-9)

(1) 4.34g (30mmol) of 2-chloro-4-fluorotoluene and 0.084g (1.5mmol) of iron dust were mixed. 5.93g (37mmol) of bromine was dropwise added at room temperature over 10 minutes, and the resulting mixture was stirred at the same temperature for 2 hours. The reaction mixture was filtered through a Celite layer. Aqueous sodium sulfite aqueous (50ml) was added to the filtrate, and the mixture was washed with aqueous sodium bicarbonate solution (50ml) and brine, dried and concentrated to remove the solvents. The residue was purified by silica gel column chromatography (ethyl acetate : hexane = 15:1) to obtain 5-bromo-2-chloro-4-fluorotoluene (6.33g).
   ¹H-NMR(CDCl₃) δ 2.32(3H, s), 7.14(1H, d, J=8.2Hz), 7.41(1H, d, J=7.3Hz)
   ¹⁹F-NMR(CDCl₃) 6 -110.1
   IR(Nujol) 1598, 1571, 1485, 1260, 1072, 875, 770 cm⁻¹
(2) Ether solution (45mmol) containing the compound prepared by the method of the Reference Example 9 (1) (3.15g, 14mmol) was stirred at -78°C, followed by dropwise addition of n-butyl lithium (1.6M in hexane, 10.2ml, 16mmol) over 10 minutes, and the stirring was continued at the same temperature for 30 minutes. Then, trimethyl borate (1.77g, 17mmol) in ether (8ml) was dropwise added at the same temperature over 10 minutes, and the resulting mixture was stirred for 3 hours at the same temperature. The temperature of the mixture was gradually increased to the room temperature by standing overnight. 1N hydrochloric acid (50ml) was added with ice cooling and the mixture was stirred at room temperature for 1 hour. The ether layer was separated, and the aqueous layer was extracted with ether. The ether layers were combined, washed with brine, dried and concentrated by evaporation of the solvent. A small amount of petroleum ether was added to the residue, followed by filtration of deposited crystals. The crystals were dried to obtain 4-chloro-2-fluoro-5-methylphenylboronic acid (2.94g).
   m.p.250-253°C
   ¹H-NMR(DMSO-d₆) δ 2.29(3H, s), 7.23(1H, d, J=8.8Hz), 7.50(1H, d, J=6.6Hz), 8.23(2H, br s)
   ¹⁹F-NMR(DMSO-d₆) δ -106.1
   IR(Nujol) 3360, 1608, 1568, 1235, 1132, 1045, 944, 795 cm⁻¹
(3) To the end product of the Reference Example 9 (2) (7.74g, 41mmol) suspended in water 200ml was added sodium hydroxide (3.6g, 84mmol), followed by stirring at 35°C. A 250ml aqueous solution of potassium permanganate (14.7g, 93mmol) was dropwise added over 1 hour, and the mixture was stirred at 50°C for 7 hours. After cooling, the reaction mixture was filtered by the use of Celite to remove insolubles, and the filtrate was made acidic by addition of concentrated hydrochloric acid, followed by extraction with ethyl acetate (twice). The mixture was washed with brine solution, dried and concentrated by evaporation of the solvent. The residue was washed with petroleum ether to give 5-carboxy-4-chloro-2-fluorophenylboronic acid (8.19g).
   ¹H-NMR(DMSO-d₆) δ 7.41(1H, d, J=8.9Hz), 8.06(1H, d, J=6.6Hz), 8.35-8.55(2H, br), 13.10-13.50(1H, br)
   ¹⁹F-NMR(DMSO-d₆) δ -97.2
   IR(Nujol) 3300-2500, 1700, 1608, 1559, 1297, 1260 cm⁻¹
(4) Concentrated sulfuric acid (4.18g, 43mmol) was added to a methanol solution (25ml) containing the compound prepared in the Reference Example 9 (3) (4.15g, 19mmol), and the resulting mixture was refluxed under heating. After cooling, methanol was evaporated off and to the residue was added iced water. The mixture was extracted with ethyl acetate, washed brine solution, dried and concentrated by evaporation of the solvent to give 4-chloro-2-fluoro-5-methoxycarbonylphenylboronic acid (4.36g).
   m.p.201-203°C
   ¹H-NMR(DMSO-d₆) δ 3.86(3H, s), 7.45(1H, d, J=8.9Hz), 8.40-8.55(2H, br)
   ¹⁹F-NMR(DMSO-d₆) δ -96.2
   IR(Nujol) 3373, 1721, 1606, 1564, 1294, 1262, 1122, 1058 cm⁻¹

### Reference Example 10

### 4,5-Dichloro-6-isopropylpyrimidine (Compound No. 1-10)

(1) Formamidine hydrochloride (4.0g, 49.7mmol) was dissolved in methanol (50ml), followed by dropwise addition of sodium methoxide (9.6g, 49.7mmol, 28% in MeOH). Ethyl isobutyrylacetate (4.0g, 25.3mmol) was further dropwise added at room temperature and the resulting mixture was stirred at 40°C for 48 hours. After cooling, a diluted sulfuric acid (concentrated sulfuric acid 5.0g (51.0mmol) + water 2ml) was added, followed by reflux under heating for 5 hours. After cooling the reaction mixture was concentrated, neutralized with sodium hydrogencarbonate, and extracted with ethyl acetate (5 times). The extract was dried, and the solvent was evaporated off. The residue was crystallized from isopropyl ether, and the crystals were collected by filtration and dried to obtain 4-hydroxy-6-isopropylpyrimidine (2.0g).
   ¹H-NMR(CDCl₃) δ 1.29(6H, d, J=6.8Hz), 2.78-2.83(1H, m), 6.32(1H, s), 8.11(1H, s), 12.9(1H, br s)
(2) The compound obtained in the Reference Example 10 (1) (1.0g, 7.3mmol) was dissolved in acetic acid. After addition of antiformin (12ml) thereto, the mixture was stirred at room temperature. The reaction mixture was concentrated, and the residue was neutralized with aqueous sodium hydrogencarbonate, followed by extraction with ethyl acetate (5 times). The extract was dried and concentrated by evaporation of the solvent. The residue was purified by silica gel column chromatography (ethyl acetate: hexane = 2:1) to give 5-chloro-4-hydroxy-6-isopropylpyrimidine (0.8g).
   ¹H-NMR(CDCl₃) δ 1.23(6H, d, J=6.8Hz), 3.46-3.50(1H, m), 8.14(1H, s), 12.7(1H, br s)
(3) The compound obtained in the Reference Example 10 (2) (0.4g, 2.3mmol) was added to toluene (10ml), followed by addition of phosphorus oxychloride (4ml) and reflux under heating for 1 hour. After cooling the reaction mixture was concentrated, diluted with ethyl acetate, washed with aqueous sodium hydrogen carbonate, dried and concentrated by evaporation of the solvent. The residue was purified by silica gel column chromatography (ethyl acetate : hexane = 1:5) to give 4,5-dichloro-6-isopropylpyrimidine (0.35g).
   ¹H-NMR(CDCl₃) δ 1.29(6H, d, J=6.8Hz), 3.5-3.59(1H, m), 8.79(1H, s)

### Reference Example 11

### 4,5,6-trichloropyrimidine (Compound No. 1-11)

(1) 4,6-Dichloropyrimidine (5.00g, 32.6mmol) was added to 3N hydrochloric acid (50ml), and the reaction was carried out at 100°C for 1 hour. After cooling an aqueous ammonia solution was added so that the reaction mixture was made alkaline. The insolubles were collected by filtration on florisil, and washed well with water. The filtrate and the washing were combined and the resulting solution was made acidic with concentrated hydrochloric acid. After cooling the crystals were collected by filtration. The filtrate was extracted 10 times with ethyl acetate (30ml). The extracts were combined, dried and concentrated by evaporation of the solvent. The residue was combined with the crystals above obtained, washed with isopropyl ether and collected by filtration to give 4-chloro-6-hydroxypyrimidine (2.99g).
   ¹H-NMR(CDCl₃) δ 6.51(1H, s), 8.21(1H, s), 12.99(1H, br s)
   IR(Nujol) 2500-3150(br), 1668, 1588, 1340, 1397, 1070, 980 cm⁻¹
   m.p.: 194-195°C
(2) The compound obtained in the Reference Example 11(1) (1.92g, 14.7mmol) was dissolved in acetic acid 20ml, followed by heating at 80°C and dropwise addition of antiformin (20ml). After end of the dropwise addition, the mixture was stirred by at 80°C for 30 minutes. The reaction mixture was concentrated and the crystals were collected by filtration. The filtrate was extracted with ethyl acetate. The crystals obtained above were combined with the extract, followed by drying and concentration by evaporation of the solvent. Thus obtained crystals were washed with isopropyl ether and collected by filtration to give 4,5-dichloro-6-hydroxypyrimidine (1.29g).
   ¹H-NMR(DMSO-d₆) δ 8.22(1H, s), 13.4(1H, br s)
   IR(Nujol) 3262, 3070, 1694, 1646, 1625, 1584, 1389, 1054, 1045 cm⁻¹
   m.p.:not higher than 202°C
(3) The compound obtained in the Reference Example 11(2) (1.23g, 7.46mmol) was suspended in thionyl chloride (3ml), followed by addition of DMF (0.1ml, catalytic amount), and the reaction was carried out at 70°C to 80°C for 1.5 hours. The reaction mixture was concentrated and the residue was purified by silica gel short column chromatography (hexane : ethyl acetate = 1:1) to give 4,5,6-trichloropyrimidine (1.15g).
   ¹H-NMR(CDCl₃) δ 8.68(1H, s)
   IR(Nujol) 1514, 1406, 1323, 1237, 1056, 808, 754 cm⁻¹
   m.p.: 50-52°C

### Reference Example 12

### 4-chloro-5,6-dimethylpyrimidine (Compound No. 1-12)

(1) Sodium methoxide (17.5g, 28% in MeOH, 91mmol) was dropwise added to ethanol (55ml) solution of 2-methylacetoacetic acid ethyl ester (4.33g, 30mmol) and formamidine hydrochloride(3.62g, 45mmol), followed by refluxing under heating for 15 hours. After cooling diluted sulfuric acid (concentrated sulfuric acid 4.53g + water 4g) was added, followed by refluxing under heating for 1 hour. After cooling insolubles were filtered off and the filtrate was concentrated. Water was added to the residue, followed by neutralization to pH 7 with aqueous sodium hydrogen carbonate. The mixture was extracted 3 times with chloroform, and the extract was dried and concentrated by evaporation of the solvent. Diethyl ether was added to the residue, and the crystals were collected by filtration to obtain 4-hydroxy-5,6-dimethylpyrimidine (1.82g).
   m.p.120-121°C
   ¹H-NMR(DMSO-d₆) δ 1.91(3H, s), 2.20(3H, s), 7.92(1H, s), 12.20-12.40(1H, br)
   IR(Nujol) 1652, 1636, 1609, 1378, 1240, 1200, 1142, 861 cm⁻¹
(2) The product obtained in the Reference Example 12(1) (0.5g, 4mmol) was dissolved in acetonitrile (5ml), followed by addition of thionyl chloride (0.62g, 5.2mmol) and dimethylformamide (0.047g, 0.65mmol). The mixture was stirred at 50°C for 1 hour. After cooling the reaction mixture was concentrated, and the residue was neutralized with aqueous sodium hydrogen carbonate and extracted with chloroform. The extract was washed with brine solution and dried. The solvent was evaporated off, and the residue was purified by silica gel column chromatography (ethyl acetate : hexane = 1:5) to obtain 4-chloro-5,6-dimethylpyrimidine (0.23g).
   m.p.45-47°C
   ¹H-NMR(CDCl₃) δ 2.37(3H, s), 2.56(3H, s), 8.69(1H, s)
   IR(Nujol) 1561, 1532, 1206, 1032, 980, 869, 822, 754 cm⁻¹

### Reference Example 13

### 4-Chloro-5-methyl-6-trifluoromethylpyrimidine (Compound No. 1-13)

(1) Ethyl 2-methyltrifluoroacetoacetate (3.96g, 20mmol) and formamidine hydrochloride (1.96g, 24mmol) were dissolved in ethanol 30ml, followed by dropwise addition of sodium methoxide (4.63g, 28% in methanol, 24mmol) under cooling with iced water. The mixture was stirred at room temperature for 2 hours, and refluxed under heating for 13 hours. After cooling the reaction mixture was concentrated. Water was added to the residue and the mixture was neutralized with 1N hydrochloric acid. The mixture was extracted with chloroform, followed by drying and evaporation of the solvent. The residue was washed with petroleum ether and isopropyl ether, and the crystals were collected by filtration and dried to give 4-hydroxy-5-methyl-6-trifluoromethylpyrimidine (0.47g).
   m.p.182-184°C
   ¹H-NMR(DMSO-d₆) δ 2.10(3H, s), 8.22(1H, s), 12.80-13.20(1H, br)
   IR(Nujol) 1678, 1612, 1566, 1148, 1036, 826 cm⁻¹
(2) Thionyl chloride (3.26g, 27mmol) and dimethylformamide (0.28g, 3.7mmol) were added to the compound obtained in the Reference Example 13(1), and the mixture was stirred at 50°C for 1 hour and further at 80°C for 1 hour. After cooling the reaction mixture was concentrated, and the residue was neutralized with aqueous sodium hydrogen carbonate, and extracted with chloroform. The extract was washed with brine solution, dried and concentrated by evaporation of the solvent. The residue was purified by silica gel column chromatography (ethyl acetate : hexane = 1:5) to give 4-chloro-5-methyl-6-trifluoromethylpyrimidine (1.43g, oil).
   ¹H-NMR(CDCl₃) δ 2.55(3H, s), 8.95(1H, s)
   IR(neat) 1560, 1364, 1327, 1242, 1143, 1021, 712 cm⁻¹

### Reference Example 14

### 4-Chloro-6-difluoromethyl-5-methylpyrimidine (Compound No. 1-14)

(1) Ethyl 2-methyldifluoroacetoacetate (12.16g, 68mmol) and formamidine hydrochloride (6.53g, 81mmol) were dissolved in ethanol (130mmol), followed by dropwise addition of sodium methoxide (15.8g, 28% in methanol, 82mmol) under cooling with iced water. The mixture was stirred at room temperature for 2 hours and refluxed under heating for 20 hours. After cooling, the reaction mixture was concentrated, followed by addition of water (60ml) to the residue and neutralization with 1N hydrochloric acid. The crystals were collected by filtration, washed with water and dried. The filtrate was extracted with ethyl acetate, dried and concentrated by evaporation of the solvent. The residue was washed with diethyl ether, and the crystals were collected by filtration, followed by drying to give 6-difluoromethyl-4-hydroxy-5-methylpyrimidine. Total yield 9.55g
   m.p.166-167°C
   ¹H-NMR((DMSO-d₆) 6 2.06(3H, s), 6.94(1H, t, J=53.4Hz), 8.15(1H, s), 12.80 (1H, br s)
   ¹⁹F-NMR(DMSO-d₆) 6 -118.1
   IR(Nujol) 3097, 3029, 1691, 1651, 1620, 1572, 1048, 859, 780 cm⁻¹
(2) Thionyl chloride (4.64g, 39mmol) and dimethylformamide (0.51g, 7mmol) were added to the compound obtained in the Reference Example 14(1), followed by stirring at 50°C for 1 hour and further at 80°C for 1 hour. After cooling, the reaction mixture was concentrated and the residue was neutralized with aqueous sodium hydrogen carbonate, followed by extraction with chloroform. The extract was washed with brine solution, dried and concentrated by the evaporation of the solvent. The residue was purified by silica gel column chromatography (ethyl acetate : hexane = 1:7) to obtain 4-chloro-6-difluoromethyl-5-methylpyrimidine (2.23g, oil).
   ¹H-NMR(CDCl₃) δ 2.55(3H, s), 6.64(1H,t,J=53.9Hz), 8.88(1H, s)
   ¹⁹F-NMR(CDCl₃) δ -115.9
   IR(neat) 1548, 1361, 1210, 1107, 1053, 820, 789, 686 cm⁻¹

### Reference Example 15

### 4,6-Dichloro-5-methylpyrimidine (Compound No. 1-15)

(1) Diethyl methylmalonate (26.0g, 0.15mol) and formamidine hydrochloride (14.4g, 0.18mol) were dissolved in ethanol (150ml). Sodium methoxide (108g, 0.56mmol, 28% in MeOH) was dropwise added to the above solution and the mixture was refluxed under heating for 8 hours. After cooling, the reaction mixture was concentrated, and water was added to the residue, followed by neutralization with concentrated hydrochloric acid (pH value = 4). The crystals were collected by filtration, washed with water and dried to obtain 4,6-dihydroxy-5-methylpyrimidine (0.41g).
   ¹H-NMR((DMSO-d₆) δ 1.73(3H, s), 7.87(1H, s), 11.55(br, s)
(2) The compound obtained in the Reference Example 15(1) (1.26g, 10mmol) was added to phosphorus oxychloride (8ml), followed by stirring at 80°C for 2 hours and 100°C for 1 hour. After cooling, water 100ml was portionwise added to the reaction mixture and the resulting mixture stirred at the same temperature for 30 minutes, followed by extraction with chloroform (twice). The extract was washed with water, dried and concentrated by evaporation of the solvent. The residue was purified by silica gel colum chromatography (ethyl acetate : hexane = 1:5) to obtain 4,6-dichloro-5-methylpyrimidine (1.15g).
   m.p. 54-56 °C
   ¹H-NMR(CDCl₃) δ 2.50(3H, s), 8.62(1H, s)

### Reference Example 16

### 4-Chloro-5-cyano-6-methoxypyrimidine (Compound No. 1-16)

(1) Phosphorus oxychloride (22ml, 236.5mmol) was dropwise added to DMF (7ml, 90.1mmol) at 10°C. The mixture was stirred for 30 minutes at the same temperature, followed by adding portionwise 4,6-dihydroxypyrimidine (4.8g, 42.9mmol). The mixture was stirred at room temperature for 2 hours and refluxed under heating for 3 hours. After cooling, the reaction mixture was portionwise added to iced water, and the mixture was extracted 3 times by ether. The ether extracts were combined, washed with aqueous sodium hydrogen carbonate, dried and concentrated by evaporation of the solvent. Hexane was added to the residue, and the crystals were collected by filtration to give 4,6-dichloro-5-formylpyrimidine (4.5g).
   ¹H-NMR(CDCl₃) δ 8.90(1H, s), 10.4(1H, s)
(2) The compound obtained in the Reference Example 16(1) (0.2g, 1.13mmol) and hydroxylamine hydrochloride (0.1g, 1.44mmol) were dissolved in acetonitrile (5ml), followed by addition of sodium acetate (0.1g, 1.22mmol). The mixture was stirred at 40°C for 3 hours. The reaction mixture was concentrated and water was added to the residue. The precipitated crystals were collected by filtration and dried to give 4,6-dichloro-5-hydroxyiminopyrimidine (0.1g). ¹H-NMR(CDCl₃) δ 8.33(1H, s), 8.57(1H, s), 8.76(1H, s)
(3) The compound obtained in the Reference Example 16(2) (0.3g, 2,87mmol) was dissolved in thionyl chloride (4ml), followed by refluxing under heating for 1 hour. After cooling, excess of thionyl chloride was evaporated off, and the residue was dissolved in ethyl acetate. The ethyl acetate solution was washed with aqueous sodium hydrogen carbonate, dried and concentrated by the evaporation of the solvent. Pentane was added to the residue. The resultant crystals were collected by filtration and dried to obtain 4,6-dichloro-5-cyanopyrimidine (0.15g).
(4) The compound obtained in the Reference Example 16(3) (0.5g, 2.87mmol) was dissolved in methanol (20ml) and sodium hydrogen carbonate (0.25g, 2.98mmol) was added to the solution. The mixture was stirred at room temperature for 12 hours. Water was added to the reaction mixture and the precipitated crystals were collected by filtration, followed by washing with water and drying to give 4-chloro-5-cyano-6-methoxypyrimidine (0.2g).
   ¹H-NMR(CDCl₃) δ 4.17(3H, s), 8.71(1H, s)

### Reference Example 17

### 4-Chloro-5-cyano-6-difluoromethylpyrimidine (Compound No. 1-17)

(1) Sodium methoxide (19.5 g, 0.1 mol) was dissolved in methanol (50 ml) to a concentration of 28%, and to the solution were added methyl cyanoacetate 10g (0.1 mol) and ethyl difluoroacetate 12.5 g (0.1 mol). The mixture was stirred at 60°C for 12 hours, cooled, and concentrated to give an oil. The oil was repeatedly washed with diethyl ether to precipitate crystals. The crystals were filtered off, washed with diethyl ether and dried to give methyl 2-cyano-4,4-difluoro-3-hydroxycrotonate sodium salt (22 g).
(2) The sodium salt (3.0 g) (15 mmol) prepared in the Reference Example 17(1) was suspended in toluene (10 ml), and phosphorous pentoxide 3.1 g (15 mmol) was added. The mixture was stirred at 45°C for one hour, and phosphorous pentoxide 1.5 g (7.5 mmol) was further added. After stirring at 55°C for 2 hours, the reaction mixture was filtered through a Celite and the filtrate was concentrated. The resultant residue was purified by distillation under reduced pressure (b.p.90-100°C/40mmHg) to give methyl 3-chloro-2-cyano-4,4-difluorocrotonate (1.4 g).
   ¹HNMR(CDCl₃) δ 3.95 (3H, s), 7.49 (1H, t, J=53.6Hz)
(3) Formamidine hydrochloride 6.2 g (77 mmol) was dissolved in methanol (100 ml), and 28% sodium methoxide (53 g, 0.275 mol) in methanol was added. The product 11 g (70 mmol) obtained in the Reference Example 17(2) was dropwise added thereto under ice-cooling. The resultant mixture was stirred at 55°C for 5 hours, cooled, concentrated, neutralized with dilute hydrochloric acid, and extracted with ethyl acetate. The extract was washed with brine, dried, and concentrated by evaporation of the solvent to give a residue. The residue was crystallized from ether and the crystals were collected by filtration, washed, and dried to give 5-cyano-6-difluoromethyl-4-hydroxypyrimidine (7.6 g).
   ¹HNMR(CDCl₃) δ 6.94 (1H, t, J=52.8Hz), 8.63 (1H, s), 14.00 (1H, br, s)
(4) 4-Hydroxypyrimidine 0.3 g (1.75 mmol) prepared in the Reference Example 17(3) was suspended in toluene (5 ml), and to the suspension were added phosphorous oxychloride (0.5 ml) and dimethylformamide (one drop). The mixture was stirred at 80°C for 1 hr, and then cooled. After removal of the insolubles by decantation, the toluene solution was concentrated. The residue was purified by silica gel column chromatography (hexane : ethyl acetate = 1 : 1) to give 4-chloro-5-cyano-6-difluoromethylpyrimidine (0.2 g). ¹HNMR(CDCl₃) 6 6.72 (1H, t, J=53.1Hz), 9.23 (1H, s)

### Reference Example 18

### 4-Chloro-5-cyano-6-methylpyrimidine (Compound No. 1-18)

(1) Formamidine hydrochloride 1.5 g (18.6 mmol) was dissolved in methanol (20 ml), and to the solution was added 28% sodium methoxide (7.0 g, 36.3 mol) in methanol. A solution of methyl 2-cyano-3-methoxycrotonate 3.0 g (17.8 mmol) in methanol (20 ml) was dropwise added to the reaction mixture under ice-cooling, and the mixture was stirred at the same temperature for 2 hours. The reaction mixture was concentrated, diluted with water, neutralized with concentrated hydrochloric acid, and extracted with ethyl acetate. The extract was dried and concentrated.
   Crystals precipitated upon addition of ether to the residue were collected by filtration, and dried to give 5-cyano-4-hydroxy-6- methylpyrimidine (1.5 g).
   ¹HNMR(DMSO-d₆) δ 2.43 (3H, s), 8.40 (1H, s), 13.30 (1H, br, s)
(2) 4-Hydroxypyrimidine 6.8 g (50.4 mmol) was suspended in phosphorous oxychloride (25 ml), and the suspension was stirred at 100°C for 30 minutes. After cooling, the reaction mixture was concentrated and the residue was diluted with ethyl acetate, then washed with water. The ethyl acetate layer was dried and concentrated. The residue was dissolved in hexane and the insolubles were removed by filtration. The filtrate was concentrated to give 4-chloro-5-cyano-6-methylpyrimidine (2.7 g).
   ¹HNMR(CDCl₃) δ 2.80 (3H, s), 9.00 (1H, s)

### Example 1

### 4-(4-Chloro-2-fluorophenyl)-5-chloro-6-methylpyrimidine (Compound No. E-1)

(1) A mixture of compound no.1-6 (7.0g, 40.5mmol) and dimethylacetamide dimethylacetal (24ml) was stirred at 80 °C for 4 hours. (Methanol generated halfway was evaporated by aspirator.) After cooling, petroleum ether was added to the reaction mixture, and stirred, and then the supernatant was decanted (4 times). The decanted solution was allowed to stand overnight, and the separated crystals were collected by filtration, and dried to give enaminone compound (4.9g).
   ¹H-NMR(CDCl₃) δ 2.66(3H, s), 3.06(6H, s), 5.51(1H, d, J=1.91Hz), 7.03-7.23(2H, m), 7.69(1H, t, J=8.06Hz).
(2) To a solution of the compound prepared in Example 1(1) (4.4g, 18.2mmol) in DMF (35ml) was gradually added NCS (2.8g, 20.9mmol) at about 20 °C or so, and the resulting mixture was stirred at room temperature for 30 minutes. The reaction mixture was added to brine solution, extracted with ethyl acetate, washed with water, dried, and then evaporated to give α-chloroenaminone.
(3) To a solution of the compound (total amount) prepared in Example 1(2) in ethanol (90ml) was immediately added formamidine hydrochroride (1.7g, 21.1mmol), and 28% sodium methylate (4.2g, 21.7mmol) was further added dropwise at about 15 °C or so. The resulting mixture was gradually heated to reflux for 1.5 hours. After cooling, the reaction mixture was evaporated, and the residue was dissolved in ethyl acetate. The solution was washed with water, dried, and then evaporated. The residue was purified by silica gel column chromatography (hexane:acetone=2:1), to give 4-(4-chloro-2-fluorophenyl)-5-chloro-6-methylpyrimidine (2.1g).
   m.p.:77-79 °C
   ¹H-NMR(CDCl₃) δ 2.72(3H, s), 7.18(3H, m), 9.01(1H, s)

### Example 2

### 4-(4-chloro-2-fluoro-5-isopropoxyphenyl)-5-bromo-2-methylpyrimidine (Compound No. G-2)

(1) A mixture of 4-chloro-2-fluoro-5-methoxyacetophenone (2.0g, 10mmol) and dimethylformamide dimethylacetal (6ml) was stirred at 70 °C for 4 hours and at 80 °C for 8 hours. Methanol by-produced during the reaction was removed by evaporation by with an aspirator. After cooling, hexane was added to the reaction mixture, and the separated crystal was collected by filtration, and dried to give enaminone compound (2.3g).
   ¹H-NMR(CDCl₃) δ 2.92(3H, s), 3.16(3H, s), 3.92(3H, s), 5.67(1H, d, J=12.17Hz), 7.13-7.41(1H, d, J=6.37Hz), 7.81(1H, d)
(2) To a solution of the compound prepared in Example 2(1) (1.1g, 3.9mmol) in DMF (10ml), was gradually added NBS(0.7g, 3.9mmol) at about 20 °C. The resulting mixture was stirred at room temperature for 2 hours, and poured into ice water, to precipitate crystals, which were collected by filtration and dried to give α-bromoenaminone (1.1g).
   ¹H-NMR(CDCl₃) δ 3.25(6H, s), 3.88(3H, s), 6.89(1H, d, J=5.73Hz), 7.14(1H, d, J=8.34Hz), 7.31(1H, d, J=2.33Hz)
(3) To a solution of the compound prepared in Example 2(2) (1.1g, 3.26mmol) and acetamidine hydrochloride (0.4g, 4.2mmol) in ethanol (15ml) was dropwise added 28% sodium methylate (0.82g, 4.2mmol) under cooling with ice. The resulting mixture was stirred at 50 °C for 1 hour, and heated under reflux for 3 hours. After cooling, the reaction mixture was evaporated, and the residue was dissolved in chloroform. The solution was washed with water, dried, and evaporated to remove the solvent. The residue was purified by silica gel column chromatography (hexane:ethyl acetate=1:1) to give 4-(4-chloro-2-fluoro-5-methoxyphenyl)-5-bromo-2-methylpyrimidine(0.9g).
   m.p.:106-107 °C
   ¹H-NMR(CDCl₃) δ 2.75(3H, s), 3.92(3H, s), 6.95(1H, d, J=6.00Hz), 7.26(1H, d, J=8.81Hz).
(4) The compound prepared in Example 2(3) (0.8g, 2.4mmol) was added to 47% hydrobromic acid (2.5ml), and the resulting mixture was stirred at 130 °C for 2 hours. After cooling, water was added to the reaction mixture, and the separated crystals were collected by filtration, and washed with water. The crystals were dissolved in chloroform, dried, and then evaporated. A mixture of hexane:ethyl acetate=5:1 in a small amount was added to the residue, and the separated crystal was collected by filtration, and dried to give 4-(4-chloro-2-fluoro-5-hydroxyphenyl)-5-bromo-2-methylpyrimidine (0.4g).
   ¹H-NMR(CDCl₃) δ 2.75(3H, s), 5.75(1H, br s), 7.08(1H, d, J=6.35Hz), 7.20(1H, d, J=8.75Hz), 8.81(1H, s)
(5) To a solution of the compound prepared in Example 2(4) (0.15g, 0.47mmol) and potassium carbonate (0.08g, 0.56mml) in DMF (5ml) was added 2-iodopropane (0.095g, 0.56mmol) under cooling with ice. The resulting mixture was stirred at room temperature for 14 hours. The reaction mixture was added to ice water, extracted with ethyl acetate, washed with water, dried, and then evaporated. The residue was purified by silica gel column chromatography (hexane:ethyl acetate=1:1) to give 4-(4-chloro-2-fluoro-5-isopropoxyphenyl)-5-bromo-2-methylpyrimidine (95mg, oil).
   ¹H-NMR(CDCl₃)δ 1.39(6H, d, J=6.08Hz), 2.75(3H, s), 4.52(1H, septet, J=6.04Hz), 6.98(1H, d, J=7.27Hz), 7.24(1H, d), 8.81(1H, s).
   ¹⁹F-NMR(CDCl₃) δ -121.16

### Example 3

### 5-chloro-4-(2,4-difluorophenyl)-6-trifluoromethylpyrimidine (Compound No. A-35)

4,5-dichloro-6-trifluoromethylpyrimidine (4.3g, 20mmol), 2,4-difluorophenylboronicacid (2.84g, 18mmol), dichlorobis(triphenylphosphine)palladium(II) (1.0g, 1.5mmol) and sodium hydrogencarbonate (5.5g, 65mmol) were added to a mixture of dimethoxyethane (180ml) and water (35ml). The mixture was heated under reflux for 3 hours. After cooling, the reaction mixture was neutralized to pH 5 with 2N-hydrochloric acid, and the dimethoxyethane was removed by evaporation. The residue was dissolved in chloroform, and the solution was washed with water, dried, and then evaporated. The residue was purified by silica gel column chromatography (hexane:ethyl acetate=5:1) to give 5-chloro-4-(2,4-difluorophenyl)-6-trifluoromethylpyrimidine (3.1g).
m.p.:61-63 °C
¹H-NMR(CDCl₃) δ 6.92-7.17(2H, m), 7.45-7.60(1H, m), 9.27(1H, s).
¹⁹F-NMR(CDCl₃) δ -68.06, -105.30, -107.26

### Example 4

### 5-Chloro-4-(4-chloro-2-fluoro-5-propargyloxyphenyl)-6-trifluoromethylpyrimidine (Compound No. A-3)

(1) 4,5-Dichloro-6-trifluoromethylpyrimidine (1.3g, 6mmol), 4-chloro-2-fluoro-5-methoxyphenylboronic acid (1.29g, 6.3mmol), dichlorobis (triphenylphosphine) palladium(II) (0.29g, 6.3mmol) and sodium hydrogencarbonate (1.64g, 20mmol) were added to a mixture of dimethoxyethane (50ml) and water (10ml). The resulting mixture was refluxed for 2 hours. After cooling, the reaction mixture was adjusted to pH 4 with 1N-hydrochloric acid and then concentrated. Water (60ml) was added to the residue, and the mixture was extracted with chloroform, washed with water, dried, and then concentrated. The residue was purified by silica gel column chromatography (hexane:ethyl acetate=10:1) to give 5-chloro-4-(4-chloro-2-fluoro-5-methoxyphenyl)-6-trifluoromethylpyrimidine (1.42g, compound no. A-1). m.p.:109-111°C
   ¹H-NMR(CDCl₃) δ 3.94(3H, s), 7.01(1H, d, J=6.0Hz), 7.31(1H, d, J=9.0Hz), 9.28(1H, s).
   ¹⁹F-NMR(CDCl₃) δ -68.1, -120.2.
(2) The compound prepared in Example 4 (1) (1.40g, 4.1mmol was added to a mixture of acetic acid (10ml) and 47% hydrobromic acid (30ml), and the mixture was stirred at 120 °C for 8.5 hours. After cooling, the reaction mixture was added to ice water, extracted with ethyl acetate, washed with water, dried, and then concentrated. The residue was purified by silica gel column chromatography (hexane:ethyl acetate=4:1), to give 5-chloro-4-(4-chloro-2-fluoro-5-hydroxyphenyl)-6-trifluoromethylpyrimidine (1.01g).
   m.p.:130-132 °C
   ¹H-NMR(CDCl₃) δ 5.68-5.90(1H, br s), 7.15(1H, d, J = 6.2Hz), 7.26(1H, d, J=8.8Hz), 9.28(1H, s).
   ¹⁹F-NMR(CDCl₃) δ -68.1, -120.8.
   IR(Nujol) 1622, 1558, 1293, 1206, 1158, 986, 886, 768 cm⁻¹
(3) The compound prepared in Example 4(2) (0.17g, 0.52mmol) and potassium carbonate (0.93g, 0.67mmol) were added to DMF (12ml). To this was added propargylbromide (0.13g, 1.1mmol), and the resulting mixture was stirred at 40 °C for 1.5 hours. The reaction mixture was poured into ice water, and extracted with ethyl acetate. The extract was washed with water, dried, and then concentrated. The residue was purified by preparative thin layer chromatography (hexane:ethyl acetate=5:1) to give 5-chloro-4-(4-chloro-2-fluoro-5-propargyloxyphenyl)-6-trifluoromethylpyrimidine (1.01g).
   m.p.:112-113 °C
   ¹H-NMR(CDCl₃) δ 2.58(1H, m), 4.81(2H, d, J=2.4Hz), 7.21(1H, d, J=6.0Hz), 7.33(1H, d, J=9.0Hz), 9.28(1H, s).
   ¹⁹F-NMR(CDCl₃) δ -68.0, -118.8.
   IR(Nujol) 1612, 1556, 1190, 1144, 1030, 870 cm⁻¹

### Example 5

### 5-chloro-4-[4-cyano-5-(N-ethanesulfonylamino)-2-fluorophenyl]-6-trifluoromethylpyrimidine (Compound No. A-16)

(1) The compound prepared in Example 3 (2.38g, 8.07mmol) was added to concentrated sulfuric acid (19ml) under cooling with ice, and the resulting mixture was stirred for 30 minutes. Fuming nitric acid (0.35ml) was added to the mixture, and the resulting mixture was stirred at room temperature for 1 hour, after which time fuming nitric acid (0.2ml) was further added. The mixture was stirred at room temperature for 1 hour, and then poured into ice water. The reaction mixture was extracted twice extracted with ethyl acetate, and the extract was washed with water, dried, and evaporated. The residue was dissolved in hexane:ethyl acetate=1:1, and the solution was filtered by silica gel layer of grass filter. The filtrate was concentrated to give 5-chloro-4-(2,4-difluoro-5-nitrophenyl)-6-trifluoromethylpyrimidine (2.5g).
   m.p.:90-92 °C
   ¹H-NMR(CDCl₃) δ 7.18-7.32(1H, m), 8.35-8.47(1H, m), 9.32(1H, s).
   ¹⁹F-NMR(CDCl₃) 6 -68.07, -96.25, -107.47.
(2) The compound prepared in Example 5(1) (2.55g, 7.5mmol) and potassium cyanide (0.73g, 1.2mmol) was dissolved in DMF (20ml), and this solution was stirred at 50 °C for 3.5 hours. After cooling, the reaction mixture was added to ice water, extracted with ethyl acetate, washed with water (brine), dried, and then evaporated. The residue was purified by silica gel column chromatography (hexane:ethyl acetate=5:1) to give 5-chloro-4-(4-cyano-5-nitro-2-fluorophenyl)-6-trifluoromethylpyrimidine (1.2g).
   m.p.:90-92 °C
   ¹H-NMR(CDCl₃) δ 7.81(1H, d, J=8.15Hz), 8.59(1H, d, J=5.59Hz), 9.37(1H, s)
(3) The compound prepared in Example 5(2) (1.2g, 3.46mmol) was added to a mixture of acetic acid (18ml) and water (6ml). Reduced iron(0.6g, 10.7mmol) was added to the mixture at room temperature. The resulting mixture was stirred at the same temperature for 3 hours. After addition of ethyl acetate (50ml) to the reaction mixture, the insolubles were filtered off. The filtrate was concentrated, and water was added to the residue. The resulting solution was made alkaline with 2N-sodium hydroxide, extracted with ethyl acetate. The extract was washed with water, dried, and then evaporated. The residue was purified by silica gel column chromatography (hexane:ethyl acetate=1:1) to give 4-(5-amino-4-cyano-2-fluorophenyl)-5-chloro-6-trifluoromethylpyrimidine (0.54g).
   m.p. :170-172 °C
   ¹H-NMR(CDCl₃) δ 4.47(2H, br. s), 6.82(1H, d, J=5.52Hz), 7.28(1H, d), 9.27(1H, s).
   ¹⁹F-NMR(CDCl₃ ; δ) -68.07, -125.97.
   IR(Nujol) 3491, 3358, 2224, 1640, 1377, 1145, 1021 cm⁻¹
(4) The compound prepared in example 5(3) (0.54g, 1.7mmol) and triethylamine (0.46g, 4.6mmol) were dissolved in dichloroethane (5ml). After addition of ethanesulfonyl chloride (0.48g, 3.75mmol) thereto under cooling with ice, the mixture was stirred at room temperature for 14 hours. Triethylamine (0.42ml) and ethanesulfonyl chloride (0.18ml) were further added to the reaction mixture. The mixture was stirred at room temperature for 7 hours and at 50 °C for 2 hours. After cooling, the reaction mixture was poured into ice water, extracted with ethyl acetate. The extract was washed with water, dried, and evaporated. The residue was purified by silica gel column chromatography (hexane:ethyl acetate=2:1) to give 4-[5-(bisethanesulfonylamino)-4-cyano-2-fluorophenyl]-5-chloro-6-trifluoromethylpyrimidine (0.66g).
   m.p.:191-194 °C
   ¹H-NMR(CDCl₃ ) δ 1.54(6H, t), 3.73(2H, q), 3.74(2H, q), 7.62-7.75(2H, m), 9.33(1H, s).
(5) The compound prepared in Example 5(4) (0.3g, 0.61mmol) was dissolved in dioxane (2ml), and to this solution was added 2N-sodium hydroxide (0.65ml) under cooling with ice. The solution was stirred at room temperature for 1 hour, poured into ice water, and neutralized with 2N-hydrochloric acid to precipitate crystals, which were collected by filtration, washed with water, and dried to give 5-chloro-4-[4-cyano-5-(N-ethanesulfonylamino)-2-fluorophenyl]-6-trifluoromethylpyrimidine (0.66g).
   m.p.:191-194 °C
   ¹H-NMR(CDCl₃) δ 1.48(3H, s), 3.25(2H, q, J=7.41Hz), 7.52(1H, d, J=8.19Hz), 7.91(1H, d, J=5.87Hz), 9.31(1H,s)

### Example 6

### 5-chloro-4-[4-cyano-5-(N-ethanesulfonyl-N-propargylamino)-2-fluorophenyl]-6-trifluoromethylpyrimidine (Compound No. A-21)

To a suspension of compound no. A-5 (0.14g, 0.45mmol) and potassium carbonate (0.083g, 0.6mmol) in DMF (2ml) was added propargylbromide (0.07g, 0.6mmol). The mixture was stirred at room temperature for 4 hours. Ice water was added to the reaction mixture, and the mixture was extracted with ethyl acetate. The extract was washed with water, dried, and evaporated. The residue was purified by silica gel column chromatography (hexane:ethyl acetate=2:1) to give 5-chloro-4-[4-cyano-5-(N-ethanesulfonyl-N-propargylamino)-2-fluorophenyl]-6-trifluoromethylpyrimidine (0.14g, oil).
¹H-NMR(CDCl₃) δ 1.52(3H, t, J=7.38Hz), 2.42(1H, t, J=2.44Hz), 3.31(2H, q, J=7.40Hz), 4.54(2H, d, J=2.47Hz), 7.62(1H, d, J=8.38Hz), 7.87(1H, d, J=6.17Hz), 9.32(1H, s)

### Example 7

### 4-[5-(N-acetyl-N-ethanesulfonylamino)-4-cyano-2-fluorophenyl]-5-chloro-6-trifluoromethylpyrimidine (Compound No. A-23)

Compound no. A-5 (0.18g, 0.44mmol) and triethylamine (0.06g, 0.58mmol) were dissolved in acetonitrile (7ml), and to this was added acetyl chloride (0.044g, 0.56mmol) at room temperature. The solution was stirred at the same temperature for 2.5 hours. The reaction mixture was concentrated, and water was added to the residue. The mixture was extracted with ethyl acetate, washed with water, dried, and then concentrated. The residue was purified by silica gel column chromatography (chloroform:methanol=50:1) to give 4-[5-(N-acetyl-N-ethanesulfonylamino)-4-cyano-2-fluorophenyl]-5-chloro-6-trifluoromethylpyrimidine (0.18g).
m.p. :144-146 °C
¹H-NMR(CDCl₃) δ 1.58(3H, t, J=7.5Hz), 2.18(3H, s), 3.70-4.05(2H, m), 7.69(1H, d, J=8.3Hz), 7.75(1H, d, J=6.0Hz), 9.33(1H, s).
¹⁹F-NMR(CDCl₃) δ -68.1, -105.9.
IR(Nujol) 2238, 1556, 1340, 1152, 863 cm⁻¹

### Example 8

### 5-Chloro-4-(7-fluoro-3-oxo-4-propargyl-2H-1,4-benzoxazine-6-yl)-6-trifluoromethylpyrimidine (Compound No. A-37)

(1) The compound prepared in Example 5(1) (1.02g, 3mmol) and ethyl glycolate (0.83g, 8mmol) were dissolved in dioxane (8ml). Then, potassium fluoride (0.7g, 12mmol) was added to the reaction mixture, and the mixture was refluxed for 7 hours. After cooling, the reaction mixture was evaporated, and water was added to the residue. The mixture was extracted with ethyl acetate, and the extract was washed with water, dried and evaporated. The residue was purified with silica gel column chromatography (hexane:ethyl acetate=4:1) to give ethyl [5-fluoro-2-nitro-4-(5-chloro-6-trifluoromethylpyrimidin-4-yl)phenoxy]acetate (0.18g).
   m.p.:127-129 °C
   ¹H-NMR(CDCl₃) δ 1.33(3H, t, J=7.1Hz), 4.33(2H, q, J=7.1Hz), 4.87(2H, s), 6.83(1H, d, J=10.8Hz), 8.25(1H, d, J=4.2Hz), 9.29(1H, s)
   ¹⁹F-NMR(CDCl₃) δ -68.1, -98.8
   IR(Nujol) 1743, 1637, 1538, 1230, 1149, 1077, 848, 646 cm⁻¹
(2) To a mixture of acetic acid (2ml) and water (2ml) was added reduced iron (0.9g, 16mmol) and the mixture was stirred at 50°C. To the mixture was dropwise added a solution prepared by dissolving the compound prepared in Example 8(1) (0.97g, 2.3mmol) in a mixture of acetic acid (9ml) and ethyl acetate (5ml). The mixture was stirred at 60°C for 1 hour. After cooling, the insolubles in the reaction mixture was removed, and the filtrate was extracted with ethyl acetate. The extract was washed with aqueous sodium bicarbonate and water, dried and evaporated. The residue was purified with silica gel column chromatography (hexane:ethyl acetate=2:1) to give 5-chloro-4-(7-fluoro-3-oxo-2H-1,4-benzoxazin-6-yl)-6-trifluoromethylpyrimidine (0.75g).
   m.p.223-225°C
   ¹H-NMR(CDCl₃) δ 4.72(2H, s), 6.88(1H, d, J=10.0Hz), 6.98(1H, d, J=6.4Hz), 9.25(1H, s)
   ¹⁹F-NMR(CDCl₃) δ -68.0, -114.5
   IR(Nujol) 1694, 1505, 1324, 1203, 1144, 1052, 776, 646 cm⁻¹
(3) The compound prepared in Example 8(2) (0.35g, 1.0mmol) and potassium carbonate (0.28g, 2mmol) were suspended in DMF (5ml). To the suspension were added propargyl bromide (0.25g, 2.1mmol) at room temperature, and the mixture was stirred at 50°C for 3 hours. After cooling, water was added to the reaction mixture, and extracted with ethyl acetate, washed with water, dried and evaporated. The residue was purified with silica gel column chromatography(hexane:ethyl acetate=2:1) to give 5-chloro-4-(7-fluoro-3-oxo-4-propargyl-2H-1,4-benzoxazin-6-yl)-6-trifluoromethylpyrimidine (0.29g).
   m.p.:158-159 °C
   ¹H-NMR(CDCl₃) δ 2.30(1H, m), 4.73(2H, s), 4.75(2H, s), 6.92(1H, d, J=9.9Hz), 7.33(1H, d, J=6.4Hz), 9.28(1H, s)
   ¹⁹F-NMR(CDCl₃) δ -68.0, -114.9
   IR(Nujol) 1698, 1514, 1212, 1154, 1054, 869 cm⁻¹

### Example 9

### 5-Chloro-4-(4-chloro-2-fluoro-5-isopropoxyphenyl)-6-methylpyrimidine (Compound No. E-2)

(1) To a mixture of dimethoxyethane (25ml) and water(5m1) were added 4,5-dichloro-6-methylpyrimidine (0.5g, 3.06mmol), 4-chloro-2-fluoro-5-methoxyphenylboronic acid (0.63g, 3.06mmol), dichlorobis(triphenylphosphine)paradium (II) (0.21g, 0.3mmol) and sodium hydrogencarbonate (0.84g, 10mmol). The mixture was heated under reflux for 2 hours. After cooling, the reaction mixture was neutralized to pH 2 with 2N-hydrochloric acid, and evaporated to remove the dimethoxyethane. The residue was dissolved in chloroform, and the solution was washed with water, dried, then evaporated to remove the solvent. The residue was purified with silica gel column chromatography (hexane:ethyl acetate=1:2) to give 5-chloro-4-(4-chloro-2-fluoro-5-methoxyphenyl)-6-methylpyrimidine (0.6g).
   m.p.136-140°C
   ¹H-NMR(CDCl₃) δ 2.72(3H, s), 3.92(3H, s), 6.99(1H, d, J=6.04Hz), 7.26(1H, d, J=8.92Hz), 9.01(1H, s)
(2) To a mixture of hydrobromic acid (47%, 9.4ml) and acetic acid(4.5ml) was added the compound prepared in Example 9(1) (0.6g, 2.0mmol). The mixture was stirred at 130°C for 7 hours. After cooling, the acetic acid was removed by evaporation , and water was added to the residue. The mixture was extracted with chloroform, and the extract was washed with water, dried and removed the solvent by evaporation. A mixture of hexane:ethyl acetate=10:1 was added to the residue, then the crystals separated were collected by filtration and dried to give 5-chloro-4-(4-chloro-2-fluoro-5-hydroxyphenyl)-6-methylpyrimidine(0.43g).
   m.p.162-163°C
   ¹H-NMR(CDCl₃) δ 2.71(3H, s), 5.74(1H, br.s), 7.11(1H, d, J=6.26Hz), 7.12(1H, d, J=8.75Hz), 9.02(1H, s)
(3) The compound prepared in Example 9(2) (0.15g, 0.55mmol) and potassium carbonate (0.1g, 0.71mmol) were added to DMF(3ml), and 2-iodopropane(0.12g, 0.7mmol) was added to the above mixture under ice-cooling. The mixture was stirred at room temperature for 19 hours. Iced water was added to the reaction mixture, and then extracted with ethyl acetate, washed with water, dried and evaporated to remove the solvent. The residue was purified with silica gel column chromatography (hexane:ethyl acetate=1:1) to give 5-chloro-4-(4-chloro-2-fluoro-5-isopropoxyphenyl)-6-methylpyrimidine (0.15g, oil).
   ¹H-NMR(CDCl₃) δ 1.39(6H, t, J=6.09Hz), 2.71(3H,s), 4.52(1H, m), 7.02(1H, d, J=6.24Hz), 7.25(1H, d, J=9.05Hz), 9.00(1H, s)

### Example 10

### 4-(5-Allyloxy-4-chloro-2-fluorophenyl)-5-chloro-6-difluoromethylpyrimidine (Compound NO. D-2)

(1) To a mixture of dimethoxyethane(40ml) and water (8ml) were added 4,5-dichloro-6-difluoromethylpyrimidine (0.9g, 4.5mmol), phenylboronic acid (0.84g, 4.1mmol), dichlorobis(triphenylphosphine)paradium(II) (0.35g, 0.5mmol) and sodium hydrogencarbonate (1.2g, 14.6mmol). The mixture was stirred for 4 hours. After cooling, the reaction mixture was neutralized to pH 2 with 2N hydrochloric acid, and evaporated to remove the dimethoxyethane. The residue was dissolved in chloroform, washed with water, dried and evaporated to remove the solvent. The residue was purified by silica gel column chromatography (hexane:ethyl acetate=2:1) to give 5-chloro-4-(4-chloro-2-fluoro-5-methoxyphenyl)-6-difluoromethylpyrimidine (0.8g).
   m.p.118-119°C
   ¹H-NMR(CDCl₃) δ 3.93(3H, s), 6.93(1H, t, J=53.2Hz), 7.01(1H, d, J=5.87Hz), 7.30(1H, d, J=8.98Hz), 9.27(1H, s).
   ¹⁹F-NMR(CDCl₃) δ -121.0(d, J=56.2Hz).
(2) The compound prepared in Example 10(1) (0.5g, 1.64mmol) was dissolved in dichloromethane (5ml). and then boron tribromide (1M in CH₂Cl₂) (3.3ml, 3.3mmol) was dropwise added to the solution under ice-cooling. The mixture was stirred at room temperature for 3 hours, poured into ice-water, and extracted with chloroform. The extract was washed with water, dried and evaporated to remove the solvent. Hexane was added to the residue, and the resultant crystal was collected by filtration and dried to give 5-chloro-4-(4-chloro-2-fluoro-5-hydroxyphenyl)-6-difluoromethylpyrimidine (0.5g).
   m.p.141-143°C
   ¹H-NMR(CDCl₃) δ 6.92(1H, t, J=53.2Hz), 7.16(1H, d, J=6.22Hz), 7.26(1H, d, J=8.63Hz), 9.26(1H, s)
(3) The compound prepared in Example 10(2) (0.15g, 0.485mmol) and potassium carbonate (0.085g, 0.60mmol) were added to DMF(2ml). To the mixture was added allylchloride (0.0445g, 0.582mmol) under ice-cooling. The mixture was stirred at room temperature for 14 hours and then further stirred at 50°C for 1 hour. The ice water was added to the reaction mixture, and the mixture was extracted with ethyl acetate. The extract was washed with water, dried and evaporated to remove the solvent. The residue was purified by silica gel column chromatography (hexane:ethyl acetate=2:1) to give 4-(5-allyloxy-4-chloro-2-fluorophenyl)-5-chloro-6-difluoromethylpyrimidine(0.15g).
   m.p.69-71°C
   ¹H-NMR(CDCl₃) δ 4.60-4.70(2H, m), 5.30-5.55(2H, m), 5.95-6.18(1H, m), 6.93(1H, t, J=53.2Hz), 7.02(1H, d, J=6.07Hz), 7.03(1H, d, J=8.99Hz), 9.26(1H, s).

### Example 11

### Ethyl 2-chloro-3-[2-chloro-5-(5-chloro-6-trifluoromethylpyrimidin-4-yl)-4-fluorophenyl]-propionate (Compound No. A-9)

(1) To a mixture of dimethoxyethane(120ml) and water (24ml) were added 4,5-dichloro-6-trifluoromethylpyrimidine (2.9g, 13.5mmol), 4-chloro-2-fluorophenylboronic acid (2.25g, 12.9mmol), dichlorobis(triphenylphosphine) paradium(II) (0.7g, 1mmol) and sodium hydrogencarbonate (3.7g, 44mmol). The mixture was heated under reflux for 3 hours. After cooling, the reaction mixture was adjusted to pH 2 with 2N-hydrochloride, and evaporated to remove the dimethoxyethane. The residue was dissolved in chloroform, and the solution was washed with water, dried and evaporated to remove the solvent. The residue was purified by silica gel column chromatography (hexane:ethyl acetate=5:1) to give 4-(4-chloro-2-fluorophenyl)-5-chloro-6-trifluoromethylpyrimidine (2.65g).
   m.p.100-101°C
   ¹H-NMR(CDCl₃) δ 7.28-7.54(3H, m), 9.27(1H, s), ¹⁹F-NMR(CDCl₃) δ -68.0, -109.3
(2) The compound prepared in Example 11(1) (2.15g, 6.9mmol) was dissolved in concentrated sulfuric acid (17ml) under ice-cooling, and then fuming nitric acid (0.45g, 7.3mmol) was dropwise added thereto. The mixture was stirred for 1 hour at the same temperature, and further stirred for 2 hours at room temperature. The reaction mixture was poured into ice, and extracted twice with ethyl acetate. The extract was washed with water, dried and evaporated to remove the solvent. The crystal formed upon addition of hexane to the residue was collected by filtration and dried to give 5-chloro-4-(4-chloro-2-fluoro-5-nitrophenyl)-6-trifluoromethylpyrimidine (2.2g).
   m.p.103-104°C
   ¹H-NMR(CDCl₃) δ 7.52(1H, dd, J=0.31, 8.80Hz), 8.22(1H, d, J=6.34Hz), 9.32(1H, s)
(3) The compound prepared in Example 11(2) (0.5g, 1.4mmol) was added to a mixture of acetic acid (3ml) and water (0.5ml), and reduced iron (0.23g, 4.2mmol) was portionwise added thereto at 50°C. The temperature was raised to 60°C. After stirring the reaction mixture at 50°C for 1 hour, ethyl acetate and water were added to the reaction mixture, then filtered to remove the insoluble materials. The filtrate was evaporated, and water was added to the residue. The solution was made alkaline with 2N-sodium hydroxide, and then extracted with ethyl acetate. The extract was washed with water, dried, and evaporated to give 4-(5-amino-4-chloro-2-fluorophenyl)-5-chloro-6-trifluoromethylpyrimidine (0.42g).
   m.p. not higher than 87°C
   ¹H-NMR(CDCl₃) δ 4.08(2H, br s), 6.85(1H, d, J=6.32Hz), 7.19(1H, d, J=9.14Hz), 9.24(1H, s)
   ¹⁹F-NMR(CDCl₃ :δ) -68.0, -124.4
(4) Ethyl acrylate (5.54g, 55.4mmol), t-butyl nitrite (0.5g, 4.8mmol) and cuprous chloride (0.6g, 4.5mmol) were suspended in acetonitrile (5ml). To the suspension was dropwise added a solution of the compound (1.0g, 3.06mmol) prepared in Example 11(3) in acetonitrile over 0.5 hour under ice-cooling. The mixture was stirred at the same temperature for 1 hour, and further stirred at room temperature for 1 hour. The reaction mixture was evaporated, and water was added to the residue. The solution was extracted with ethyl acetate, and the extract was washed with 1N-hydrochloride and water, dried and evaporated. The resultant residue was purified by silica gel column chromatography (hexane:ethyl acetate=5:1) to give ethyl 2-chloro-3-[2-chloro-5-(5-chloro-6-trifluoromethylpyrimidin-4-yl)]-4-fluorophenyl]propionate (0.95g, oil).
   ¹H-NMR(CDCl₃) δ 1.27(3H, t, J=7.17Hz), 3.25-3.66(2H, m), 4.10-4.32(2H, m), 4.50-4.65(1H, m), 7.32(1H, d, J=9.27Hz), 7.46(1H, d, J=7.34Hz), 9.27(1H, s)
   ¹⁹F-NMR(CDCl₃) δ -68.0, -111.2
   IR(Nujol) 1743, 1311, 1154, 973 cm⁻¹

### Example 12

### Methyl 2-{2-[4-(5-chloro-6-trifluoromethylpyrimidin-4-yl)phenoxymethyl]phenoxy}propionate(Compound No. A-40)

(1) Under nitrogen atmosphere, magnesium (0.49g, 20mmol) was added to THF(10ml), and a catalytic amount of bromoethane and iodine was added thereto. After dropwise addition of p-iodoanisole (4.68g, 20mmol) in THF (10ml), the mixture was stirred at 40 °C for 2 hours and at 50 °C for 30 minutes. On the other hand, a solution of trimethyl borate (2.04g, 19.6mmol) in THF(10ml) was cooled at -78 °C, and the Grignard reagent obtained above was dropwise added. The resulting mixture was stirred at the same temperature for 2.5 hours. Then, the reaction mixture was gradually heated gradually, and water was added to the reaction mixture at -60 °C, then dilute sulfuric acid (concentrated sulfuric acid(0.6ml)+water(10ml)) was added thereto at -30 °C. The reaction mixture was heated to room temperature, and concentrated to give a residue, to which water was added. The solution was extracted with diethyl ether, washed with brine, dried, and concentrated. Petroleum ether was added to the residue, and the separated crystal was collected by filtration, dried, to give 4-methoxyphenylboronic acid (0.45g).
   m.p. :180-182 °C
   ¹H-NMR(CDCl₃) δ 3.76(3H, s), 6.87(2H, d, J=8.6Hz), 7.73(2H, d, J=8.6Hz), 7.81(2H, br s).
   IR(Nujol) 3600-3100, 1606, 1251, 1180, 1027, 826, 668 cm⁻¹
(2) 4,5-Dichloro-6-trifluoromethylpyrimidine (0.37g, 2.4mmol), 4-methoxyphenylboronic acid(0.61g, 2.8mmol), dichlorobis(triphenylphosphine)palladium(II) (0.11g, 0.16mmol) and sodium hydrogencarbonate (0.65g, 7.8mmol) were added to a mixture of dimethoxyethane(20ml) and water(5ml). The resulting mixture was heated under reflux for 2.5 hours. After cooling, the reaction mixture was adjusted to pH 2 with 2N-hydrochloric acid, and evaporated to remove the dimethoxyethane. The residue was dissolved in chloroform, and the solution was washed with water, dried, and evaporated. The residue was purified by silica gel column chromatography (hexane:ethyl acetate=10:1) to give 5-chloro-4-(4-methoxyphenyl)-6-trifluoromethylpyrimidine (0.51g).
   m.p. :104-106 °C
   ¹H-NMR(CDCl₃) δ 3.90(3H, s), 7.04(2H, d, J=8.9Hz), 7.89(2H, d, J=9.0Hz), 9.18(1H, s).
   ¹⁹F-NMR(CDCl₃) δ -67.9.
   IR(Nujol) 1608, 1552, 1259, 1158, 1044, 838 cm⁻¹
(3) To a mixture of acetic acid(3ml) and 47% hydrobromic acid(12ml) was added the compound prepared in Example 12(2) (0.48g, 1.7mmol). The mixture was stirred at 120°C for 8 hours. After cooling, the reaction mixture was poured into ice water, and extracted with ethyl acetate. The extract was washed with water, dried and evaporated. The residue was purified by silica gel column chromatography (hexane:ethyl acetate=4:1) to give 5-chloro-4-(4-hydroxyphenyl)-6-trifluoromethylpyrimidine(1.01g).
   m.p.:149-151 °C
   ¹H-NMR(CDCl₃) δ 5.42-5.58(1H, br s), 6.97(2H, d, J=8.8Hz), 7.83(2H, d, J=8.8Hz), 9.18(1H, s).
   ¹⁹F-NMR(CDCl₃) δ -67.9.
(4) To a suspension of the compound prepared in Example
   12(3) (0.15g, 0.69mmol) and potassium carbonate (0.14g, 1.0mmol) in DMF (5ml) was added methyl 2-(2-chloromethylphenoxy)propionate (0.38g, 1.7mmol). The resulting mixture was stirred at 60 °C for 4 hours. After cooling, brine was added to the reaction mixture, and the mixture was extracted with ethyl acetate. The extract was washed with water, and dried. The residue was purified by silica gel column chromatography (hexane:ethyl acetate=4:1) to give methyl 2-{2-[4-(5-chloro-6-trifluoromethylpyrimidin-4-yl)phenoxymethyl]phenoxy}propionate(0.28g, oil).
   ¹H-NMR(CDCl₃) δ 1.65(3H, d, J=6.8Hz), 3.75(3H, s), 4.87(1H, q, J=6.8Hz), 5.31(2H, br s), 6.78(1H, d, J=8.1Hz), 6.98-7.06(1H, m), 7.15(2H, d, J=8.9Hz), 7.22-7.30(1H, m), 7.48(1H, d, J=7.5Hz), 7.88(2H, d, J=8.9Hz), 9.17(1H, s).
   ¹⁹F-NMR(CDCl₃) δ -67.9.
   IR(Nujol) 1757, 1606, 1553, 1275, 1208, 1175, 1150, 1045, 754 cm⁻¹

### Example 13

### 5-Chloro-4-[4-chloro-2-fluoro-5-(difluoromethoxy) phenyl]-6-difluoromethylpyrimidine (Compound No. D-7)

Potassium carbonate (0.18g, 1.30mmol, 1.2eq) and ethyl bromodifluoroacetate (0.27g, 1.33mmol, 1.2eq) were added to DMF 3ml solution containing the compound obtained in the Example 10(2) (0.30g, 1.10mmol). The reaction was carried out at room temperature for 22 hours. Water (150ml) was added to the reaction mixture, followed by extraction with ethyl acetate (75ml). The extract was washed with water, dried and concentrated by evaporation of the solvent. The residue was purified by silica gel column chromatography (hexane : ethyl acetate = 5:1). The purified product was dissolved in ethanol 3ml, followed by addition of aqueous solution of 0.5ml containing sodium hydroxide (44mg, 1.10mmol). The mixture was stirred at room temperature for 3 hours. The reaction mixture was concentrated and water 30ml was added to the residue, followed by extraction with diethyl ether. The extract was washed with water, dried and concentrated by evaporation of the solvent. The residue was purified by silica gel column chromatography (hexane : ethyl acetate = 2:1) to give 5-chloro-4-[4-chloro-2-fluoro-5-(difluoromethoxy)phenyl]-6-difluoromethylpyrimidine (74mg).
¹H-NMR(CDCl₃) δ 6.57(1H, t, J=7.2Hz), 6.92(1H, t, J=53.2Hz), 7.39(1H, d, J=8.8Hz), 7.45(1H, d, J=6.3Hz), 9.28(1H, s)
¹⁹F-NMR(CDCl₃) δ -82.29, -112.68, -121.19
IR(nujol) : 1556, 1491, 1417, 1377, 1352, 1238, 1186. 1135, 1047 cm⁻¹
m.p. : 56-57°C

### Example 14

### 5-Chloro-4-(4-chloro-5-ethylsulfonylamino-2-fluorophenyl)-6-difluoromethylpyrimidine (Compound No. D-14)

(1) 4,5-dichloropyrimidine (1.7g, 8.6mmol) prepared in the Reference Example 4, 2-chloro-4-fluorophenylboronic acid (1.5g, 8.6mmol) prepared in the Reference Example 6, dichlorobis(triphenylphosphine)palladium(II) (0.18g, 0.26mmol) and sodium hydrogen carbonate (22.4g, 28mmol) were added to a mixture of 1,2-dimethoxyethane (50ml) and water (13ml), followed by refluxing under heating for 3 hours. After cooling, the reaction mixture was concentrated, and water was added to the residue, followed by neutralization with 2N hydrochloric acid (pH value = 3) and extraction with chloroform. The extract was washed with water, dried and concentrated by evaporation of the solvent. The residue was purified by silica gel column chromatography (hexane : ethyl acetate = 5:1) to give 5-chloro-4-(4-chloro-2-fluorophenyl)-6-difluoromethylpyrimidine (1.5g).
   m.p.76-77°C
   ¹H-NMR(CDCl₃) δ 6.93(1H, t, J=53.2Hz), 7.23-7.47(3H, m), 9.26(1H, s)
(2) The product produced in the Example 14(1) (1.5g, 5.1mmol) was added to concentrated sulfuric acid (10ml) under cooling with ice. After addition of fuming nitric acid (0.2ml), the mixture was stirred at the same temperature for 1.5 hours and further at room temperature for 2 hours. The reaction mixture was poured on ice, followed by extraction with ethyl acetate. The extract was washed with aqueous sodium hydrogen carbonate and water, dried and concentrated by evaporation of the solvent to give 5-chloro-4-(4-chloro-2-fluoro-5-nitrophenyl)-6-difluoromethylpyrimidine (1.7g).
   ¹H-NMR(CDCl₃) δ 6.91(1H, t, J=53.1Hz), 7.49(1H, d, J=8.8Hz), 8.22(1H, d, J=6.5Hz), 9.30(1H, s)
(3) The compound produced in the Example 14(2) (1.7g, 5.1mmol) was added to acetic acid (10ml) and water (2.6ml), and reduced iron (0.86g, 15.5mmol) was portionwase added at 40°C, while the temperature was raised to 50°C. The mixture was stirred at the same temperature for 1.5 hours. Ethyl acetate was added to the reaction mixture, and the insolubles were removed by filtration using Celite layer, and the filtrate was concentrated. Water was added to the residue, followed by neutralization with 2N sodium hydroxide solution and extraction with ethyl acetate. The extract was washed with water, dried and concentrated by evaporation of the solvent to give 4-(5-amino-4-chloro-2-fluorophenyl)-5-chloro-6-difluoromethylpyrimidine (1.5g).
   m.p.106-108°C
   ¹H-NMR(CDCl₃) δ 4.07(2H, br.s), 6.86(1H, d, J=6.3Hz), 6.93(1H, t, J=53.1Hz), 7.18(1H, d, J=9.1Hz), 9.24(1H, s)
(4) The compound produced in the Example 14(3) (0.2g, 0.62mmol) and triethylamine (0.18g, 1.8mmol) were dissolved in THF (5ml), followed by addition of ethanesulfonyl chloride (0.2g, 1.6mmol) under cooling with ice. The mixture was stirred at the same temperature for 3 hours. The reaction mixture was poured into brine solution, followed by extraction with ethyl acetate. The extract was washed with water, dried and concentrated by evaporation of the solvent. Hexane was added to the residue, and the resultant crystals were collected by filtration and dried to give a bis form product. Yield 0.3g.
   ¹H-NMR(CDCl₃) δ 1.52(6H, t, J=7.4Hz), 3.50-3.90(4H, m), 6.92(1H, t), 7.46(1H, d, J=8.9Hz), 7.74(1H, d, J=6.7Hz), 9.28(1H, s)
   The bis form product (0.3g) was dissolved in dioxane (5ml), followed by addition of 2N sodium hydroxide aqueous solution (0.5ml) under cooling with ice. The solution was stirred at room temperature for 2 hours. Further 2N sodium hydroxide aqueous solution (0.25ml) was added and the mixture was stirred at room temperature for 2 hours. Water was added to the reaction mixture, followed by neutralization with 2N hydrochloric acid. The resultant crystals were collected by filtration, washed with water and dried to give 5-chloro-4-(4-chloro-5-ethylsulfonylamino-2-fluorophenyl)-6-difluoromethylpyrimidine (0.22g).
   m.p.138-139°C
   ¹H-NMR(CDCl₃) δ 1.42(3H, t, J=7.3Hz), 3.17(2H, q, J=7.3Hz), 6.74(1H, br s), 6.91(1H, t, J=53.2Hz), 7.36(1H, d, J=8.8Hz), 7.85(1H, d, J=6.5Hz), 9.27(1H, s)

### Example 15

### 5-Chloro-4-(2,4-dichloro-5-ethylsulfonylaminophenyl) -6-difluoromethylpyrimidine (Compound No. D-16)

(1) 4,5-Dichloropyrimidine (1.15g, 5.8mmol) produced by the Reference Example 4, 2,4-dichlorophenylboronic acid (1.1g, 5.8mmol), dichlorobis(triphenylphosphine) palladium(II) (0.12g. 0.17mmol) and sodium hydrogen carbonate (1.6g, 19mmol) were added to a mixture of 1,2-dimethoxyethane (40ml) and water (9ml), followed by refluxing under heating for 3 hours. After cooling, the reaction mixture was concentrated, and water was added to the residue, followed by neutralization with 2N hydrochloric acid (pH value = 3) and extraction with ethyl acetate. The extract was washed with water, dried by evaporation of the solvent. The residue was purified by silica gel column chromatography (hexane : ethyl acetate = 5:1) to give 5-chloro-4-(2,4-dichlorophenyl)-6-difluoromethylpyrimidine (1.09).
   m.p.55-56°C
   ¹H-NMR(CDCl₃) δ 6.92(1H, t, J=53.2Hz), 7.28-7.50(2H, m), 7.56(1H, d, J=1.9Hz), 9.27(1H, s)
(2) The compound obtained in the Example 15(1) (1.0g, 3.2mmol) was added to concentrated sulfuric acid (7ml), followed by addition of fuming nitric acid (0.13ml). The mixture was stirred at the same temperature for 1 hour and at room temperature for 2 hours. The reaction mixture was poured onto ice, followed by extraction using chloroform. The extract was washed with aqueous sodium hydrogen carbonate and water, dried and concentrated by evaporation of the solvent. Hexane was added to the residue, and the resultant crystals were collected by filtration, followed by drying, to give 5-chloro-4-(2,4-dichloro-5-nitrophenyl)-6-difluoromethylpyrimidine (0.9g).
   m.p.107-109°C
   ¹H-NMR(CDCl₃) δ 6.91(1H, t, J=53.1Hz), 7.78(1H, s), 8.01(1H, s), 9.31(1H, s)
(3) The compound produced by the Example 15(2) (0.9g, 2.6mmol) was added to acetic acid (5ml) and water (1.3ml), followed by portionwise addition of reduced iron (0.44g, 7.9mmol) at 40°C, while the temperature of the mixture is raised up to 45°C, followed by stirring at the same temperature for 1 hour. Ethyl acetate was added to the reaction mixture, and the insolubles were removed by filtration through Celite layer, followed by concentration of the filtrate. To the residue was added water, and the mixture was neutralized with aqueous sodium hydrogen carbonate and extracted with ethyl acetate. The extract was washed with water, dried and concentrated by evaporation of the solvent. The residue was purified by silica gel column chromatography (hexane : ethyl acetate = 2:1) to give 4-(5-amino-2,4-dichlorophenyl)-5-chloro-6-difluoromethylpyrimidine (0.8g).
   m.p.97-99°C
   ¹H-NMR(CDCl₃) δ 4.22(2H, br.s), 6.73(1H, s), 6.92(1H, t, J=53.2Hz), 7.42(1H, s), 9.25(1H, s)
(4) The compound produced in the Example 15(3) (0.2g, 0.2mmol) and triethylamine (0.18g, 1.8mmol) were dissolved in THF (5ml), followed by addition of ethanesulfonyl chloride (0.2g, 1.6mmol) under cooling with ice and by stirring at the same temperature for 3 hours. The reaction mixture was poured onto brine and extracted with ethyl acetate. The extract was washed with water, dried and concentrated by evaporation of the solvent. A mixture of hexane and ethyl acetate (5:1) was added to the residue, and the resultant crystals were collected by filtration, followed by drying, to give a bis form product. The yield was 0.21g.
   ¹H-NMR(CDCl₃) δ 1.43-1.65(6H, m), 3.50-4.00(4H, m), 6.91(1H, t, J=53.1Hz), 7.48(1H, s), 7.74(1H, s), 9.29(1H, s)
   The bis form product (0.21g) was dissolved in dioxane (5ml), followed by addition of 2N sodium hydroxide aqueous solution (0.5mo) under cooling with ice and by stirring at room temperature for 2 hours. Furthermore 2N sodium hydroxide aqueous solution (0.25ml) was added, followed by stirring at room temperature for 1 hour. Water was added to the reaction mixture, followed by neutralization with 2N hydrochloric acid and by extraction with ethyl acetate. The extract was washed with water, dried and concentrated by evaporation of the solvent. The residue was purified by silica gel column chromatography (hexane : ethyl acetate = 1:1) to give 5-chloro-4-(2,4-dichloro-5-ethylsulfonylaminophenyl)-6-difluoromethylpyrimidine (0.15g).
   m.p.140-142°C
   ¹H-NMR(CDCl₃) δ 1.40(3H, t, J=7.4Hz), 3.18(2H, q, J=7.4Hz), 6.85(1H, br s), 6.91(1H, t, J=53.2Hz), 7.62(1H, s), 7.72(1H, s), 9.28(1H, s)

### Example 16

### 4-(5-Acetylamino-4-cyano-2-fluorophenyl)-5-chloro-6-difluoromethylpyrimidine (Compound No. D-21)

(1) The compound produced in 4,5-dichloro-6-difluoromethylpyrimidine (4.4g, 22.1mmol) 2,4-difluorophenylboronic acid (3.0g, 19mmol), dichlorobis(triphenylphosphine)palladium(II) (1.2g, 1.7mmol) and sodium hydrogen carbonate (6.0g, 72mmol) were added to a mixture of 1,2-dimethoxyethane (180ml) and water (35ml), followed by refluxing under heating for 3 hours. After cooling, the reaction mixture was neutralized with 2N hydrochloric acid (pH value = 5), followed by evaporating off dimethoxyethane. The residue was dissolved in chloroform, washed with water, dried and concentrated by evaporation of the solvent. The residue was purified by silica gel column chromatography (hexane : ethyl acetate = 3:1) to give 5-chloro-4-(2,4-difluorophenyl)-6-difluoromethylpyrimidine (3.84g).
   m.p.69-71°C
   ¹H-NMR(CDCl₃) δ 6.93(1H,t,J=53.2Hz),6.94-7.14(2H,m), 7.45-7.60(1H, m),9.26(1H,s)
(2) The product produced by the method of the Example 16(1) (2.38g, 8.07mmol) was added to concentrated sulfuric acid (19ml) under cooling with ice, followed by addition of fuming nitric acid (0.52ml) 3 times and by stirring at the same temperature for 1 hour and at room temperature for 3 hours. The reaction mixture was poured onto ice, and extracted twice with ethyl acetate. The extract was washed with aqueous sodium hydrogen carbonate and water, dried and concentrated by evaporation of the solvent. The residue was purified by silica gel column chromatography (hexane : ethyl acetate = 3:1) to give 5-chloro-4-(2,4-difluoro-5-nitrophenyl)-6-difluoromethylpyrimidine (3.37g).
   m.p.103-105°C
   ¹H-NMR(CDCl₃) δ 6.91(1H,t,J=53.1Hz),7.20-7.40(1H,m), 8.35-8.57(1H,m),9.31(1H,s)
   IR(Nujol) 1641, 1537, 1463, 1400, 1343, 1250, 1157, 1070,1032 cm⁻¹
(3) Potassium cyanide (0.36g, 5.53mmol, 1.2eq) was added to DMF (10ml) solution containing the compound produced by the Example 16(2), and the reaction was carried out at 50°C for 5 hours. Water (100ml) was added to the reaction mixture, followed by extraction with ethyl acetate. The extract was washed with brine solution, dried and concentrated by evaporation of the solvent. The residue was purified by silica gel column chromatography (hexane : ethyl acetate = 4:1) to give 5-chloro-4-(4-cyano-2-fluoro-5-nitrophenyl)-6-difluoromethylpyrimidine (0.43g).
   ¹H-NMR(CDCl₃) δ 6.92(1H, t, J=53.1Hz), 7.81(1H, d, J=8.2Hz), 8.60(1H, d, J=6.0Hz), 9.35(1H, s)
   ¹⁹F-NMR(CDCl₃) δ -98.97(1F,dd, J=6.3, 8.7Hz), -120.85(2F, d, J=56.4Hz)
   IR(nujol) 2240, 1573, 1546, 1422, 1346, 1136, 1066 cm⁻¹
   m.p.: 100-102°C
(4) Reduced iron (0.56g, 10.0mmol, 5eq) was suspended into acetic acid (5ml) and 2-propanol (10ml), followed by heating up to 70°C. The compound produced by the method of the Example 16(3) (0.64g, 1.99mmol) in acetic acid (10ml) and 2-propanol (5ml) solution was dropwise added over 10 minutes, and the reaction was carried out at 80°C for 15 minutes. The reaction mixture was added to ethyl acetate, and the insolubles were filtered off, followed by concentration of the filtrate. The residue was purified by silica gel column chromatography (hexane : ethyl acetate = 1:1) to give 4-(5-amino-4-cyano-2-fluorophenyl)-5-chloro-6-difluoromethylpyrimidine (0.46g).
   ¹H-NMR(DMSO-*d*₆) δ 6.26(2H, brs), 6.93(1H, d, J=6.0Hz), 7.32(1H, t, J=52.7Hz), 7.61(1H, d, J=9.8Hz) 9.40(1H, s)
   ¹⁹F-NMR(DMSO-*d*₆) δ ppm : -121.59, -130.18
   IR(nujol) 3491, 3377, 2226, 1640, 1503, 1452, 1437, 1239, 1098, 1051 cm⁻¹
   m.p.: 212-213°C
(5) Pyridine (81µl, 1.00mmol) was added to THF (3ml) solution containing the compound produced in the Example 16(4) (300mg, 1.00mmol), followed by addition of acetyl chloride (85µl, 1.20mmol) at room temperature and by stirring for 14 hours. The reaction mixture was concentrated, and ethyl acetate (50ml) and water (50ml) were added to the residue. Ethyl acetate layer was separated, washed with water (50ml), dried and concentrated by evaporation of the solvent. The resultant crystals were washed with hexane and collected by filtration to give 4-(5-acetyl amino-4-cyano-2-fluorophenyl)-5-chloro-6-difluoromethylpyrimidine (0.30g).
   ¹HNMR(CDCl₃) δ 2.29(3H, s), 6.92(1H, t, J=53.2Hz), 7.46(1H, d, J=8.4Hz), 7.71(1H, br s), 8.62(1H, d, J=6.2Hz), 9.29(1H, s)
   IR(nujol) 3252, 2238, 1668, 1528, 1425, 1118, 1075 cm⁻¹
   m.p. : 160-162°C

### Example 17

### 4-[5-(N-acetyl-N-propargylamino)-4-cyano-2-fluorophenyl]-5-chloro-6-difluoromethylpyrimidine (Compound No. D-22)

The compound produced in the Example 16(4) (0.15g, 0.44mmol) was portionwise added to an oily suspension of 60% sodium hydride (26mg, 0.65mmol) in THF (2ml), followed by addition of propargyl bromide (50µl, 0.66mmol), and the reaction was carried out at room temperature for 19 hours. The reaction mixture was concentrated, and brine solution was added to the residue, followed by extraction with ethyl acetate. The extract was dried and concentrated by evaporation of the solvent. The residue was purified by silica gel column chromatography (hexane : ethyl acetate = 2:1 and chloroform : acetone = 10:1) to give 4-[5-(N-acetyl-N-propargylamino)-4-cyano-2-fluorophenyl]-5-chloro-6-difluoromethylpyrimidine (0.14g).
¹H-NMR(CDCl₃) δ 1.97(3H, s), 2.28(1H, t, J=2.4Hz),4.28, 4.96(2H, dq_{AB}, J_{d}=2.1Hz, J_{AB}=17.5Hz), 6.93(1H, t, J=53.1Hz), 7.69(1H, d, J=6.1Hz), 7.69(1H, d, J=8.4Hz), 9.33(1H, s)

### Example 18

### Ethyl 2-chloro-3-[2-chloro-5-(5-chloro-6-difluoromethylpyrimidin-4-yl)-4-fluorophenyl]-propionate (Compound No. D-23)

Tert-butyl nitrite (0.32ml, 2.4mmol) and cupric chloride (0.29g, 1.9mmol) were added to acetonitrile (6ml) containing ethyl acrylate (0.5g, 1.6mmol) at room temperature, followed by cautious dropwise addition of acetonitrile solution (4ml) containing the compound produced in the Example 14(3) (0.50g, 1.6mmol). The mixture was stirred at the same temperature for 2.5 hours. The reaction mixture was concentrated under reduced pressure, and ethyl acetate (50ml) was added to the residue, followed by washing with 1N hydrochloric acid, purified water and saturated brine. The organic layer was dried over anhydrous magnesium sulfate and concentrated under reduced pressure. The residue was purified by silica gel column chromatography (hexane : ethyl acetate = 10:1) to give ethyl 2-chloro-3-[2-chloro-5-(5-chloro-6-difluoromethylpyrimidin-4-yl)-4-fluorophenyl]propionate (0.48g).
¹HNMR (CDCl₃) δ 1.26(3H, t, J=7.1Hz), 3.29-3.34(1H, m), 3.52-3.58(1H, m), 4.18-4.27(2H, m), 4.56-4.60(1H, m), 6.92(1H, t, J=53.3Hz), 7.32(1H, d, J=8.2Hz), 7.47(1H, d, J=8.2Hz), 9.26(1H, s).
¹⁹F-NMR(CDCl₃) δ -121.2(2F, s), -111.3(1F, s).
IR(Neat) 2986(CH), 1744(CO), 1616, 1556, 1490, 1373, 1270, 1231, 1181, 1131, 1062, 978 cm⁻¹

### Example 19

### Ethyl 2-[2-chloro-5-(5-chloro-6-difluoromethylpyrimidin-4-yl)-4-fluoroanilino]propionate(Compound No. D-24)

Trifluoromethanesulfonic acid anhydride (0.21ml, 1.3mmol) and 2,4,6-collidine (0.17ml, 1.3mmol) were cautiously dropwise added to methylene chloride solution (10ml) containing ethyl lactate (0.14g, 1.2mmol) under cooling with ice, followed by stirring at room temperature for 0.5 hour. After that, methylene chloride solution (5ml) containing the compound produced in the Example 14(3) (0.30g, 0.97mmol) and 2,4,6-collidine (0.17ml, 1.3mmol) were added, followed by stirring for 8 hours while refluxing under heating. The reaction mixture was concentrated under reduced pressure, followed by addition of 1N hydrochloric acid (30ml) and by extraction with ethyl acetate. The organic layer was washed successively with purified water and saturated brine, dried over anhydrous magnesium sulfate and concentrated under reduced pressure. The residue was purified by silica gel column chromatography (hexane : ethyl acetate = 5:1) to obtain ethyl 2-[2-chloro-5-(5-chloro-6-difluoromethylpyrimidin-4-yl)-4-fluoroanilino]propionate(0.11g).
¹HNMR (CDCl₃) δ 1.26(3H, t, J=7.1Hz), 1.55(3H, d, J=4.2Hz), 4.11-4.16(1H, m), 4.21(2H, q, J=7.1Hz), 4.82(1H, d, J=7.9Hz), 6.63(1H, d, J=6.1Hz), 6.93(1H, t, J=53.3Hz), 7.22(1H, d, J=6.1Hz), 9.24(1H, s).
¹⁹F-NMR(CDCl₃) δ -125.3(1F. s), -121.3(2F, s).
IR(Neat) 3400(NH), 2986(CH), 1738(CO), 1557, 1519, 1447, 1427, 1377, 1257, 1191, 1061 cm⁻¹
CI-MS m/z : 408(M+1). C₁₆H₁₄Cl₂F₃N₃O₂.

### Example 20

### 5-Chloro-4-[4-cyano-5-(ethylsulfonylamino)-2-fluorophenyl]-6-difluoromethylpyrimidine (Compound NO. D-26)

(1) Triethylamine (0.71ml, 5.12mmol, 2.5eq) was added to 1,2-dichloroethane solution (15ml) containing the compound obtained in the Example 16 (4) (0.61g, 2.04mmol), and the mixture was cooled. 1,2-Dichloroethane (5ml) solution containing ethanesulfonyl chloride (0.48g, 5.07mmol, 2.5eq) was dropwise added and the mixture was stirred at room temperature for 1 hour. The reaction mixture was again cooled, followed by addition of triethylamine (0.28ml, 2.02mmol, 1eq). 1,2-dichloroethane (3ml) solution containing ethanesulfonyl chloride (194µl, 2.05mmol, 1eq) was dropwise added, followed by stirring at room temperature for 1 hour. Again cooled triethylamine (0.57ml, 4.11mmol, 2eq) was added, followed by dropwise addition of 1,2-dichloroethane (3ml) solution containing ethanesulfonyl chloride (388µl, 4.09mmol, 2eq), and the reaction was carried out at room temperature for 2.5 hours. Again cooled triethylamine (0.28ml. 2.02mmol, 1eq) was added, followed by dropwise addition of 1,2-dichloroethane (3ml) solution containing ethanesulfonyl chloride (194µl, 2.05mmol, 1eq), and the reaction was carried out at room temperature for 3 hours. Chloroform (30ml) was added to the reaction mixture, followed by washing with water, drying and concentration by evaporation of the solvent. The residue was purified by silica gel column chromatography (hexane : ethyl acetate = 1:1) to obtain 5-chloro-4-[5-(N,N-bisethylsulfonylamino)-4-cyano-2-fluorophenyl]-6-difluoromethylpyrimidine (1.04g).
   ¹H-NMR(CDCl₃) δ 1.54(6H, t, J=7.5Hz), 3.73(2H, q, J=7.4Hz), 3,74(2H, q, J=7.5Hz), 6.91(1H, t, J=53.1Hz). 7.66(1H, d, J=8.3Hz), 7.71(1H, d, J=6.0Hz), 9.31(1H, s)
(2) The compound produced in the Example 20(1) (1.04g) was suspended in dioxane (5ml) and water (5ml), followed by cooling with ice. Aqueous solution (5ml) containing sodium hydroxide (0.55g, 13.75mmol) was dropwise added, and the reaction was carried out at room temperature for 2 hours. The reaction mixture was concentrated, and water (50ml) was added to the residue. The mixture was acidified with 2N hydrochloric acid, and extracted with ethyl acetate. The extract was washed with water, dried and concentrated by evaporation of the solvent. The residue was purified by silica gel column chromatography (hexane : ethyl acetate = 1:1), and the resulting crystals were washed with isopropyl ether and collected by filtration to give 5-chloro-4-[4-cyano-5-(ethylsulfonylamino)-2-fluorophenyl]-6-difluoromethylpyrimidine (0.56g).
   ¹H-NMR(CDCl₃) δ 1.31(3H, t, J=7.3Hz), 3.23(2H, q, J=7.4Hz), 7.35(1H, t, J=52.4Hz), 7.74(1H, d, J=6.2Hz), 8.18(1H, d, J=9.3Hz), 9.26(1H, s), 10.31(1H, br s)
   IR(nujol) 3094(br), 2230, 1558, 1506, 1425, 1326, 1147, 1076, 916cm⁻¹
   m.p. :200-202°C

### Example 21

### 4-[5-(N-acetyl-N-ethylsulfonylamino)-4-cyano-2-fluorophenyl]-5-chloro-6-difluoromethylpyrimidine (Compound No. D-27)

Triethylamine (90µl, 0.65mmol) was added to acetonitrile (3ml) solution containing the compound produced in the Example 20 (170mg, 0.435mmol), followed by dropwise addition of acetyl chloride (46µl, 0.65mmol) in acetonitrile (2ml). The mixture was stirred for 21 hours at room temperature, and the solvent was evaporated off. The residue was purified by silica gel column chromatography (chloroform : acetone = 10:1), and the resultant crystals were washed with isopropyl ether and collected by filtration to give 4-[5-(N-acetyl-N-ethylsulfonylamino)-4-cyano-2-fluorophenyl]-5-chloro-6-difluoromethylpyrimidine (0.127g).
¹H-NMR(CDCl₃) δ 1.57(3H, t, J=7.5Hz), 2.17(3H, s), 3.78-3.92(2H, br s), 6.91(1H, t, J=53.1Hz), 7.68(1H, d, J=8.3Hz), 7.75(1H, d, J=6.0Hz), 9.31(1H, s)
¹⁹F-NMR(CDCl₃) δ -105.96(1F, dd, J=6.1, 8.4Hz), -121.00(1F, d, J=53.1Hz)
-121.01(1F,d,J=53.1Hz)
IR(nujol) 2242, 1720, 1423, 1367, 1338, 1218, 1160, 1064 cm⁻¹
m.p. : 121-122°C

### Example 22

### 2-Chloro-4-fluoro-5-(5-chloro-6-difluoromethylpyrimidin-4-yl)benzoic acid methyl ester (Compound No. D-41)

The compound produced by the method of the Reference Example 9 (1.51g. 6.5mmol), dichlorobis(triphenylphosphine)palladium(II)(0.15g, 0.21mmol) and sodium hydrogen carbonate (1.64g, 20mmol) were added to a mixture of the compound produced by the method of the Reference Example 4 (1.33g, 6.7mmol), dimethoxyethane (45ml) and water (9ml), followed by stirring at 70°C for 5 hours. After cooling, the reaction mixture was concentrated. Water was added to the residue, followed by neutralization with 1N hydrochloric acid and by extraction with chloroform. The extract was washed with water, dried and concentrated by evaporation of the solvent. The residue was purified by silica gel column chromatography (hexane : ethyl acetate = 12:1) to give 2-chloro-4-fluoro-5-(5-chloro-6-difluoromethylpyrimidin-4-yl)benzoic acid methyl ester (1.31g).
m.p.100-101°C
¹H-NMR(CDCl₃) δ 3.95(3H, s), 6.92(1H, t, J=53.2Hz), 7.39(1H, d, J=9.3Hz), 8.13(1H, d, J=7.4Hz), 9.29(1H, s),
¹⁹F-NMR(CDCl₃) δ -104.7, -121.2
IR(Nujol) 1739, 1611, 1544, 1299, 1234, 1052 cm⁻¹

### Example 23

### 2-chloro-4-fluoro-5-(5-chloro-6-difluoromethylpyrimidin-4-yl)benzoyloxy)acetic acid ethyl ester (Compound No. D-43)

(1) 2N sodium hydroxide (6ml) was dropwise added to THF (30ml) solution containing the compound produced by the method of the Example 22 (1.3g, 3.7mmol), followed by stirring at room temperature for 3.5 hours. Furthermore 2N sodium hydroxide solution (2ml) was added, followed by stirring at the same temperature for 3 hours. The reaction mixture was concentrated, followed by addition of water. The mixture was neutralized with 1N hydrochloric acid and extracted with ethyl acetate. The extract was washed with water and the solvent was evaporated off to obtain a crude product, to which was added petroleum ether. The resultant crystals were collected by filtration and dried to obtain 2-chloro-4-fluoro-5-(5-chloro-6-difluoromethylpyrimidin-4-yl)-benzoic acid (1.18g).
   m.p.217-219°C
   ¹H-NMR(DMSO-d₆) δ 7.31(1H, t, J=52.5Hz), 7.80(1H, d, J=9.7Hz), 8.12(1H, d, J=7.6Hz), 9.41(1H, s), 13.5-13.8(1H, br)
   ¹⁹F-NMR(DMSO-d₆) δ -106.6, -121.5
   IR(Nujol) 3200-2500, 1720, 1613, 1243,1053 cm⁻¹
(2) Carbonyldiimidazole (0.25g, 1.5mmol) was added to THF (8ml) solution containing the compound produced by the method of the Example 23(1)(0.34g, 1mmol), and the mixture was stirred at room temperature for 40 minutes. Then ethyl glycolate (0.16g, 1.5mmol) was added, and the mixture was refluxed under heating for 3 hours. After cooling the reaction mixture was concentrated by evaporation of the solvent. Water was added to the residue, followed by extraction with ethyl acetate. The extract was washed with aqueous sodium hydrogen carbonate and brine, dried and concentrated by evaporation of the solvent. The residue was purified by silica gel column chromatography (hexane : ethyl acetate = 12:1) to give 2-chloro-4-fluoro-5-(5-chloro-6-difluoromethylpyrimidin-4-yl)benzoyloxy)acetic acid ethyl ester (0.32g, oil).
   ¹H-NMR(CDCl₃) δ 1.30(3H, t, J=7.1Hz),4.26(2H, q, J=7.1Hz), 4.86(2H, s), 6.92(1H, t, J=53.2Hz), 7.41(1H, d, J=9.4Hz), 8.24(1H, d, J=7.4Hz), 9.29(1H, s)
   ¹⁹F-NMR(CDCl₃) δ -103.8, -121.2
   IR(Nujol) 1745, 1615, 1557, 1206, 1120, 1060 cm⁻¹

### Example 24

### 5-Chloro-4-[4-cyano-5-(ethylsulfonylamino)-2-fluorophenyl]-6-methylpyrimidine (Compound No. E-9)

(1) 4,5-dichloropyrimidine (1.0g, 6.1mmol) prepared by the method of Reference Example 3 is dissolved in 1,2-dimethoxyethane (10ml), and the temperature of the mixture of tetrakis(triphenylphosphine)palladium (0.9g, 0.8mmol), sodium hydrogen carbonate (0.84g, 8mmol) and water (4ml) was raised up to 60°C, followed by portionwise addition of 2,4-difluoro-5-nitrophenylboronic acid (1.6g, 8mmol). The mixture was refluxed under heating for 2 hours. After cooling, the reaction mixture was concentrated, and water was added to the residue. The mixture was adjusted to pH 3 with 2N hydrochloric acid, and extracted with ethyl acetate. The extract was washed with water, dried and filtered by silica gel layer. The filtrate was concentrated by evaporation of the solvent, and the residue was purified by silica gel column chromatography (hexane: ethyl acetate = 3:1) to obtain 5-chloro-4-(2,4-difluoro-5-nitrophenyl)-6-methylpyrimidine (0.41g).
   m.p.95-97°C
   ¹H-NMR(CDCl₃) δ 2.74(3H, s), 7.15-7.23(1H, m), 8.32-8.40(1H, m), 9.04(1H, s)
(2) The compound prepared by the method of Example 24(1) (1.6g, 5.6mmol) was dissolved in DMF (15ml), followed by addition of potassium cyanide (0.47g, 7.2mmol). The mixture was stirred at the same temperature for 2.0 hours. The reaction mixture was poured into iced water and extracted 3 times with ethyl acetate. The extract was washed with brine solution, dried and concentrated by evaporation of the solvent. The residue was purified by silica gel column chromatography (hexane : ethyl acetate = 2:1) to obtain 5-chloro-4-(4-cyano-2-fluoro-5-nitrophenyl)-6-methyl pyrimidine (0.93g). m.p.123-125°C
   ¹H-NMR(CDCl₃) δ 2.76(3H, s), 7.75(1H, d, J=8.1Hz), 8.55(1H, d, J=6.0Hz), 9.08(1H, s)
(3) The compound prepared by the method of the Example 24(2) (0.92g, 3.1mmol) was portionwise added to a mixture of acetic acid (9ml), isopropanol (4.5ml) and reduced iron (0.7g, 12.4mmol) at 70°C, followed by stirring at 70 to 80°C for 1 hour. After cooling, THF (20ml) was added to the reaction mixture, and the insolubles were removed by filtration with Celite layer. The filtrate was concentrated, followed by addition of water to the residue. The mixture was neutralized with aqueous sodium hydrogen carbonate and extracted with ethyl acetate. The extract was washed, dried and concentrated by evaporation of the solvent, followed by addition of water to the residue. The mixture was neutralized with aqueous sodium hydrogen carbonate and extracted with ethyl acetate. The extract was washed with water, dried and concentrated by evaporation of the solvent. The residue was purified by silica gel column chromatography (hexane : ethyl acetate = 2:1) to obtain 4-(5-amino-4-cyano-2-fluorophenyl)-5-chloro-6-methyl pyrimidine (0.57g).
   ¹H-NMR(CDCl₃) δ 2.72(3H, s), 4.42(3H, s), 6.81(1H, d, J=5.5Hz), 7.23(1H, d, J=8.7Hz), 9.00(1H, s)
(4) The compound prepared by the method of the Example 24(3) (0.3g, 1.14mmol) and triethylamine (0.35g, 3.42mmol) were dissolved in THF (7ml), followed by dropwise addition of THF solution containing ethanesulfonyl chloride (0.44g, 3.42mmol) under cooling with ice. The mixture was stirred for 11 hours. Further at the intervals of 1 hour triethylamine (0.16ml) and ethanesulfonyl chloride (0.11ml) were added 4 times. The reaction mixture was concentrated, followed by dissolving the residue into chloroform. The solution were washed with water, dried and concentrated by evaporation of the solvent to give a bis form compound (0.6g).
   ¹H-NMR(CDCl₃) δ 1.63(6H, t), 3.68(4H, m), 7.61(1H, d), 7.68(1H, d), 9.05(1H, s)
(5) The bis form compound prepared by the method of the Example 24(4)(0.6g) was dissolved in THF (7ml), followed by addition of 2N sodium hydroxide (0.7ml). The solution was stirred at room temperature for 1 hour. Furthermore, at the intervals of 1 hour, 2N sodium hydroxide (0.7ml) was added 3 times. Water (20ml) was added to the reaction mixture, and the mixture was neutralized with 2N hydrochloric acid (pH value = 2), followed by extraction with ethyl acetate. The extract was washed with water, dried and concentrated by evaporation of the solvent. The residue was dissolved in hexane : ethyl acetate = 1:4 and filtered by silica gel layer and the solvent was removed by evaporation. Hexane : ethyl acetate = 5:1 was added to the residue. The resultant crystals were collected by filtration and dried to give 5-chloro-4-[4-cyano-5-(ethylsulfonylamino)-2-fluorophenyl]-6-methylpyrimidine (0.13g).
   m.p. : 182-184°C
   ¹H-NMR(CDCl₃) δ 1.46(3H, t, J=7.4Hz) 3.24(2H, q, J=7.4Hz), 6.91(1H, br.s), 7.47(1H, d, J=8.3Hz), 7.86(1H, d, J=5.9Hz), 9.04(1H, s)

### Example 25

### 5-Chloro-4-[4-cyano-5-(ethylsulfonylamino)-2-fluorophenyl]-6-isopropylpyrimidine (Compound No. E-13)

(1) 4,5-Dichloropyrimidine (0.19g, 1mmol) prepared by the method of the Reference Example 10, 2,4-difluorophenylboronic acid (0.19g, 1.2mmol), dichlorobis(triphenylphosphine)palladium(II) (0.04g, 0.05mmol) and sodium hydrogen carbonate (0.25g, 3mmol) were added to a mixture of 1,2-dimethoxyethane (5ml) and water (1ml), followed by refluxing at 80°C for 3 hours. After cooling, the reaction mixture was concentrated, followed by addition of water. The mixture was extracted with ethyl acetate, and the extract was washed with water, dried and concentrated by evaporation of the solvent. The residue was purified by silica gel column chromatography (hexane : ethyl acetate = 5:1) to give 5-chloro-4-(2,4-difluorophenyl)-6-isopropylpyrimidine (0.22g, oil).
   ¹H-NMR(CDCl₃) δ 1.34(6H, d, J=6.8Hz), 3.62-3.65(1H, m), 6.92-7.06(2H, m), 7.44-7.51(1H, m), 9.07(1H, s)
(2) The compound prepared by the method of the Example 25(1) (0.4g, 1.49mmol) was added to concentrated sulfuric acid (5ml) under cooling with ice, followed by addition of fuming nitric acid (0.15g, 2.38mmol). The mixture was stirred at room temperature for 1 hour. The reaction mixture was poured on to ice, and extracted with chloroform. The extract was washed with water, dried and concentrated by evaporation of the solvent. The residue was purified by silica gel column chromatography (hexane : ethyl acetate = 5:1) to obtain 5-chloro-4-(2,4-difluoro-5-nitrophenyl)-6-isopropylpyrimidine (0.22g, oil).
   ¹H-NMR(CDCl₃) δ 1.35(6H, d, J=6.8Hz), 3.62-3.65(1H. m), 7.16-7.21(1H, m), 8.33-8.38(1H, m), 9.11(1H,s)
(3) Potassium cyanide (0.4g, 6.2mmol) was added to the compound produced by the method of Example 25(2) (1.3g, 4.2mmol) in DMF (10ml), followed by stirring at room temperature for 2 hours. Water (100ml) was added to the reaction mixture, and the mixture was extracted with chloroform. The extract was washed with aqueous sodium chloride, dried and concentrated by evaporation of the solvent. The residue was purified by silica gel column chromatography (hexane : ethyl acetate = 1:2) to give 5-chloro-4-(4-cyano-2-fluoro-5-nitrophenyl)-6-isopropylpyrimidine (0.34g).
   ¹H-NMR(CDCl₃) δ 1.35(6H, d, J=6.8Hz), 3.63-3.67(1H. m), 7.75(1H, d, J=8.1Hz), 8.56(1H, d, J=5.9Hz), 9.14(1H, s)
(4) Reduced iron (0.6g, 10.7mmol, 5eq) was suspended in acetic acid (5ml) and 2-propanol (10ml), and the suspension was heated to 80°C. To the mixture was dropwise added the compound produced by the method of the Example 25(3) (4.19g, 14mmol) in acetic acid (14ml) and 2-propanol solution (7ml) over 1 hour. The reaction was carried out at the same temperature for 30 minutes. After cooling, the insolubles were filtered off by the use of Celite layer, and the filtrate was concentrated. The residue was dissolved in ethyl acetate, washed with aqueous sodium hydrogen carbonate and brine, dried and concentrated by evaporation of the solvent. The residue was purified by silica gel column chromatography (hexane : ethyl acetate = 2:1) to give 5-chloro-4-(5-amino-4-cyano-2-fluorophenyl)-6-isopropylpyrimidine (2.38g).
   ¹H-NMR(CDCl₃) δ 1.34(6H, d, J=6.8Hz), 3.61-3.65(1H. m), 4.41(2H, br s), 7.82(1H, d, J=5.6Hz), 7.72(1H, d, J=8.7Hz), 9.07(1H, s)
(5) Ethanesulfonyl chloride (0.553g, 4.3mmol) was dropwise added to dichloromethane (10ml) solution containing the compound produced by the method of the Example 25(4) (0.25g, 0.86mmol) and triethylamine (0.435ml, 4.3mmol), followed by stirring at room temperature for 1 hour. Again, triethylamine (0.21g, 2.15mmol) and ethanesulfonyl chloride (0.277g, 2.15mmol) were added to the reaction mixture at room temperature for 1 hour, followed by concentration of the reaction mixture. The residue was purified by silica gel column chromatography (hexane : ethyl acetate = 4:1) to give a bis form product. The bis form product was dissolved in methanol 10ml, and potassium carbonate (0.33g, 2.39mmol) was added to the solution, followed by stirring at room temperature for 30 minutes. The reaction mixture was concentrated and neutralized with 1N hydrochloric acid. The resultant crystals were collected by filtration, washed with hexane and dried to obtain 5-chloro-4-[4-cyano-5-(ethylsulfonylamino)-2-fluorophenyl]-6-isopropylpyrimidine (0.23g).
   ¹H-NMR(DMSO-d₆) δ 1.35(6H, d, J=6.9Hz), 1.46(3H, t, J=7.4Hz), 3.24(2H, q, J=7.4Hz), 3.61-3.65(1H, br s), 7.04(1H, d, J=8.2Hz), 7.85(1H, d, J=5.9Hz), 9.10(1H, s) IR(nujol) 2251, 3100 cm⁻¹
   m.p. :160-162°C

### Example 26

### 5-Chloro-4-(4-chloro-2-fluoro-5-methoxyphenyl)-6-methoxypyrimidine (Compound No. H-1)

(1) 4,5,6-Trichloropyrimidine (1.10g, 6.00mmol) produced by the method of the Reference Example 11, phenylboronic acid (0.82g, 4.00mmol) produced by the method of the Reference Example 8, dichlorobis(tripehnylphosphine)palladium(II) (84mg, 0.12mmol, 3mol%) and sodium hydrogen carbonate (1.01g, 12.0mmol) were added to 1,2-dimethoxyethane (30ml) and water (7.5ml), and the reaction was carried out at 80°C for 1.5 hours. The reaction mixture was cooled with ice and was made acidic by addition of 2N hydrochloric acid. After addition of water (50ml), the mixture was extracted with ethyl acetate. The extract was dried and evaporated to give a residue, which was purified by silica gel column chromatography (hexane : ethyl acetate = 5:1) to obtain 5,6-dichloro-4-(4-chloro-2-fluoro-5-methoxyphenyl)-pyrimidine (0.59g).
   m.p. 116-117°C
   ¹H-NMR(CDCl₃) δ 3.93(3H,s,), 7.00(1H, d, J=6.0Hz), 7.29(1H, d, J=9.1Hz), 8.94(1H, s)
   ¹⁹F-NMR(CDCl₃) δ -120.10
   IR(Nujol) 1536, 1495, 1430, 1386, 1315, 1194, 1038, 967, 866, 745 cm⁻¹
(2) 28% Sodium methoxide (150mg, 0.78mmol) in methanol was dropwise added to a suspension of the compound produced by the method of the Example 26(1) (0.20g, 0.65mmol) in methanol (3ml). The mixture was stirred at the same temperature for 1 hour. The reaction mixture was concentrated, followed by addition of water, and the mixture was extracted with ethyl acetate. The extract was washed with water, dried and concentrated by evaporation of the solvent. Cold petroleum ether was added to the residue and the resultant crystals were collected by filtration to obtain 5-chloro-4-(4-chloro-2-fluoro-5-methoxyphenyl)-6-methoxypyrimidine (0.15g).
   ¹H-NMR(CDCl₃) 6 3.92(3H, s), 4.14(3H, s), 7.00(1H, d, J=6.0Hz), 7.26(1H, d, J=8.9Hz), 8.71(1H, s)
   ¹⁹F-NMR(CDCl₃) δ -120.74
   IR(Nujol) 1569, 1526, 1493, 1472, 1369, 1061, 1026 cm⁻¹
   m.p. : 142-143°C

### Example 27

### 5-Chloro-4-[4-cyano-5-(ethylsulfonylamino)-2-fluorophenyl]-6-methoxypyrimidine (Compoudn No. H-7)

(1) 4,6-dichloropyrimidine (14.9g, 0.1mol), 2,4-difluorophenylboronic acid (10.5g, 67mmol), dichlorobis(triphenylphosphine)palladium(II) (1.4g, 2mmol) and sodium hydrogen carbonate (16.8g, 0.2mol) were added to a mixture of 1,2-dimethoxyethane (300ml) and water (50ml), followed by refluxing for 3 hours. After cooling, the reaction mixture was concentrated and water was added to the residue. The mixture was adjusted to pH 3 with 2N hydrochloric acid, and extracted with chloroform. The extract was washed with water, dried and concentrated by evaporation with the solvent, followed by addition of hexane (200ml). The insolubles were removed by filtration. The filtrate was concentrated, and the residue was purified by silica gel column chromatography (from hexane : chloroform = 1:1 to hexane : ethyl acetate = 1:1) to obtain 4-chloro-6-(2,4-difluorophenyl)pyrimidine (17.2g).
   m.p.: 65-69°C
   ¹H-NMR(CDCl₃) δ 6.95-7.00(1H, m), 7.02-7.10(1H, m), 7.86(1H, m), 8.22-8.30(1H, m), 9.05(1H, s)
(2) The compound produced by the method of the Example 27(1) (6.9g, 30mmol) was dissolved in THF (50ml), and sodium methoxide (28% in MeOH, 11.6g (60mmol) was dropwise added. The mixture was stirred at the same temperature for 13 hours. The reaction mixture was concentrated, and water was added to the residue, followed by the extraction using chloroform. The extract was washed with water, dried and concentrated by evaporation of the solvent. The residue was purified by silica gel column chromatography (hexane : ethyl acetate = 5:1) to obtain 4-(2,4-difluorophenyl)-6-methoxypyrimidine (3.7g).
   ¹H-NMR(CDCl₃) δ 4.03(3H, s), 6.88-6.96(1H, m), 6.98-7.05(1H, m), 7.22(1H, s), 8.12-8.20(1H, m), 8.85(1H, s)
(3) Acetic acid (100ml) solution containing the compound produced by the method of the Example 27(2) was heated to 50°C, followed by dropwise addition of antiformin (100ml) over about 40 minutes. The reaction mixture was stirred at the same temperature for 30 minutes , followed by concentration under a reduced pressure. Water (100ml) and ethyl acetate (100ml) were added to the residue, and the ethyl acetate layer was separated, dried over anhydrous magnesium sulfate and concentrated by evaporation of the solvent. The residue was purified by silica gel column chromatography (hexane :ethyl acetate = 7:1) to obtain 5-chloro-4-(2,4-difluorophenyl)-6-methoxypyrimidine (7.08g).
   ¹H-NMR(CDCl₃) δ 4.14(3H, s), 6.94(1H, ddd, J=2.4, 8.9, 9.9Hz), 6.99-7.04 (1H, m), 7.48(1H, ddd, J=6.3, 8.3, 8.3Hz), 8.71 (1H, s)
   IR(nujol) 1614, 1567, 1509, 1433, 1398, 1387, 1015cm⁻¹
   m.p. :76-78°C
(4) The compound produced by the method of the Example 27(3) (21.19g, 82.57mmol) in concentrated sulfuric acid (100ml) was cooled, followed by dropwise addition of fuming nitric acid (5.24ml, 0.124mmol). The mixture was stirred at room temperature for 30 minutes. The reaction mixture was poured into iced water (500ml), and extracted with ethyl acetate (200ml). The extract was washed with water and aqueous sodium hydrogen carbonate, dried and concentrated by evaporating the solvent off. The resultant crystals were washed with hexane and collected by filtration to obtain 5-chloro-4-(2,4-difluoro-5-nitrophenyl)-6-methoxypyrimidine (20.39g).
   ¹H-NMR(CDCl₃) δ 4.16(3H, s), 7.18(1H, dd, J=9.0, 10.2Hz), 8.36(1H, dd, J=7.1, 8.1Hz), 8.36 (1H, s)
   ¹⁹F-NMR (CDCl₃) δ -96.44(1F, d, J=15.1Hz). -109.51(1H, d, J=15.1Hz)
   IR (nujol) 1563, 1540, 1502, 1416, 1394, 1356, 1072 cm⁻¹
   m.p. :84-88°C
(5) Potassium cyanide powder (4.78g, 71.6mmol) was portionwise added to the compound produced by the method of the Example 27(4) (18.00g, 59.67mmol) dissolved in DMF (75ml), followed by stirring at 50°C for 45 minutes. The reaction mixture was concentrated under a reduced pressure, and water (500ml) was added to the residue. The mixture was extracted with ethyl acetate. The extract was washed with brine, dried and concentrated by evaporation of the solvent. The residue was purified twice by silica gel column chromatography (hexane : ethyl acetate = 5:1) to obtain 5-chloro-4-(4-cyano-2-fluoro-5-nitrophenyl)-6-methoxypyrimidine (8.94g).
   ¹H-NMR(CDCl₃) δ 4.18(3H, s), 7.74(1H, d, J=8.1Hz),8.56 (1H, d, J=5.9Hz), 8.76 (1H, s)
   ¹⁹F-NMR (CDCl₃) δ -98.96
   IR (nujol) 2242, 1564, 1539, 1473, 1378, 1347, 1064, 1054 cm⁻¹
   m.p. :161-162°C
(6) Reduced iron (8.09g, 0.115mol) was suspended in acetic acid (30ml) and 2-propanol(50ml), and the mixture was heated to 70 to 80°C, followed by dropwise addition of acetic acid (90ml) solution containing the compound produced by the method of the Example 27(5) (8.94g, 29.0mmol) over 30 minutes. The mixture was stirred at the same temperature for 1 hour. The reaction mixture was filtered with Celite, and the filtrate was concentrated. The thus obtained crude product was purified by silica gel column chromatography (chloroform : acetone = 10:1) to obtain 4-(5-amino-4-cyano-2-fluorophenyl)-5-chloro-6-methoxypyrimidine (7.11g).
   ¹H-NMR(CDCl₃) δ 4.14(3H,s), 4.41(2H, br s), 6.82 (1H, d, J=5.4Hz), 7.22 (1H, d, J=8.6Hz), 8.70(1H, s)
   IR (nujol) 3381, 2230, 1642, 1569, 1527, 1504, 1442, 1380, 1086 cm⁻¹
   m.p. :186-189°C
(7) Triethylamine (2.98ml, 21.5mmol) was added to THF (20ml) solution containing the compound produced by the method of the Example 27(6) (2.00g, 7.18mmol), followed by addition of THF (10ml) solution containing ethanesulfonyl chloride (2.04ml, 21.5mmol) under cooling with ice. The mixture was stirred at room temperature for 2 hours. Triethylamine (1.49ml, 10.7mmol) was further added, and ethanesulfonyl chloride (1.02ml, 10.8mmol) in THF (5ml) was dropwise added thereto. The mixture wa stirred at room temperature for 1 hour. To the reaction mixture were added water (100ml) and chloroform (50ml), and the resultant layers were separated. The aqueous layer was further extracted with chloroform (50ml). The chloroform layers were combined, washed with water, dried and concentrated by evaporated of the solvent. The resultant crystals from isopropyl ether were collected by filtration to obtain 5-chloro-4-[4-cyano-5-(N,N-bisethylsulfonyl)amino]-2-fluorophenyl)-6-methoxypyrimidine (3.16g).
   ¹H-NMR(CDCl₃) δ 1.53(3H,t,J=7.4Hz), 3.77 (2H,m), 4.16(3H,s), 7.62 (1H,d,J=8.3Hz), 7.69(1H,d,J=6.0Hz). 8.73(1H,s)
(8) Aqueous solution (5ml) containing sodium hydroxide (0.28g, 7.00mmol) was dropwise added to the compound produced by the method of the Example 27(7) in THF 25ml, followed by stirring for 30 minutes at room temperature. Further aqueous solution (5ml) containing sodium hydroxide (0.23g, 5.75mmol) was dropwise added, followed by stirring at room temperature for 30 minutes. Further aqueous solution (5ml) containing sodium hydroxide (0.25g, 6.25mmol) was dropwise added, and the mixture was stirred at room temperature. The reaction mixture was concentrated, followed by addition of water. The mixture wa adjusted to pH 3 with 2N hydrochloric acid. The mixture was extracted with chloroform (50ml), and the extract was washed with water, dried and concentrated by evaporation of the solvent. The residue was purified by silica gel column chromatography (chloroform : ethanol = 10:1) to obtain 5-chloro-4-[4-cyano-5-(ethylsulfonylamino)-2-fluorophenyl]-6-methoxypyrimidine (2.19g).
   ¹H-NMR(CDCl₃) δ 1.46(3H, t, J=7.4Hz), 3.22 (2H, q, J=7.4Hz), 4.16 (3H, s), 7.33 (1H, s), 7.45(1H, d, J=8.2Hz), 7.81 (1H, d, J=5.9Hz), 8.76(1H, s)
   ¹⁹F-NMR (CDCl₃) δ -114.12
   IR (nujol) 2700-3100, 2239, 1578, 1507, 1469, 1444, 1384, 1334, 1145 cm⁻¹
   m.p. : 194-196°C

### Example 28

### 4,5-Dichloro-6-[4-cyano-5-(ethylsulfonylamino)-2-fluorophenyl]pyrimidine (Compound No. H-16)

(1) The compound produced by the method of the Example 27 (2.14g, 5.77mmol) was added to dioxane (25ml) and 2N hydrochloric acid (25ml), followed by refluxing under heating for 1 hour. The reaction mixture was concentrated, and diethyl ether was added to the residue. The resultant crystals were collected by filtration to give 5-chloro-6-[4-cyano-5-(ethylsulfonylamino)-2-fluorophenyl]pyrimidin-4(3H)-one (2.04g).
   ¹H-NMR(CDCl₃) δ 1.31(3H, t, J=7.3Hz), 3.21(2H, q, J=7.3Hz), 7.61(1H, d, J=6.2 Hz), 8.09 (1H, d, J=9.2Hz), 8.34(1H, s), 10.24(1H, s), 13.41(1H, br)
   IR (nujol) 2700-3100, 2232, 1674, 1603, 1506, 1402, 1337, 1151, 1050, 923 cm⁻¹
   m.p. :249-252°C
(2) Phosphorous oxychloride (10ml) was added to the compound produced by the method of the Example 28(1)(1.64g, 4.61mmol), followed by refluxing under heating for 1 hour. After cooling, the reaction mixture was concentrated, and the residue was purified by silica gel column chromatography (chloroform : acetone = 10:1). The thus obtained crystals were washed with hexane and collected by filtration to obtain 4,5-dichloro-6-[4-cyano-5-(ethylsulfonylamino)-2-fluorophenyl]pyrimidine (1.68g).
   ¹H-NMR(CDCl₃) δ 1.47(3H, t, J=7.4Hz), 3.25(2H, q, J=7.4Hz), 7.02(1H, s), 7.50 (1H, d, J=8.3Hz), 7.88(1H, d, J=5.9Hz), 8.98(1H, s)
   IR (nujol) 3100(br), 2230, 1547, 1510, 1443, 1350, 1335, 1156, 899 cm⁻¹
   m.p. :149-150°C

### Example 29

### 5-Chloro-6-[4-cyano-5-(ethylsulfonylamino)-2-fluorophenyl]-6-methylaminopyrimidine (Compound No. H-20)

The compound obtained by the method of the Example 28 (0.20g, 0.533mmol) in THF (2ml) was added to 40% aqueous methylamine solution (0.15g, 1.93mmol) in THF(3ml). The mixture was stirred at room temperature for 1 hour, followed by concentration of the reaction mixture. Ethyl acetate was added to the residue, and the mixture was washed with water, dried and concentrated by evaporation of the solvent. The resultant crystals were washed with isopropyl ether and collected by filtration to obtain 5-chloro-6-[4-cyano-5-(ethylsulfonylamino)-2-fluorophenyl]-6-methylaminopyrimidine (0.177g).
¹H-NMR(CDCl₃) δ 1.45(3H, t, J=7.4Hz), 3.17(3H, d, J=5.0Hz), 3.21(2H, q, J=7.4Hz), 5.78(1H, br), 7.41(1H, d, J=8.2Hz), 7.72(1H, d, J=5.9Hz), 8.03(1H, br), 8.64(1H, s)
IR (nujol) 3391, 2600-3000, 2230, 1595, 1504, 1377, 1334, 1150, 1047 cm⁻¹
m.p. :211-212°C

### Example 30

### 5-Chloro-4-[4-cyano-5-(ethylsulfonylamino)-2-fluorophenyl]-6-methoxyaminopyrimidine (Compound No. H-27)

0.1g/ml aqueous sodium hydroxide (0.64ml, 1.60mmol) was added to 2ml aqueous solution containing O-methylhydroxylamine hydrochloride (134mg, 1.60mmol), followed by cooling with ice. After addition of THF (3ml) solution containing the compound produced by the method of the Example 28 (0.20g, 0.533mmol) dissolved in THF (3ml), the mixture was stirred at room temperature for 1 hour. O-Methylhydroxyamine hydrochloride (45mg, 0.54mmol) and 0.1g/ml aqueous sodium hydroxyde (0.21ml, 0.53mmol) were additionally added, followed by stirring at room temperature for 53 hours. Furthermore, O-methylhydroxylamine hydrochloride (90mg, 1.08mmol) and 0.1g/ml aqueous sodium hydroxyde (0.42ml, 1.05mmol) were added, followed by stirring at room temperature for 17 hours. The insolubles were removed by filtration, and the filtrate was concentrated. The residue was purified by silica gel column chromatography (hexane : ethyl acetate = from 1:1 to 2:1) to obtain 5-chloro-4-[4-cyano-5-(ethylsulfonylamino)-2-fluorophenyl]-6-methoxyaminopyrimidine (0.137g).
¹H-NMR(CDCl₃) δ 1.31(3H, t, J=7.2Hz), 3.20(2H, q, J=7.4Hz), 3.81 (3H, s), 7.54(1H, d, J=5.4Hz), 7.76(1H, s), 8.03 (1H, d, J=9.1Hz,), 10.18 (1H, s), 11.79 (1H, s)
IR (nujol) 3268, 2227, 1622, 1504, 1329, 1146, 1060, 1035 cm⁻¹
m.p. :188-189°C

### Example 31

### 4-(4-Chloro-2-fluoro-2-methoxyphenyl)-5,6-dimethylpyrimidine (Compound No. J-1)

4-Chloro-5,6-dimethylpyrimidine produced by the method of the Reference Example 12 (1.28g, 9mmol), phenylboronic acid produced by the method of the Reference Example 8 (2.04g, 10mmol), dichlorobis(triphenylphosphine)palladium(II) (0.19g, 0.27mmol) and sodium hydrogen carbonate (2.27g, 27mmol) were added to a mixture of 1,2-dimethoxyethane (60ml) and water (10ml), followed by refluxing the mixture under heating for 2.5 hours. After cooling, the reaction mixture was concentrated, and water was added to the residue. The mixture was adjusted to pH 5 with 2N hydrochloric acid, and chloroform was added. The chloroform layer was washed with water, dried and concentrated by evaporation of the solvent. The residue was purified by silica gel column chromatography (hexane : ethyl acetate =2:1) to obtain 4-(4-chloro-2-fluoro-5-methoxypehnyl)-5,6-dimethylpyrimidine (1.97g).
m.p.156-157°C
¹H-NMR(CDCl₃) δ 2.20(3H, d, J=2.9Hz), 2.59(3H, s), 3.93(3H, s), 7.01(1H, d, J=6.1Hz), 7.24(1H, d, J=8.9Hz), 8.99(1H, s)
¹⁹F-NMR(CDCl₃) δ -122.6
IR(Nujol) 1621, 1563, 1261, 1194, 1062, 898, 767 cm⁻¹

### Example 32

### 4-(4-Chloro-2-fluoro-5-propargyloxyphenyl)-5,6-dimethyl pyrimidine (Compound No. J-3)

(1) The compound produced by the method of the Example 31 (0.93g, 3.5mmol) was dissolved in methylene chloride (10ml). Under cooling with ice, boron tribromide (1M-methylene chloride solution) (7ml) was dropwise added, followed by stirring at room temperature for 2 hours. The reaction mixture was poured into iced water, followed by extraction with chloroform. The extract was dried and concentrated by evaporating the solvent off. The residue was purified by silica gel column chromatography (hexane : ethyl acetate = 1:2) to give 4-(4-chloro-2-fluoro-5-hydroxyphenyl)-5,6-dimethylpyrimidine (0.68g).
   m.p.176-178°C
   ¹H-NMR(CDCl₃) δ 2.19(3H, d, J=2.1Hz), 2.60(3H, s), 7.06(1H, d, J=6.4Hz), 7.16(1H, d, J=8.6Hz), 7.20(1H, br s), 8.99(1H, s)
   ¹⁹F-NMR(CDCl₃) δ -123.8
   IR(Nujol) 1621, 1566, 1181, 1061, 863, 756 cm⁻¹
(2) The compound produced by the method of the Example 32(1) (0.18g, 0.71mmol) and potassium carbonate (0.12g, 0.87mmol) were added to DMF (6ml), followed by dropwise addition of propargyl bromide (0.13g, 1.05mmol) at room temperature. The mixture was stirred at the same temperature for 3.5 hours. Water was added to the reaction mixture and the mixture was extracted with ethyl acetate. The extract was washed with water, dried and concentrated by evaporation of the solvent. The residue was purified by silica gel column chromatography (hexane :ethyl acetate = 1:1) to obtain 4-(4-chloro-2-fluoro-5-propargyloxyphenyl)-5,6-dimethylpyrimidine (0.18g).
   m.p.131-133°C
   ¹H-NMR(CDCl₃) δ 2.20(3H, d, J=2.6Hz), 2.55(1H, t J=2.4Hz), 2.59(3H, s), 4.80(2H,d, J=2.4Hz), 7.16(1H, d, J=6.1Hz), 7.24-7.27(1H, m), 9.00(1H, s)
   ¹⁹F-NMR(CDCl₃) δ -121.5
   IR(Nujol) 3300, 2122, 1613, 1564, 1258, 1187, 1057 cm⁻¹

### Example 33

### 4-(4-Chloro-2-fluoro-5-methoxyphenyl)-5-methyl-6-trifluoromethylpyrimidine (Compound No. J-5).

4-Chloro-5-methyl-6-trifluoromethylpyrimidine (1.42g, 7.2mmol) produced by the method of the Reference Example 13, phenylboronic acid (1.64g, 8mmol) produced by the method of the Reference Example 8, dichlorobis(triphenylphosphine)palladium(II)(0.15g, 0.21mmol) and sodium hydrogen carbonate (1.82g, 22mmol) were added to a mixture of 1,2-dimethoxyethane (50ml) and water (10ml), followed by refluxing under heating for 1.5 hours. After cooling, the reaction mixture was neutralized with 2N hydrochloric acid (pH value = 5), followed by concentration. Water was added to the residue, and the mixture was extracted with chloroform. The extract was washed with water, dried and concentrated by evaporating the solvent. The residue was purified by silica gel column chromatography (hexane : ethyl acetate = 6:1) to obtain 4-(4-chloro-2-fluoro-5-methoxyphenyl)-5-methyl-6-trifluoromethyl pyrimidine (1.99g).
m.p.144-146°C
¹H-NMR(CDCl₃) δ 2.40(3H, br s), 3.94(3H, s), 7.01(1H, d, J=6.1Hz), 7.29(1H, d, J=8.4Hz), 9.25(1H, s)
¹⁹F-NMR(CDCl₃) δ -67.2, -122.6
IR(Nujol) 1620, 1562, 1197, 1137, 1048, 766 cm⁻¹

### Example 34

### 4-(4-Chloro-2-fluoro-5-propargyloxyphenyl)-5-methyl-6-trifluoromethylpyrimidine (Compound No. J-7)

(1) The compound produced by the method of the Example 33 (1.70g, 5.3mmol) was added to a mixture of acetic acid (10ml) and 47% hydrobromic acid (40ml), followed by stirring at 120°C for 8 hours. After cooling, the reaction mixture was poured into iced water, followed by extraction with ethyl acetate. The extract was washed with brine, dried and concentrated by evaporating the solvent. The residue was purified by silica gel column chromatography (hexane : ethyl acetate =3:1) to obtain 4-(4-chloro-2-fluoro-5-hydroxy phenyl)-5-methyl-6-trifluoromethyl pyrimidine (1.43g).
   m.p.141-142°C
   ¹H-NMR(CDCl₃) δ 2.32-2.55(3H, m), 5.85(1H, s), 7.11(1H, d, J=6.4Hz), 7.24(1H, d, J=8.9Hz), 9.25(1H, s)
   IR(Nujol) 3400-3000, 1622, 1564, 1149, 1032, 887, 778, 718 cm⁻¹
(2) The compound produced by the method of the Example 34(1) (0.19g, 0.62mmol) and potassium carbonate (0,10g, 0.72mmol) were added to DMF (6ml), followed by dropwise addition of propargyl bromide (0.11g, 0.93mmol) at room temperature. The mixture was stirred at 40°C for 2 hours. The reaction mixture was poured into iced water, followed by extraction with ethyl acetate. The extract was washed with brine, dried and concentrated by evaporating the solvent. The residue was purified by silica gel column chromatography (hexane : ethyl acetate = 4:1) to 4-(4-chloro-2-fluoro-5-propargyloxyphenyl)-5-methyl-6-trifluoromethylpyrimidine (0.17g).
   m.p.90-92°C
   ¹H-NMR(CDCl₃) δ 2.38-2.42(3H, m), 2.57(1H, t, J=2.4Hz), 4.87(2H,d, J=2.4Hz), 7.18(1H, d, J=6.2Hz), 7.31(1H, d, J=9.0Hz), 9.25(1H, s)
   ¹⁹F-NMR(CDCl₃) 6 -67.4, -121.3
   IR(Nujol) 3256, 2119, 1611, 1562, 1224, 1188, 1138, 1047, 874 cm⁻¹

### Example 35

### 4-(4-Cyano-5-ehtylsulfonylamino-2-fluorophenyl)-5-methyl-6-difluoromethylpyrimidine (Compound No. J-12)

(1) 4-Chloropyrimidine (6.25g, 35mmol) produced by the method of the Reference Example 14, 2,4-difluorophenylboronic acid (6.0g, 38mmol), dichlorobis(triphenyl phosphine)palladium(II)(0.77g, 1.1mmol) and sodium hydrogen carbonate (8.9g, 106mmol) were added to a mixture of 1,2-dimethoxyethane (240ml) and water (50ml), followed by refluxing under heating for 2 hours. After cooling, the reaction mixture was concentrated, and water was added to the residue, followed by neutralizing the mixture with 2N hydrochloric acid (pH value = 5). The mixture was extracted with chloroform. The extract was washed with water, dried and concentrated by evaporating the solvent. The residue was purified by silica gel column chromatography (hexane : ethyl acetate = 7:1) to obtain 4-(2,4-difluorophenyl)-6-difluoromethyl-5-methylpyrimidine (8.6g).
   m.p. 54-55°C
   ¹H-NMR(CDCl₃) δ 2.39(3H, s), 6.70(1H, t, J=54.0Hz), 6.93-7.00(1H, m), 7.05-7.08(1H, m), 7.44-7.51(1H, m), 9.17(1H,s)
   ¹⁹F-NMR(CDCl₃) δ -107.2, -109.9, -116.9
   IR(Nujol) 1616, 1595, 1564, 1376, 1268, 1030, 966 cm⁻¹
(2) Under cooling with ice, the compound produced by the method of the Example 35(1) (1.28g, 5mmol) was added to concentrated sulfuric acid (5ml), and fuming nitric acid (0.24ml) was added, followed by stirring at room temperature for 2 hours. The reaction mixture was poured into iced water. The resultant crystals were collected by filtration, washed with water and dried to give 6-difluoromethyl-4-(2,4-difluoro-5-nitrophenyl)-5-methylpyrimidine (1.45g).
   m.p.85-86°C
   ¹H-NMR(CDCl₃) δ 2.42(3H, s), 6.71(1H, t, J=53.9Hz), 7.21(1H, d.d, J=9.1, 10.0Hz), 8.35(1H, d.d, J=7.2, 7.6Hz), 9.21(1H, s)
   ¹⁹F-NMR(CDCl₃) δ -98.8, -109.3, -116.8
   IR(nujol) 1634, 1596, 1544, 1370, 1354, 1270, 1155, 1053 cm⁻¹
(3) Potassium cyanide (3.0g, 46mmol) was added to the compound produced by the method of the Example 35(2) (9.34g, 31mmol) in DMF solution (80ml), and the reaction was carried out at 50°C for 5 hours. Water (100ml) was added to the reaction mixture, followed by extraction with ethyl acetate. The extract was washed with brine, dried and concentrated by evaporating the solvent. The residue was purified by silica gel column chromatography (hexane : ethyl acetate = 5:1) to obtain 4-(4-cyano-2-fluoro-5-nitrophenyl)-6-difluoromethyl-5-methylpyrimidine(4.3g).
   ¹H-NMR(CDCl₃) δ 2.43(3H, s), 6.72(1H, t, J=53.8Hz), 7.78(1H, d, J=8.2Hz), 8.56(1H,d, J=6.0Hz), 9.25(1H, s)
   ¹⁹F-NMR(CDCl₃) δ -101.6, -116.7
   IR(nujol) 2240, 1629, 1562, 1376, 1352, 1097, 156 cm⁻¹
   m.p. 85-86°C
(4) Reduced iron (3.35g, 54mmol, 5eq) was suspended in acetic acid (30ml) and 2-propanol (50ml), followed by heating the mixture at 70°C. To the mixture was dropwise added the compound produced by the method of the Example 35(3) (4.19g, 14mmol) dissolved in a mixture of acetic acid (10ml) and 2-propanol (20ml) over 35 minutes, and the reaction was carried out at 70°C for 1 hour. After cooling, the reaction mixture was filtered through Celite layer to remove the insolubles, followed by the concentration of the filtrate. The residue was dissolved in ethyl acetate. The solution was washed with aqueous sodium hydrogen carbonate and brine, dried and concentrated by evaporating the solvent. The residue was purified by silica gel column chromatography (hexane : ethyl acetate = 2:3) to give 4-(5-amino-4-cyano-2-fluorophenyl)-6-difluoromethyl-5-methylpyrimidine (2.38g).
   H-NMR(DMSO-*d*₆) δ 2.28(3H, s), 6.18(2H, br s), 6.87(1H, d, J=6.1Hz), 7.19(1H, t, J=53.1Hz), 7.54(1H, d, J=9.6Hz) 9.25(1H, s)
   ¹⁹F-NMR(DMSO-*d*₆) δ: -119.1, -131.6
   IR(nujol) 3443, 3364, 3254, 2222, 1654, 1567, 1378, 1026, 881 cm⁻¹
   m.p. : 213-214°C
(5) Ethanesulfonyl chloride (0.72g, 5.6mmol) was dropwise added to tetrahydrofurane (8ml) solution containing the compound produced by the method of the Example 35(4) (0.31g, 1.1mmol) and triethylamine (0.69ml, 6mmol), followed by stirring the mixture at room temperature for 4.5 hours. To the reaction mixture were again added triethylamine (0.43g, 4.3mmol) and ethanesulfonyl chloride (0.54g, 4.2mmol), and the resultant mixture was stirred at room temperature for 9 hours. The reaction mixture was concentrated, and to the residue was added water. The mixture was extracted with ethyl acetate. The extract was washed with brine, dried and concentrated by evaporating the solvent. The residue was purified by silica gel column chromatography (hexane : ethyl acetate =2:1) to give a bis form product. The bis form product was dissolved in dioxane (5ml), followed by addition of 2N aqueous sodium hydroxide (1ml, 2mmol) under cooling with ice, and the reaction was carried out at room temperature for 2 hours. The reaction mixture was concentrated, and water (50ml) was added to the residue. The mixture was made acidic by addition of 2N hydrochloric acid. The precipitated crystals were collected by filtration, washed with water and dried to obtain 4-[4-cyano-5-(ethylsulfonylamino)-2-fluorophenyl]-6-difluoromethyl-5-methylpyrimidine (0.56g).
   ¹H-NMR(DMSO-d₆) δ 1.31(3H, t, J=7.2Hz), 2.29(3H, s), 3.22(2H, q, J=7.1Hz), 7.26(1H, t, J=53.0Hz), 7.66(1H, d, J=6.3Hz), 8.11(1H, d,J=9.3Hz), 9.30(1H, s), 10.27(1H, br s)
   ¹⁹F-NMR(DMSO-*d*₆) δ: -116.2, -119.0
   IR(nujol) 2230, 1570, 1506, 1147, 1068, 1056, 921 cm⁻¹
   m.p. :188-190°C

### Example 36

### 4-[5-(N-acetyl-N-ethylsulfonylamino)-4-cyano-2-fluorophenyl]-5-chloro-6-difluoromethylpyrimidine (Compound No. J-14)

Triethylamine (0.061g, 0.5mmol) was added to the compound produced by the method of the Example 35(5) (0.15g, 0.4mmol) dissolved in acetonitrile (3ml), followed by addition of acetyl chloride (0.039g, 0.5mmol). The resultant mixture was stirred at room temperature for 1 hour, followed by evaporating the solvent. Water was added to the residue, and the mixture was extracted with ethyl acetate. The extract was washed with brine, dried and concentrated by evaporating the solvent. The residue was purified by silica gel column chromatography (hexane : ethyl acetate = 1:1). The resultant crystals were washed with petroleum ether and collected by filtration to obtain 4-[5-(N-acetyl-N-ethylsulfonylamino)-4-cyano-2-fluorophenyl]-5-chloro-6-difluoromethylpyrimidine (0.14g).
¹H-NMR(CDCl₃) δ 1.55-1.59(3H, m), 2.18(3H, s), 2.42(3H, s), 3.70-4.00(2H, br s), 6.71(1H, t, J=53.9Hz), 7.66(1H, d, J=8.2Hz), 7.71(1H, d, J=6.1Hz), 9.21(1H, s)
¹⁹F-NMR(CDCl₃) δ -108.4, -116.8
IR(nujol) 2243, 1708, 1564, 1504, 1342, 1232, 1154, 1057 cm⁻¹
m.p. : 123-124°C

### Example 37

### 4-(4-cyano-5-methylsulfonylamino-2-fluorophenyl)-6-methoxy-5-methylpyrimidine (Compound No. J-16)

(1) The compound produced by the method of the Reference Example 15 (6.2g, 38mmol) was dissolved in THF (100ml), followed by addition of 2,4-difluorophenylboronic acid (7.2g, 45.6mmol), tetrakis(triphenylphosphine)palladium (2.3g, 2mmol), potassium carbonate (12.6g, 91.3mmol) and water (30ml), and the mixture was refluxed under heating for 4 hours. 2,4-Difluorophenylboronic acid (2.4g, 15mmol) and tetrakistriphenylphosphine palladium (0.8g, 0.7mmol) were additionally added, and the mixture was refluxed under heating for 2 hours. The reaction mixture was concentrated, and water was added to the residue, followed by extraction with chloroform. The extract was washed with water, dried and concentrated by evaporationg the solvent. The residue was purified by silica gel column chromatography (hexane : ethyl acetate = 5:1) to obtain 4-chloro-6-(2,4-difluorophenyl)-5-methylpyrimidine (4.6g, oil).
   ¹H-NMR(CDCl₃) δ 2.30(3H, s), 6.90-7.10(2H, m), 7.43-7.52(1H, m), 8.89(1H, s)
(2) The compound produced by the method of the Example 37(1) (11.7g, 48.6mmol) was dissolved in methanol (100ml), followed by dropwise addition of sodium methoxide (28% in MeOH) (14.0g, 73mmol), and the resultant mixture was stirred at room temperature for 2 hours. The reaction mixture was concentrated, and water was added to the residue. The precipitated crystals were collected by filtration and dried to obtain 4-(2,4-difluorophenyl)-6-methoxy-5-methtylpyrimidine (10.4g).
   m.p.88-89°C
   ¹H-NMR(CDCl₃) δ 2.06(3H, s), 4.05(3H, s), 6.85(2H, m), 7.40-7.50(1H, m), 8.69(1H, s)
(3) The compound produced by the method of the Example 37(2) (10.4g, 43.9mmol) was added to concentrated sulfuric acid (53ml), followed by dropwise addition of fuming nitric acid (2.9ml). The mixture was stirred at the same temperature for 1 hour. The reaction mixture was poured on ice, and the mixture was extracted 10 times with ethyl acetate. The extract was washed with aqueous sodium hydrogen carbonate and water, dried and concentrated by evaporating the solvent. The residue was dissolved in a mixture of hexane : ethyl acetate = 1:1 and purified by short silica gel column chromatography (hexane : ethyl acetate = 3:1) to obtain 4-(2,4-difluoro-5-nitrophenyl)-6-methoxy-5-methylpyrimidine (9.45g).
   m.p.114-117°C
   ¹H-NMR(CDCl₃) δ 2.08(3H, s), 4.08(3H, s), 7.12-7.20(1H, m), 8.28(1H, m), 8.71(1H, s)
(4) The compound produced by the method of the Example 37(3) (9.45g, 33.6mmol) was dissolved in DMF (110ml), followed by addition of potassium cyanide (2.84g, 43.6mmol) at room temperature, and the resultant mixture was stirred at the same temperature for 1.5 hours. The reaction mixture was poured into iced water, and the mixture was extracted with ethyl acetate. The extract was washed with brine, dried and concentrated by evaporating the solvent. The residue was purified by silica gel column chromatography (hexane : ethyl acetate = 2:1) to obtain 4-(4-cyano-2-fluoro-5-nitrophenyl)-6-methoxy-5-methylpyrimidine (0.5g).
   m.p.129-131°C
   ¹H-NMR(CDCl₃) δ 2.09(3H, s), 4.09(3H, s), 7.71(1H, d, J=8.2Hz), 8.54(1H, d, J=6.0Hz), 8.73(1H, s)
   IR(nujol) 2360, 2245, 1547, 1375, 1348, 1073, 920 cm⁻¹
(5) Reduced iron (0.85g, 2.3mmol) was added to a mixture of acetic acid (10ml) and isopropanol (5ml), followed by dropwise addition of the compound produced by the method of the Example 37(4) (1.0g, 3.46mmol) dissolved in acetic acid (4ml). The resulting mixture was stirred at 70 to 80°C for 30 minutes. After cooling, THF (10ml) was added to the reaction mixture, and the insolubles were removed by filtration of the mixture through Celite layer. The filtrate was concentrated. To the residue was added water, and the mixture was neutralized with aqueous sodium hydrogen carbonate. The mixture was extracted with ethyl acetate, washed with water, dried and concentrated by evaporating the solvent. The residue was purified by silica gel column chromatography (chloroform : acetone = 5:1) to obtain 4-(5-amino-4-cyano-2-fluorophenyl)-6-methoxy-5-methylpyrimidine (0.5g).
   ¹H-NMR(CDCl₃) δ 2.04(3H, s), 4.03(3H, s), 4.35(2H, br.s), 6.79(1H, d, J=5.7Hz), 7.17(1H, t, J=8.8Hz), 8.66(1H, s)
(6) The compound produced by the method of the Example 37(5) (0.27g, 1.05 mmol) and triethylamine (0.31g, 3.1mmol) were dissolved in THF (7ml), followed by dropwise addition of methanesulfonyl chloride (0.35g, 3.1mmol) dissolved in THF under cooling with ice. The resultant mixture was stirred under cooling with ice for 1 hour. Further, triethylamine (0.1g, 1.0mmol) and methanesulfonylchloride (0.12g, 1.0mmol) were added, and the resulting mixture was stirred at room temperature for 5 hours. The reaction mixture was concentrated. The residue was dissolved in chloroform, washed with water, dried and concentrated by evaporating the solvent to obtain a bis form product (0.5g).
   ¹H-NMR(CDCl₃) δ 2.09(3H, s), 3.54(6H, s), 4.07(3H, s), 7.58-7.67(2H, m), 8.71(1H, s)
(7) The bis form product (0.5g) obtained by the method of the Example 37(6) was dissolved in a mixture of THF (5ml) and water (2ml), followed by dropwise addition of 2N sodium hydroxide (0.6ml). The resulting mixture was stirred at room temperature for 1 hour. Further 2N sodium hydroxide (0.5ml) was dropwise added, followed by stirring at room temperature for 1 hour. Furthermore, 2N sodium hydroxide (0.5ml) was dropwise added, followed by stirring at room temperature for 1 hour. Water (20ml) was added to the reaction mixture, and the mixture was adjusted to pH 2 with 2N hydrochloric acid. The precipitated crystals were collected by filtration, washed with water and dried to obtain 4-(4-cyano-5-methylsulfonylamino-2-fluorophenyl)-6-methoxy-5-methylpyrimidine (0.3g).
   m.p. 218-220°C
   ¹H-NMR(CDCl₃) δ 2.07(3H, s), 3.13(2H, s), 4.08(3H, s), 7.43(1H, d, J=8.16Hz), 7.70(1H, d, J=5.9Hz), 8.75(1H, s), 8.75(1H, s)
   IR(Nujol) 2232, 1581, 1473, 1378, 1329, 1148, 1108, 1004, 722 cm⁻¹
   ¹⁹F-NMR(CDCl₃) δ -115.17(s)

### Example 38

### 4-(4-Chloro-2-fluoro-5-methoxyphenyl)-5-cyano-6-methoxypyrimidine (Compound No. K-1)

4-Chloropyrimidine produced by the method of the Reference Example 16 (0.17g, 1mmol), sodium hydrogen carbonate (0.25g, 2.98mmol) and dichlorobis(triphenylphosphine)palladium(II)(0.03g, 0.04mmol) were added to 1,2-dimethoxyethane (10ml) and water (2ml), followed by addition of 4-chloro-2-fluoro-5-methoxyphenylboronic acid (0.21g, 1.03mmol) produced by the method of the Reference Example 8 at 70°C. The mixture was stirred for 3 hours. After cooling, the mixture was concentrated, and water was added to the residue. The mixture was extracted with ethyl acetate. The extract was dried and concentrated by evaporating the solvent. The residue was purified by silica gel column chromatography (hexane : ethyl acetate = 2:1) to obtain 4-(4-chloro-2-fluoro-5-methoxyphenyl)-5-cyano-6-methoxypyrimidine (0.17g).
m.p. 183-185°C
¹H-NNR(CDCl₃) δ 3.95(3H, s), 4.20(3H, s), 7.14(1H, d, J=6.1Hz), 7.33(1H, d, J=9.3Hz), 8.96(1H, s)
IR(Nujol) 2310 cm⁻¹

### Example 39

### 4-(4-Cyano-5-ethylsulfonylamino-2-fluorophenyl)-5-cyano-6-methoxypyrimidine (Compound No. K-3)

(1) 4-Chloropyrimidine produced by the method of the Reference Example 16 (0.63g, 3.72mmol), dichlorobis(triphenylphosphine)palladium(II)(0.04g, 0.05mmol) and sodium hydrogen carbonate (0.25g, 3mmol) were added to a mixture of 1,2-dimethoxyethane (5ml) and water (1ml), followed by portionwise addition of 2,4-difluorophenylboronic acid (0.63g, 3.99mmol) at 80°C. The mixture was stirred at the same temperature for 3 hours. After cooling, the reaction mixture was concentrated, and water was added to the residue. The mixture was neutralized with 2N hydrochloric acid and was extracted with ethyl acetate. The extract was washed with water, dried and concentrated by evaporating the solvent. The residue was purified by silica gel column chromatography (hexane : ethyl acetate = 2:1) to obtain 4-(2,4-difluorophenyl)-5-cyano-6-methoxypyrimidine (0.65g, oil).
   ¹H-NMR(CDCl₃) δ 4.19(3H,s), 6.99-7.09(2H, m), 7.64-7.67(1H, m), 8.96(1H, s)
(2) The compound produced by the method of the Example 39(1) (1.0g, 4.1mmol) was added to concentrated sulfuric acid (10ml), followed by addition of fuming nitric acid (0.28g, 4.4mmol) under cooling with ice. The mixture was stirred at room temperature for 90 minutes, and fuming nitric acid (0.15g, 2.4mmol) was further added. The reaction mixture was poured on ice, and the mixture was extracted with chloroform. The extract was washed with water, dried and concentrated by evaporating the solvent, followed by addition of hexane to the residue to obtain the crystals. The crystals were collected by filtration and dried to obtain 4-(2,4-difluoro-5-nitrophenyl)-5-cyano-6-methoxypyrimidine (0.9g).
   ¹H-NMR(CDCl₃) δ 4.22(3H, s), 7.23-7.28(1H, m), 8.49-8.53(1H, m), 9.00(1H, s)
(3) Potassium cyanide (0.3g, 4.6mmol) was added to the compound produced by the method of the Example 39(2) (0.9g, 3.0mmol) dissolved in DMF (10ml), and the reaction was carried out at room temperature for 3 hours. Brine was added to the reaction mixture, followed by extraction with ethyl acetate. The extract was washed with brine, dried and concentrated by evaporating the solvent. The residue was purified by silica gel column chromatography (hexane : ethyl acetate = 2:1) to obtain 4-(4-cyano-2-fluoro-5-nitrophenyl)-5-cyano-6-methoxypyrimidine (0.28g).
   ¹H-NMR(CDCl₃) δ 4.24(3H, s), 7.82(1H, d, J=8.4Hz), 8.67(1H, d, J=6.0Hz), 9.04(1H, s)
(4) Reduced iron (0.3g, 5.36mmol, 5eq.) was suspended in a mixture of acetic acid (3ml) and 2-propanol (5ml) and the temperature of the mixture was raised to 80°C. To the mixture was followed by dropwise added the compound produced by the method of the Example 39(3) (0.28g, 0.94mmol) dissolved in a mixture of acetic acid (7ml) and 2-propanol (4ml) over 1.5 hours. The reaction was carried out at the same temperature for 30 minutes. After cooling, the insolubles were removed by the filtration of the reaction mixture through Celite layer, and the filtrate was concentrated. The residue was purified by silica gel column chromatography (hexane : ethyl acetate = from 2:1 to 1:1) to obtain 4-(5-amino-4-cyano-2-fluorophenyl)-5-cyano-6-methoxypyrimidine (0.2g).
   ¹H-NMR(CDCl₃) δ 4.20(3H, s), 4.48(2H, br s), 6.95(1H, d, J=5.6Hz), 7.30(1H, d, J=8.7Hz), 8.97(1H, s)
(5) Ethanesulfonyl chloride (0.24g, 1.87mmol) was dropwise added to dichloromethane (7ml) solution containing the compound produced by the method of the Example 39(4) (0.1g, 0.37mmol) and triethylamine (0.19g, 1.88mmol). The mixture was stirred at room temperature for 3 hours. Triethylamine (0.095g, 0.94mmol) and ethanesulfonyl chloride (0.12g, 0.93mmol) were again added, and the mixture was stirred at room temperature for 12 hours. The reaction mixture was concentrated, and the residue was dissolved in methanol (10ml), followed by addition of potassium carbonate (0.6g, 4.34mmol). The mixture was stirred at room temperature for 1 hour. The reaction mixture was concentrated, and the residue was neutralized with 1N hydrochloric acid, followed by extraction with ethyl acetate. The extract was washed with water, dried and concentrated by evaporating the solvent. The residue was purified by silica gel column chromatography (hexane : ethyl acetate = 1:1) to produce 4-[4-cyano-5-(ethylsulfonylamino)-2-fluorophenyl]-5-cyano-6-methoxypyrimidine (0.12g).
   ¹H-NMR(CDCl₃) δ 1.47(3H, t), 3.27(2H, q, J=7.4Hz), 4.22(3H, s), 7.00(1H, br s), 7.53(1H, d, J=8.5Hz), 8.01(1H, d, J=5.9Hz), 9.00(1H, s)
   IR(nujol) 3268, 2200 cm⁻¹
   m.p. :187-189°C

### EXAMPLE 40

### 5-Chloro-4-(4-chloro-2-fluoro-5-propargylthiophenyl)-6-difluoromethylpyrimidine (Compound No. D-48)

(1) Chlorosulfonic acid 10.6 ml was dropwise added to the compound prepared in Example 14(1) under ice-cooling, and the mixture was stirred at 80°C for one hour and at 110°C for 8 hours. After cooling the reaction mixture was portionwise poured onto ice and extracted with ethyl acetate. The extract was washed with brine, dried and concentrated. The residue was dissolved in hexane : ethyl acetate (3:1), and the solution was filtered through a silica gel layer. The filtrate was concentrated to give 2-chloro-5-(5-chloro-6-difluoromethyl-pyrimidin-4-yl)-4-fluorophenylsulfonyl chloride (4.2 g).
   ¹H-NMR(CDCl₃) δ 6.91 (1H, t, J=53.1Hz), 7.57(1H, d, J=8.8Hz), 8.38 (1H, d, J=6.8Hz), 9.31 (1H, s).
(2) To a solution of stannous chloride dehydrate (1.15 g (5.1 mmol) in concentrated hydrochloric acid (1.5 ml) was dropwise added a solution of the phenylsulfonyl chloride 3.2 g (10.9 mmol) prepared in the Example 40(1) in acetic acid (7 ml) below 25°C. The mixture was stirred at 50°C for 30 minutes and at 75°C for 30 minutes. After cooling, the reaction mixture was added to water (40 ml) containing concentrated hydrochloric acid (2 ml), and was then extracted with ethyl acetate. The extract was washed with brine, dried and concentrated to give 2-chloro-5-(5-chloro-6-difluoromethylpyrimidin-4-yl)-4-fluorothiophenol (0.6 g).
   ¹H-NMR(CDCl₃) δ 3.87 (1H, br, s), 6.92 (1H, t, J=53.2Hz), 7.32 (1H, d, J=8.8Hz), 7.51 (1H, d, J=6.9Hz), 9.27 (1H, s).
(3) The thiophenol 0.4 g (1.2 mmol) prepared in the Example 40(2) and potassium carbonate 0.25 g (1.8 mmol) were suspended in acetonitrile (10 ml). After addition of propargyl bromide 0.19 g (1.6 mmol) under ice-cooling, the mixture was stirred at room temperature for 4 hours.
The reaction mixture was concentrated, and water was added thereto. The mixture was then extracted with chloroform, and the extract was washed, with water, dried, then concentrated by evaporation of the solvent. The residue was purified by silica gel column chromatography using chloroform to give 5-chloro-4-(4-chloro-2-fluoro-5-propargylthiophenyl)-6-difluoromethylpyrimidine (0.095 g).
¹H-NMR(CDCl₃) δ 2.23 (1H, t, J=2.6Hz), 3.67 (2H, d, J=2.6Hz), 6.92.(1H, t, J=53.2Hz), 7.36 (1H, d, J=9.1Hz),
7.66 (1H, d, J=7.1Hz), 9.27 (1H, s).
m.p. 95-97°C

### Example 41

### 2-Chloro-5-(5-chloro-6-difluoromethylpyrimidin-4-yl)-4- fluoro-N-isopropylbenzenesulfonamide (Compound No. D-50)

(1) Isopropylamine hydrochloride 0.54 g (0.56 mmol) was dissolved in acetonitrile (10 ml), and triethylamine 0.12 g (1.2 mmol) was added thereto under ice-cooling. After stirring for 30 minutes, an acetonitrile solution (10 ml) of the phenylsulfonyl chloride 0.2 g (0.51 mmol) prepared in Example 40(1) was dropwise added to the mixture. The resulting mixture was stirred at room temperature for 2 hours, and is then concentrated. The crystals precipitated upon addition of water were harvested by filtration, washed with water, and dried to give 2-chloro-5-(5-chloro-6-difluoromethylpyrimidin-4-yl)-4-fluoro-N-isopropylbenzenesulfonamide (0.16 g).
   ¹H-NMR(CDCl₃) δ 1.15 (6H, d, J=6.5Hz), 3.47-3.58 (1H, m), 4.81 (1H, d, J=7.9Hz), 6.91 (1H, t, J=53.2Hz), 7.45 (1H, d, J=8.8Hz), 8.31 (1H, d, J=7.2Hz), 9.29 (1H, s).
   m.p. 172-175°c

### Example 42

### 5-Chloro-6-difluoromethyl-4-(7-chloro-5-fluoro-2-methyl- benzofuran-4-yl)pyrimidine (Compound No. D-47)

(1) The phenol 0.5 g (1.83 mmol) prepared in Example 10(2) and potassium carbonate 0.36 g (2.61 mmol) were added to DMF (10 ml). After addition of propargyl bromide 0.27 g (2.27 mmol) under ice-cooling, the resulting mixture was stirred at room temperature for 2 hours, and poured into ice water. The precipitated crystals were collected by filtration, washed with water and dried to give 5-chloro-6-difluoromethyl-4-(4-chloro-2-fluoro-5-propargyloxyphenyl)pyrimidine (0.51 g).
   ¹H-NMR(CDCl₃) δ 2.57(1H, t, J=2.3Hz), 4.81 (2H, J=2.3Hz), 6.93 (1H, t, J=53.2Hz), 7.21 (1H, d, J=6.0), 7.32 (1H, d, J=8.9Hz), 9.27 (1H, s).
   m.p. 93-95°C
(2) The compound 0.15 g (0.43 mmol) prepared in the Example 42(1) and cesium fluoride 0.39 g (2.57 mmol) were added to N,N-diethylaniline (4 ml), and the mixture was stirred at 180°C for 9 hours. After cooling the reaction mixture, it was diluted with ethyl acetate, and neutralized with concentrated hydrochloric acid. The ethyl acetate layer was washed, dried, and evaporated to remove the solvent. The residue was purified by silica gel column chromatography (hexane : ethyl acetate =4 : 1) to give 5-chloro-6-difluoromethyl-4-(7-chloro-5-fluoro-2-methylbenzofuran-4-yl)pyrimidine (0.07 g).
   ¹H-NMR(CDCl₃) δ 2.50 (3H, d, J=1.0Hz), 6.33 (1H, q, J=1.0Hz), 6.96 (1H, t, J=53.3Hz), 7.14 (1H, d, J=9.9Hz), 9.30 (1H, s)
   IR(Nujol): 1604, 1552, 1410, 1199 cm⁻¹

### Example 43

### 5-Chloro-4-(4-chloro-2-fluoro-5-propargyloxyphenyl)-6-dichloromethylpyrimidine (Compound No. E-19)

(1) The compound 2.75 g (9.6 mmol) prepared in the Example 9(1) and AIBN 0.16 g (1 mmol) were dissolved in chlorobenzene (20 ml). To the solution was dropwise added sulfuryl chloride 2.85 g (21.1 mmol), and the mixture was stirred at the same temperature and at 80°C for one hour. After further addition of sulfuryl chloride 0.4 ml, the mixture was stirred at 80°C for one hour, and then cooled. The reaction mixture was concentrated and the residue was purified by silica gel column chromatography in a solvent system of hexane and ethyl acetate (5 : 1) to give 5-chloro-4-(4-chloro-2-fluoro-5-methoxyphenyl)-6-dichloromethylpyrimidine (3.2 g).
   ¹H-NMR(CDCl₃) δ 3.93 (3H, s), 7.02 (1H, d, J=5.9Hz), 7.18 (1H, s), 7.29 (1H, d, J=9.0Hz), 9.27 (1H, s)
(2) The compound 1.5 g (4.2 mmol) prepared in the Example 43(1) was dissolved in chloroform (20 ml), and to the solution was dropwise added a solution (6 ml) of 1M boron trifluoride in dichloromethane under ice-cooling. The resulting mixture was stirred at room temperature for 4 hours, and a solution (4.5 ml) of 1M boron trifluoride in dichloromethane was further added. After stirring at room temperature for 13 hours, the reaction mixture was poured into ice-water, and then extracted with chloroform. The extract was washed with water, dried, and evaporated to remove the solvent. The residue was purified by silica gel column chromatography in a solvent system of hexane and ethyl acetate (2:1) to afford 5-chloro-4-(4-chloro-2-fluoro-5-hydroxyphenyl)-6-dichloromethylpyrimnidine (1.1 g).
   ¹H-NMR(CDCl₃) δ 5.71 (1H, s), 7.15 (1H, d, J=6.2Hz), 7.17 (1H, s), 7.25 (1H, d, J=9.6Hz), 9.26 (1H, s)
   m.p. 111-113°C
(3) The phenol 0.139 g (0.38 mmol) prepared in the Example 43(2) and potassium carbonate 0.076 g (0.55 mmol) were suspended in DMF (2 ml). To the suspension was added propargyl bromide 0.06 g (0.5 mmol) under ice-cooling. The mixture was stirred at room temperature for 4 hours and, after addition of brine, extracted with ethyl acetate. The extract was washed with brine, dried and evaporated to remove the solvent. The residue was purified by silica gel column chromatography in a solvent system of hexcane and ethyl acetate (3:1), thereby to give 5-chloro-4-(4-chloro-2-fluoro-5-propargyloxyphenyl)-6-dichloromethylpyrimidine (0.115 g).
   m.p. 86-88°C
   ¹H-NMR(CDCl₃) δ 2.58 (1H, t, J=2.4Hz), 4.807 (2H, d, J=2.4Hz), 7.18 (1H, s), 7.21 (1H, d, J=6.0Hz), 7.31 (1H, d, J=8.9Hz), 9.28 (1H, s)

### Example 44

### 5-Chloro-6-cyano-4-[4-cyano-5-(N-ethylsulfonylamino) -2- fluorophenyl]pyrimidine (Compound No. E-22)

(1) To a solution of 15% aqueous methylmercaptane sodium salt 1.6 g (3.3 mmol) in THF (10 ml) was dropwise added a solution of the compound 0.5 g (1.3 mmol) prepared in the Example 28, in THF (3 ml) at room temperature. The mixture was stirred at room temperature for 3 hours, and then concentrated. After addition of 1N HCl (30 ml), the mixture was extracted with chloroform, and the extract was washed, dried, then evaporated. The crystals precipitated upon addition of isopropyl ether were harvested by filtration and dried to give 5-chloro-4-[4-cyano-5-(N-ethylsulfonylamino)-2-fluorophenyl]-6-methylthioipyrimidine (0.48 g).
   m.p. 167-168°C
   ¹H-NMR(CDCl₃) δ 1.46 (3H, t, J=7.4Hz), 2.65 (1H, s), 3.22 (2H, q, J=7.4Hz), 7.46 (1H, d, J=8.2Hz), 7.81 (1H, d, J=5.9Hz), 8.94 (1H, s)
(2) The compound 0.84 g (2.2 mmol) prepared in the Example 44(1) was dissolved in chloroform (15 ml), and to this solution was dropwise added a solution of m-CPBA 1.6 g (6.5 mmol) in chloroform at room temperature. The mixture was stirred at the same temperature for 16.5 hours, and then concentrated. The residue was washed several times with diethyl ether and dried to give 5-chloro-4-[4-cyano-5-(N-ethylsulfonylamino)-2-fluorophenyl]-6-methylsulfonylpyrimidine (0.77 g).
   m.p. 182-183°C
   ¹H-NMR(CDCl₃) δ 1.48 (3H, t, J=7.4Hz), 3.25 (2H, q, J=7.4Hz), 3.50 (1H, s), 7.52 (1H, d, J=8.2Hz), 7.85 (1H, d, J=5.9Hz), 9.25 (1H, s)
   IR (Nujol): 3144, 2233, 1504, 1433, 1332, 1313, 1293, 1142 cm⁻¹
(3) The compound 0.15 g (0.36 mmol) prepared in the Example 44(2) was dissolved in THF (5 ml), and to the solution were added potassium cyanide 0.07 g (1.1 mmol) and 18-crown-6 ether (catalytic amount) at room temperature. The mixture was stirred at the same temperature for 5 hours and then concentrated. After addition of chloroform to the residue, the insolubles were removed by filtration and the filtrate was concentrated. The residue was purified by silica gel column chromatography (chloroform : acetone = 10: 1), and the eluate was crystallized from isopropyl ether.
The resulting crystals were harvested by filtration and dried to afford 5-chloro-6-cyano-4-[4-cyano-5-(N-ethylsulfonylamino)-2-fluorophenyl]pyrimidine (0.04 g).
m.p. 164-165°C
¹H-NMR(CDCl₃) δ 1.47 (3H, t, J=7.4Hz), 3.26 (2H, q, J=7.4Hz), 7.10 (1H, br, s), 7.52 (1H, d, J=8.2Hz), 7.92 (1H, d, J=5.9Hz), 9.31 (1H, s)
¹⁹F-NMR (CDCl₃) δ -113.9
IR (Nujol): 3132, 2234, 1548, 1502, 1334, 1148, 930, 900, 866, 723, 503 cm⁻¹

### Example 45

### 2-Chloro-5-(5-chloro-6-difluoromethylpyrimidin-4-yl) -4- fluoro-N-propionylbenzenesulfonamide (Compound No. D-53)

(1) The phenylsulfonyl chloride 0.3 g (0.77 mmol) prepared in the Example 40(1) was dissolved in acetonitrile (5 ml), and to this solution was dropwise added 28% aqueous ammonium hydroxide (1.5 ml) under ice-cooling. The mixture was stirred at room temperature for one hour, and then concentrated. The crystals precipitated upon addition of water were collected by filtration and dried to give 2-chloro-5-(5-chloro-6-difluoro-methylpyrimidin-4-yl)-4-fluorobenzenesulfonamide (0.24 g).
   m.p. 174-176°C
   ¹H-NMR(DMSO-d⁶) δ 7.33 (1H, t, J=52.5Hz), 7.82 (1H, s), 7.93-8.02 (1H, m), 8.24 (1H, d, J=7.4Hz), 9.42 (1H, s)
(2) The benzenesulfonamide 0.15 g (0.4 mmol) prepared in the Example 45(2) and triethylamine 0.05 g (0.5 mmol) were dissolved in acetonitrile (5 ml). After addition of propionyl chloride 0.044 g (0.48 mmol) under ice-cooling, the mixture was stirred at room temperature for 5 hours, and triethylamine (0.02 ml) and propionyl chloride (0.035 ml) were further added thereto. The mixture was stirred at the same temperature for 12 hours, and then concentrated. Water was added to the residue, and the mixture was extracted with ethyl acetate. The extract was washed with brine, dried and evaporated to remove the solvent. The residue was crystallized from isopropyl ether and the resulting crystals were harvested by filtration and dried to give 2-chloro-5-(5-chloro-6-difluoromethylpyrimidin-4-yl)-4-fluoro-N-propionylbenzenesulfonamide (0.125 g).
   m.p. not higher than 203°C
   ¹H-NMR(CDCl₃) δ 1.10-1.15 (3H, t), 2.35 (2H, q, J=7.5Hz), 6.91 (1H, t, J=53.2Hz), 7.41 (1H, d, J=8.8Hz), 8.48 (1H, d, J=7.1Hz), 9.29 (1H, s).

### Example 46

### 4-(4-Chloro-2-fluoro-5-methoxyphenyl)-5-formyl-6-methoxypyrimidine (Compound No. L-1)

(1) The 4,6-dichloropyrimidine 0.2 g (1.1 mmol) prepared in the Reference Example 16(1) was dissolved in methanol (10 ml). Sodium hydrogen carbonate 0.1 g (1.2 mmol) was dropwise added to the above solution, and the mixture was stirred at room temperature for 72 hours. The reaction mixture was concentrated, and, after addition of water to the residue, extracted with ethyl acetate. The extract was dried and evaporated to remove the solvent to give a residue, to which was added pentane. The precipitated crystals were harvested by filtration and dried to afford 4-chloro-5-formyl-6-methoxypyrimidine (0.12 g).
   ¹H-NMR(CDCl₃) δ 4.16 (3H, s), 8.67 (1H, s), 10.4 (1H, s)
(2) The compound 0.2 g (1.16 mmol) prepared in Example 46(1), sodium hydrogen carbonate 0.3 g (3.6 mmol), and dichlorobis(triphenylphosphine)palladium(II) 0.03 g (0.04 mmol) were added to a mixture of 1,2-dimethoxyethane (10 ml) and water (2 ml). The mixture was heated to 70°C, and 4-chloro-2-fluoro-5-methoxyphenylboronic acid 0.26 g (1.27 mmol) was portionwise added. The mixture was stirred at the same temperature for 2 hours, cooled, and, after addition of water, extracted with ethyl acetate.
The extract was dried and evaporated to remove the solvent. The residue was purified by silica gel column chromatography in a solvent system of hexane and ethyl acetate (2:1) to give 4-(4-chloro-2-fluoro-5-methoxyphenyl)-5-formyl-6-methoxypyrimidine (0.21 g).
m.p. 161-163°C
¹H-NMR(CDCl₃) δ 3.94 (3H, s), 4.18 (3H, s), 7.12 (1H, d, J=6.1Hz), 7.20 (1H, d, J=9.2Hz), 8.94 (1H, s), 10.30 (1H, s)
IR(Nujol):1696 cm⁻¹

### Example 47

### 4-(2,4-Dichloro-5-methylsulfonylaminophenyl)-5-cyano-6-methoxypyrimidine (Compound No. K-20)

(1) The 4-chloropyrimidine 0.98 g (5.7 mmol) prepared in Reference Example 16, 2,4-dichlorophenylboronic acid 1.0 g (5.2 mmol), dichlorobis(triphenylphosphine)palladium(II) 0.22 g (0.32 mmol) and sodium hydrogen carbonate 1.4 g (17 mmol) were added to a mixture of 1,2-dimethoxyethane (40 ml) and water (8 ml). The mixture was heated under reflux for two hours, and then cooled. The reaction mixture was concentrated and water was added to the residue. The mixture was adjusted to pH 3 with 2N-HCl and extracted with chloroform. The extract was washed, dried and evaporated to remove the solvent. The residue was purified by silica gel column chromatography in a solvent system of hexane and ethyl acetate (2 : 1) to afford 4-(2,4-dichlorophenyl)-5-cyano-6-methoxypyrimidine (0.65 g).
   m.p. 107-109°C
   ¹H-NMR(CDCl₃) δ 4.20 (3H, s), 7.40 (1H, d), 7.35-7.45 (1H, d), 8.97 (1H, s)
(2) The compound 0.65 g (2.3 mmol) prepared in the Example 47(1) was added to concentrated sulfuric acid 5 ml under ice-cooling, and fuming nitric acid 0.096 ml was added thereto. The mixture was stirred at room temperature for one hour, and fuming nitric acid 0.03 ml was further added. The mixture was stirred at room temperature for two hours, and poured onto ice. The precipitated crystals were collected by filtration and washed with water. The resulting crystals were dissolved in chloroform, and the solution was washed with water, dried, then evaporated to remove the solvent. The residue was crystallized from a mixture of hexane and ethyl acetate (5 : 1) and the crystals were harvested by filtration and dried to afford 4-(2,4-dichloro-5-nitrophenyl)-5-cyano-6-methoxypyrimidine (0.6 g).
   ¹H-NMR(CDCl₃) δ 4.22 (3H, s), 7.79 (1H, s), 8.07 (1H, s), 9.00 (1H, s)
(3) The compound 0.6 g (1.8 mmol) prepared in the Example 47(2) was added to acetic acid 4 ml and water 1 ml, and reduced iron 0.3 g (5.5 mmol) was portionwise added thereto at 40-50°C. The mixture was stirred at the same temperature for 1.5 hours, and ethyl acetate (10 ml) was then added. After removal of the insolubles by filtration through a Celite layer, the filtrate was concentrated and, after addition of water, neutralized with 2N sodium hydroxide. The neutralized solutioin was extracted with ethyl acetate, and the extract was washed with water, dried, then evaporated to remove the solvent. The residue was purified by silica gel column chromatography in the solvent system of hexane and ethyl acetate (1 : 1) to afford 4-(5-amino-2,4-dichlorophenyl)-5-cyano-6-methoxypyrimidine (0.39 g).
   ¹H-NMR(CDCl₃) δ 4.19 (3H, s), 4.21 (2H, br, s), 6.80 (1H, s), 7.43 (1H, s), 8.94 (1H, s)
(4) The compound 0.15 g (0.5 mmol) prepared in Example 47(3) and triethylamine 0.15 g (1.5 mmol) were dissolved in THF 5 ml. After addition of methanesulfonyl chloride 0.17 g (1.5 mmol) under ice-cooling, the resulting mixture was stirred at room temperature for two hours, and triethylamine 0.05 ml and methanesulfonyl chloride 0.03 ml were further added at room temperature. The mixture was stirred at room temperature for one hour, and then concentrated. After addition of brine to the residue, the mixture was extracted with ethyl acetate. The extract was washed with water, dried and evaporated to remove the solvent. The residue was crystallized from a mixture of hexane and ethyl acetate (5 : 1), and the resultant crystals were harvested by filtration and dried to afford a bis-form product, yield 0.21 g.
   ¹H-NMR(CDCl₃) δ 3.51 (6H, s), 4.21 (3H, s),7.57 (1H, s), 7.78 (1H, s), 8.99 (1H, s)
The bis-form (0.21 g) was dissolved in dioxane (3 ml) and water (1 ml), and to the solution was added 2N sodium hydroxide 0.4 ml under ice-cooling. The mixture was stirred at room temperature for one hour, and water (5 ml) was added. The solution was neutralized with 2N sodium hydroxide under ice-cooling to precipitate crystals. The resulting crystals were harvested by filtration, washed with water and dried to afford 4-(2,4-dichloro-5-methylsulfonylaminophenyl)-5-cyano-6-methoxypyrimidine (0.22 g).
m.p. 160-162°C
¹H-NMR(CDCl₃) δ 3.10 (3H, s), 4.12 (3H, s), 6.92 (1H, s), 7.64 (1H, s), 7.79 (1H, s), 8.98 (1H, s).

### Example 48

### 2-chloro-5-(5-chloro-6-difluoromethyl-pyrimidin-4-yl)-4-fluorophenylacetate (Compound No. D-57)

(1) The compound 1.98 g (5.9 mmol) prepared in the Example 23(1) was suspended in toluene (9 ml), and to the suspension were added thionyl chloride 1.05 g (8.8 mmol) and DMF 0.057 g (0.77 mmol). The mixture was stirred at 70°C for 3.5 hours, and then concentrated. The residue was dissolved in acetonitrile (9 ml), and an aqueous solution (4 ml) of sodium borohydride 0.26 g (6.5 mmol) was dropwise added at -5°C to 0°C. The mixture was stirred at the same temperature for one hour, and saturated aqueous ammonium chloride (10 ml) was added. The mixture was concentrated under reduced pressure, and water was added to the residue. The mixture was extracted with chloroform and the extract was washed with brine, dried, then evaporated to remove the solvent to give a residue, which was purified by silica gel column chromatography in a solvent system of hexane and ethyl acetate (4 : 1) to afford 2-chloro-5-(5-chloro-6-difluoro-methylpyrimidin-4-yl)-4-fluorobenzyl alcohol (1.2 g).
   m.p. 107-108°C.
   ¹H-NMR(CDCl₃) δ 2.48 (1H, br, s), 4.80 (2H, s), 6.93 (1H, t, J=53.3Hz), 7.28 (1H, d, J=8.2Hz), 7.69 (1H, d, J=7.5Hz), 9.26 (1H, s).
   ¹⁹F-NMR (CDCl₃) δ -112.1, -121.3
   IR (nujol) : 3250, 1614, 1556, 1224, 1052, 907, 669 cm⁻¹
(2) The benzyl alcohol 0.52 g (1.6 mmol) prepared in the Example 48(1) was dissolved in DMF (3 ml), and thionyl chloride 0.2 g (1.7 mmol) was added thereto under ice-cooling. The mixture was stirred at the same temperature for 30 minutes and at room temperature for one hour. After addition of water, the reaction mixture was neutralized with sodium hydrogen carbonate, and extracted with ethyl acetate. The extract was washed with brine, dried and evaporated to give 2-chloro-5-(5-chloro-6- difluoromethylpyrimidin-4-yl)-4-fluorobenzyl chloride (0.55 g).
   m.p. 132-133°C
   ¹H-NMR(CDCl₃) δ 4.73 (2H, s), 6.93 (1H, t, J=53.3Hz), 7.34 (1H, d, J=9.2Hz), 7.67(1H, d, J=7.3Hz), 9.28 (1H, s)
   ¹⁹F-NMR 6 -109.5, -121.2
   IR(nujol) : 1616, 1557, 1232, 1111, 1052, 668 cm⁻¹
(3) The benzyl chloride 0.55 g (1.6 mmol) prepared in Example 48(2) was dissolved in DMF (4 ml), and to the solution were added 18-crown-6 20 mg (0.076 mmol) and potassium cyanaide 0.104 g (1.6 mmol). The mixture was stirred at 50°C for two hours and further at room temperature for 14 hours. After addition of water (60ml), the reaction mixture was extracted with ethyl acetate. The extract was washed with brine, dried and evaporated to remove the solvent. The residue was purified by silica gel column chromatography in a solvent system of hexane and ethyl acetate (3:1) to afford 2-chloro-5-(5-chloro-6-difluoro-methylpyrimidin-4-yl)-4-fluorophenylacetonitrile (0.47 g).
   m.p. 92-93°C
   ¹H-NMR(CDCl₃) δ 3.89 (2H, s), 6.93 (1H, t, J=53.3Hz), 7.38 (1H, d, J=9.2Hz), 7.69 (1H, d, J=7.3Hz), 9.28 (1H, s) ¹⁹F-NMR (CDCl₃) δ -109.5, -121.1
   IR(nujol) : 1619, 1555, 1232, 1109, 1052, 696, 866, 672 cm⁻¹
(4) Concentrated hydrochloric acid (4 ml) and acetic acid (1.3 ml) were added to the compound 0.27 g (0.81 mmol) prepared in Example 48(3). The mixture was stirred at 60°C for 2 hours and then cooled poured onto ice (80 g) and extracted with ethyl acetate. The extract was washed with brine, dried and evaporated to remove the solvent to give a crude product (0.19 g), which was dissolved in THF (8 ml) in a stream of nitrogen gas.
Carbonyldiimidazole 0.13 g (0.78 mmol) was added to the solution, and the mixture was stirred at room temperature for one hour. After addition of ethanol 0.16 g (3.4 mmol), the mixture was heated under reflux for 2 hours, and ethanol 0.32 g (6.8 mmol) was further added. The mixture was heated under reflux for 3.5 hours and further at 45°C for 17 hours. Then, the reaction mixture was concentrated under reduced pressure, and water was added to the residue. The mixture was extracted with ethyl acetate and the extract was washed with brine, dried and evaporated to remove the solvent. The residue was purified by silica gel column chromatography in a solvent system of hexane and ethyl acetate (3 : 1) to give ethyl 2-chloro-5- (5-chloro-6-difluoromethylpyrimidin-4-yl)-4-fluorophenyl- acetate as an oil (0.05 g).
¹H-NMR(CDCl₃) δ 1.28 (3H, t, J=7.0Hz), 3.81 (2H, s), 4.20 (2H, q, J=7.1Hz), 6.93 (1H, t, J=53.3Hz), 7.32 (1H, d, J=9.3Hz), 7.49 (1H, d, J=7.5Hz), 9.26 (1H, s)
¹⁹F-NMR (CDCl₃) δ -111.6, -121.3
IR(nujol) : 1738, 1617, 1556, 1232, 1132, 1060, 1029, 981 cm⁻¹

### Example 49

### 4-(4-Chloro-2-fluoro-5-methoxyphenyl)-5-cyano-6-difl uoro- methylpyrimidine (Compound No. K-22)

4-Chloropyrimidine 0.15 g (0.79 mmol) prepared in the Reference Example 17, 4-chloro-2-fluoro-5-methoxyphenyl-boronic acid 0.18 g (0.88 mmol), dichlorobis(triphenyl-phosphine)palladium(II) 0.08 g (0.11 mmol) and sodium hydrogen carbonate 0.135 g (1.61 mmol) were added to a mixture of 1,2-dimethoxyethane 7 ml and water (three drops). The mixture was stirred at 55°C for 16 hours, and then cooled. The reaction mixture was adjusted to pH 3 with dilute hydrochloric acid and extracted with ethyl acetate. The extract was washed with water, dried and evaporated to remove the solvent. The residue was purified by silica gel column chromatography in a solvent system of hexane and ethyl acetate (4 : 1) to give 4-(4-chloro-2-fluoro-5-methoxyphenyl)-5-cyano-6-difluoro-methylpyrimidine (0.025 g).
m.p. 113-115°C
¹H-NMR(CDCl₃) δ 3.97 (3H, s), 6.81 (1H, t, J=53.2Hz), 7.18 (1H, d, J=6.1Hz), 7.38 (1H, d, J=9.3Hz), 9.51 (1H, s)
IR(nujol) : 2200 cm⁻¹

### Example 50

### Methyl 2-chloro-5-(5-cyano-6-methylpyrimidin-4-yl)-4-fluorobenzoate (Compound No. K-19)

The 4-chloropyrimidine 0.2 g (0.13 mmol) prepared in the Reference Example 18, 4-chloro-2-fluoro-5-methoxycarbonyl-phenylboronic acid 0.34 g (0.88 mmol), dichlorobis(triphenylphosphine)palladium(II) 0.06 g (0.09 mmol) and sodium hydrogen carbonate 0.6 g (7 mmol) were added to a mixture of 1,2-dimethoxyethane 10 ml and water 2 ml. The mixture was stirred at 70°C for 2 hours, and then cooled. The reaction mixture was adjusted to pH 3 with dilute hydrochloric acid, and extracted with ethyl acetate. The extract was washed with water, dried and evaporated to remove the solvent. The residue was purified by silica gel column chromatography in the solvent system of hexane and ethyl acetate (2:1 to 1:1) to afford methyl 2-chloro-5-(5-cyano-6-methylpyrimidin-4-yl)-4-fluoro-benzoate (0.11 g).
m.p. 142-144°C
¹H-NMR(CDCl₃) δ 2.87 (3H, s), 3.95 (3H, s), 7.43 (1H, d, J=9.6Hz), 8.25 (1H, d, J=7.5Hz), 9.31 (1H, s)
IR (nujol): 2200, 1741 cm⁻¹

The compounds prepared in the same manner as Examples 1 to 50 are shown in Tables 1 to 15 together with the compounds of Examples 1 to 50.

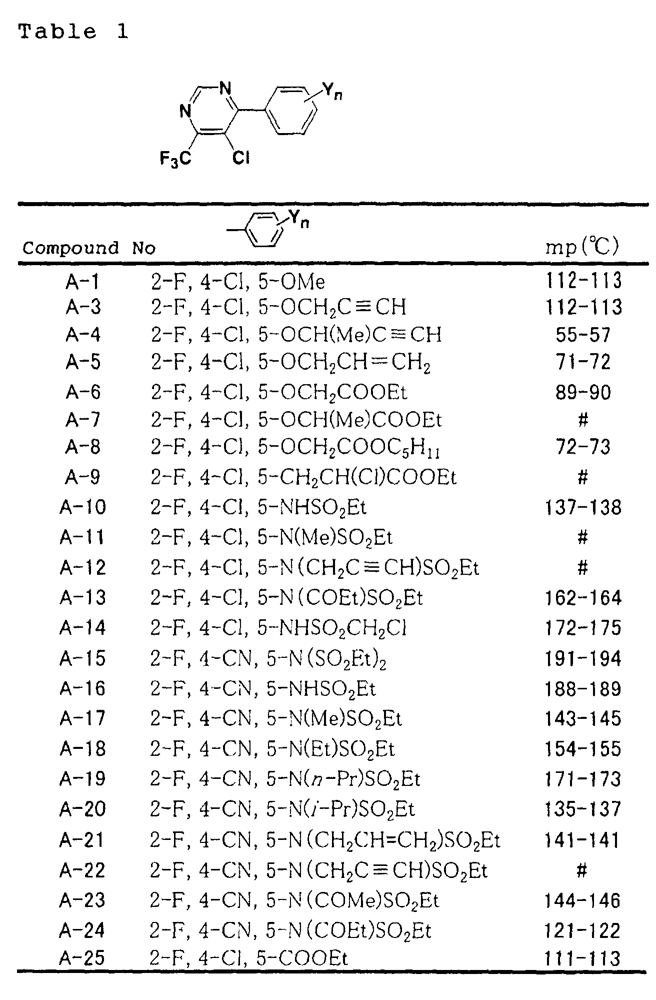

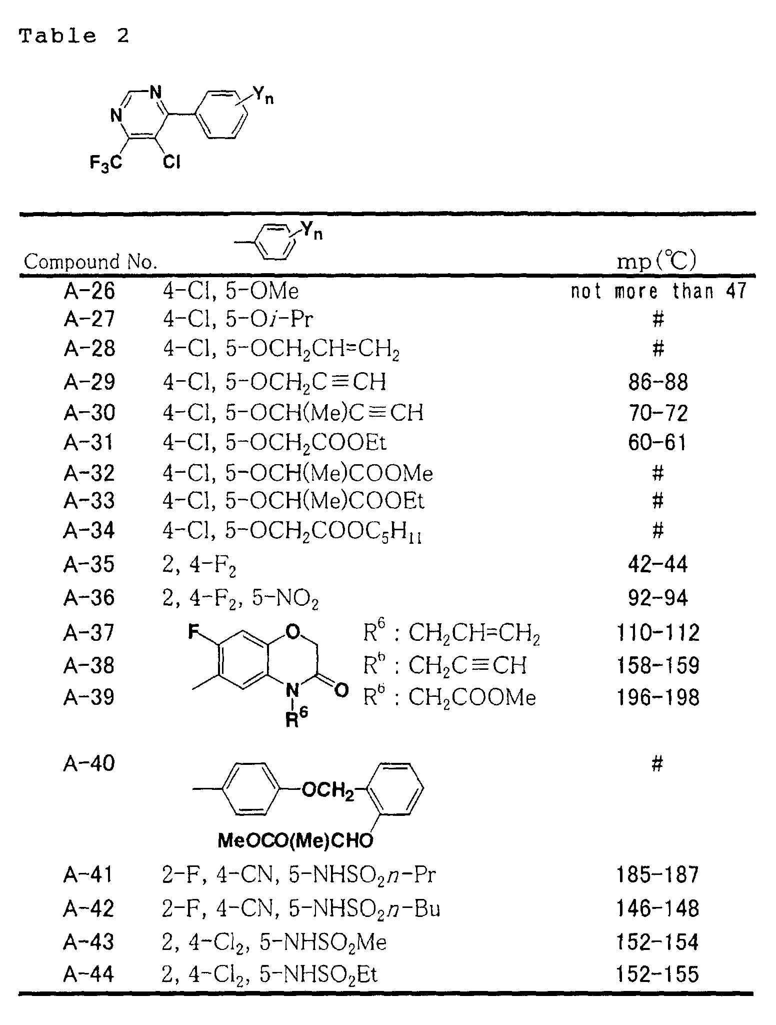

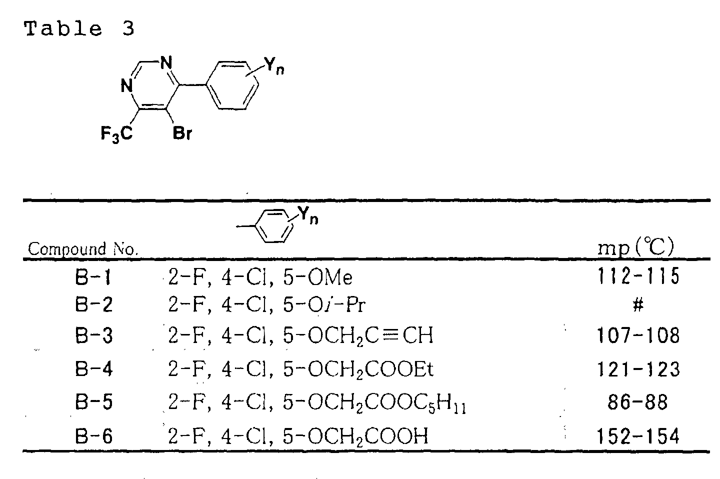

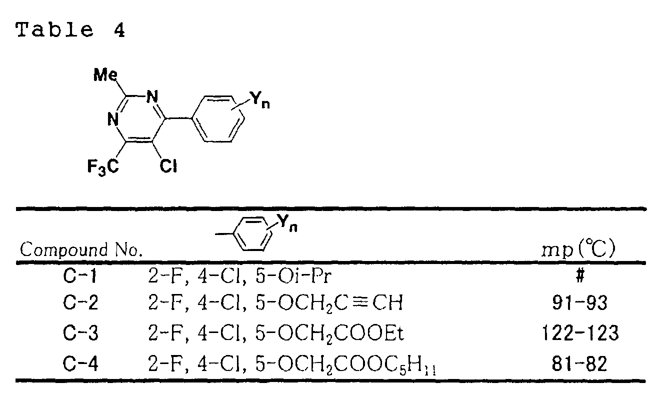

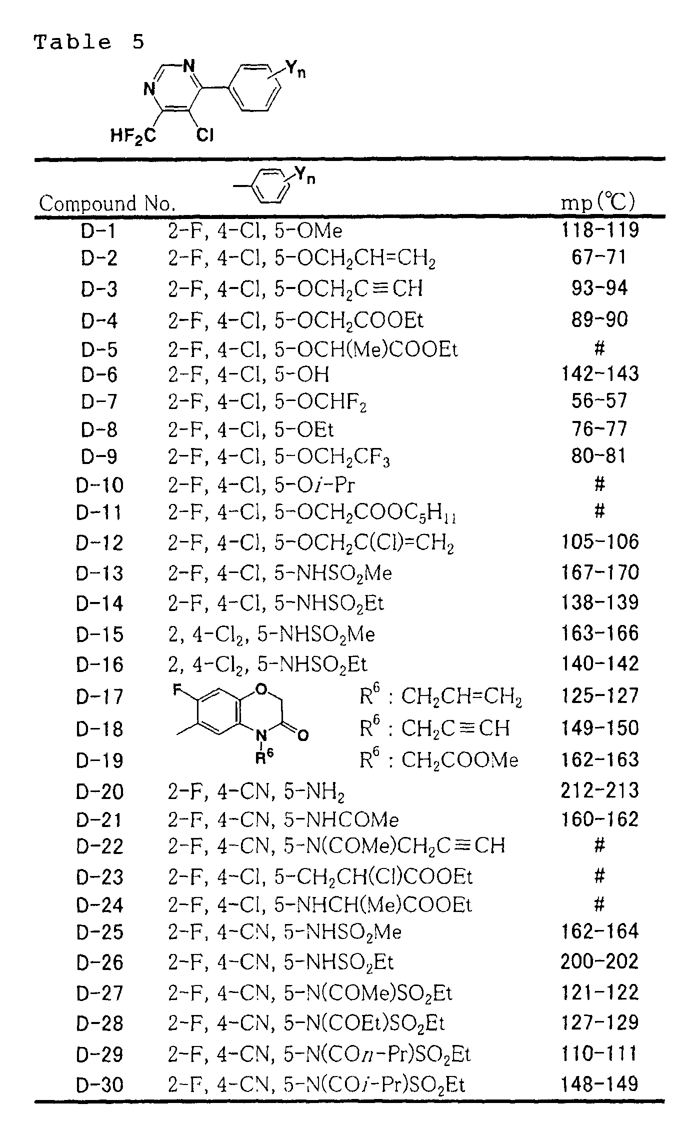

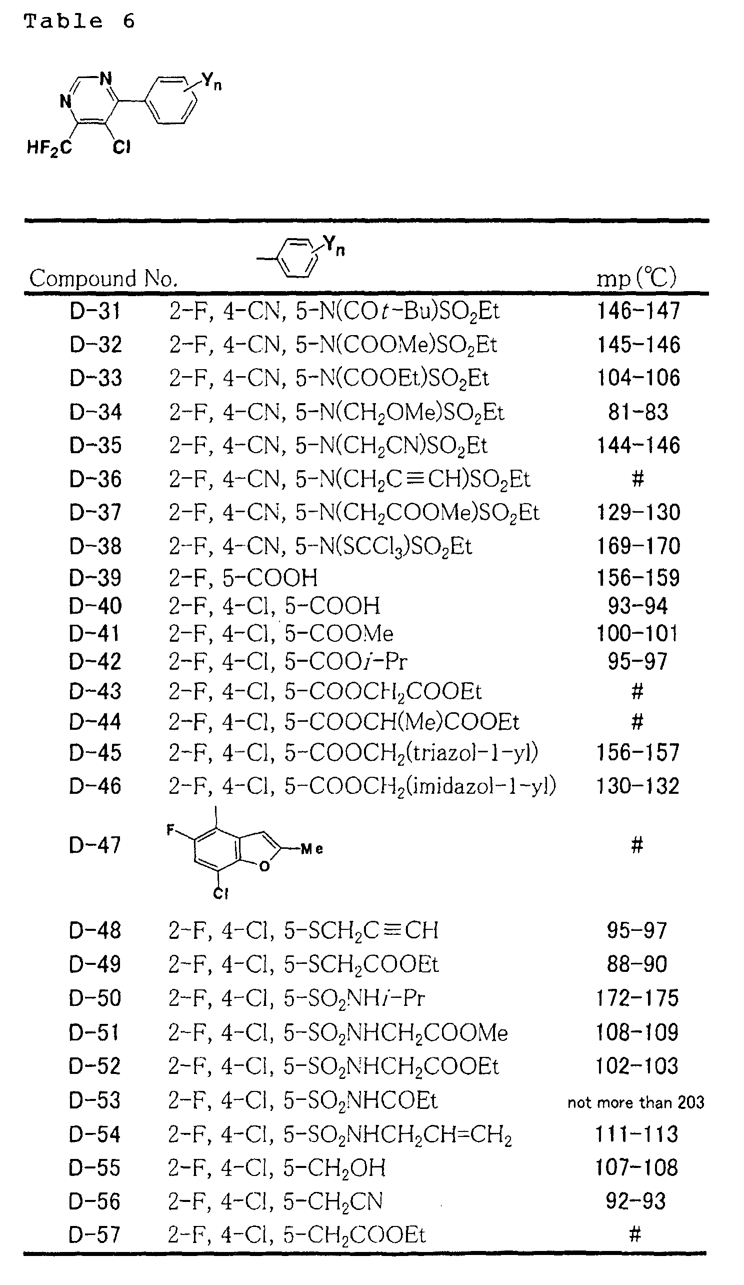

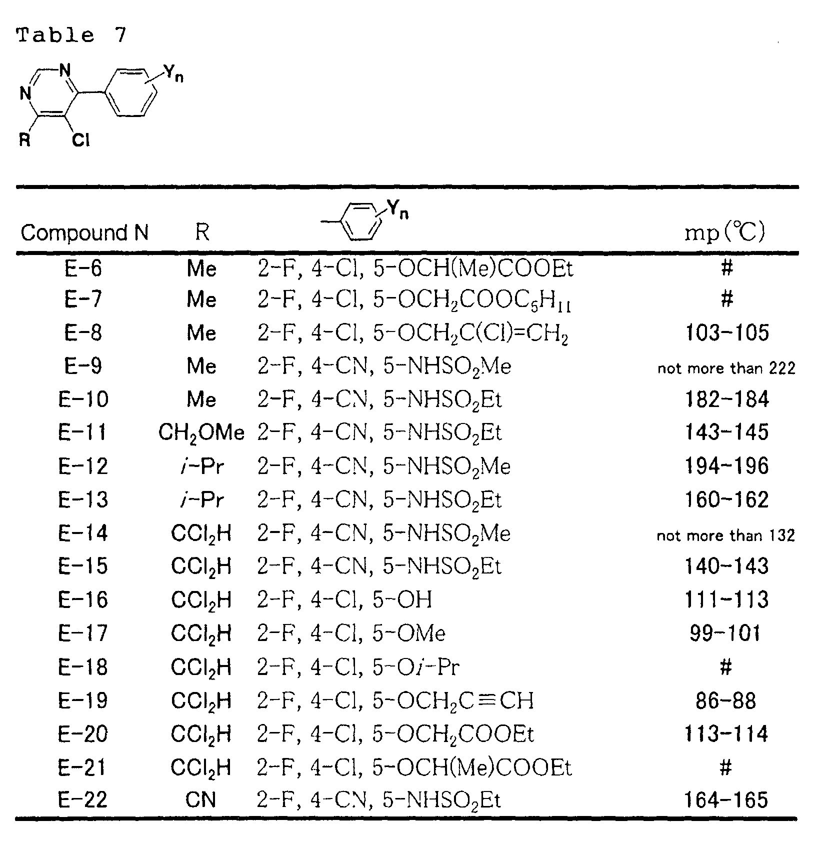

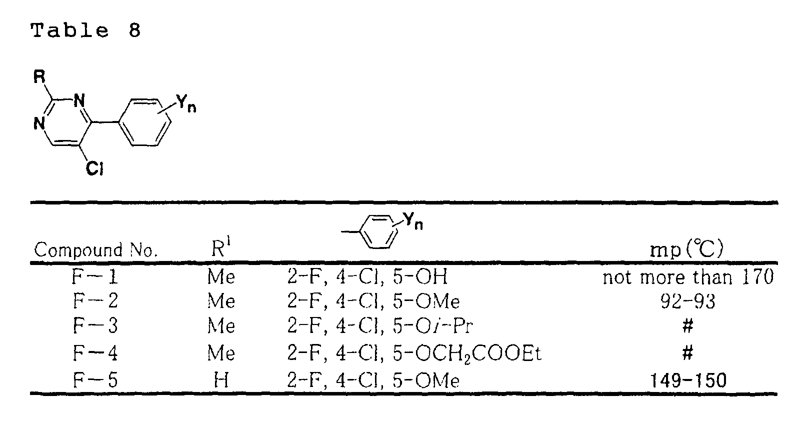

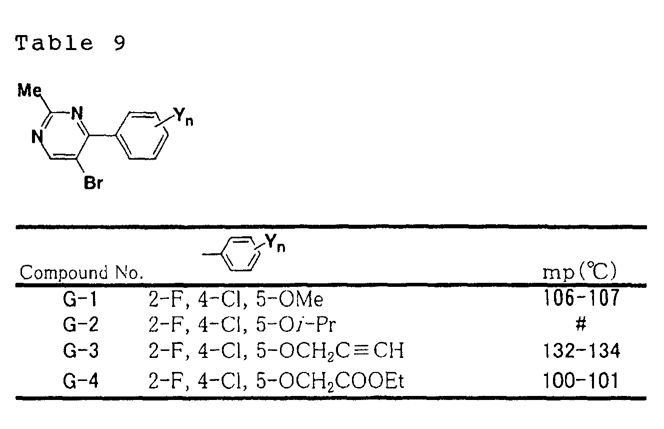

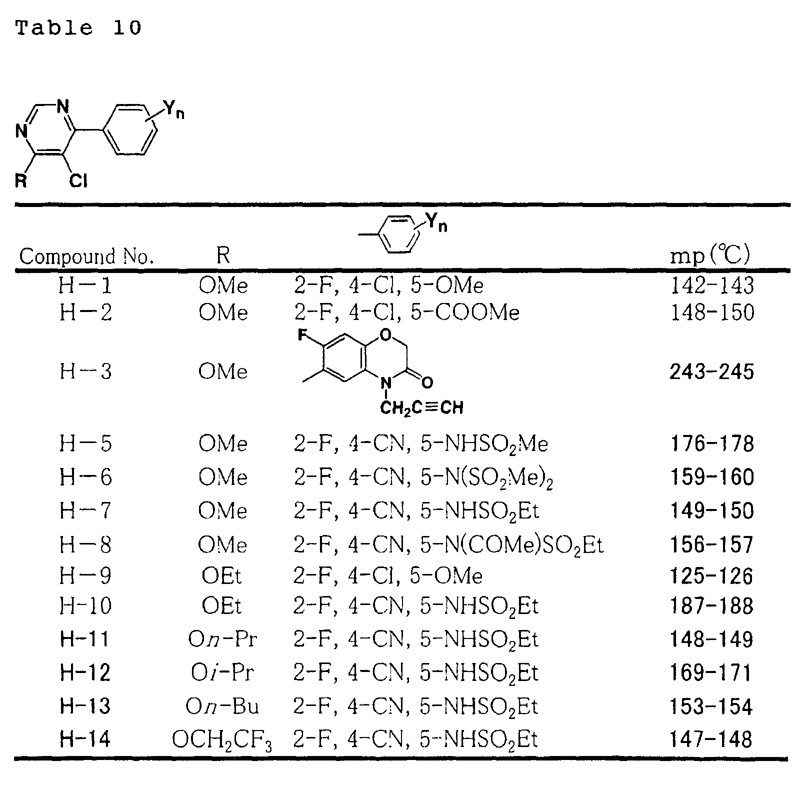

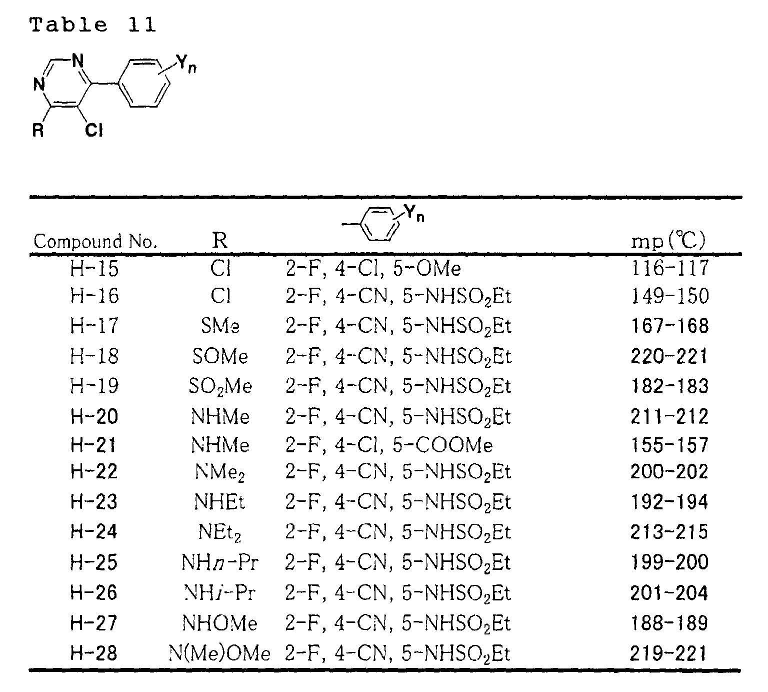

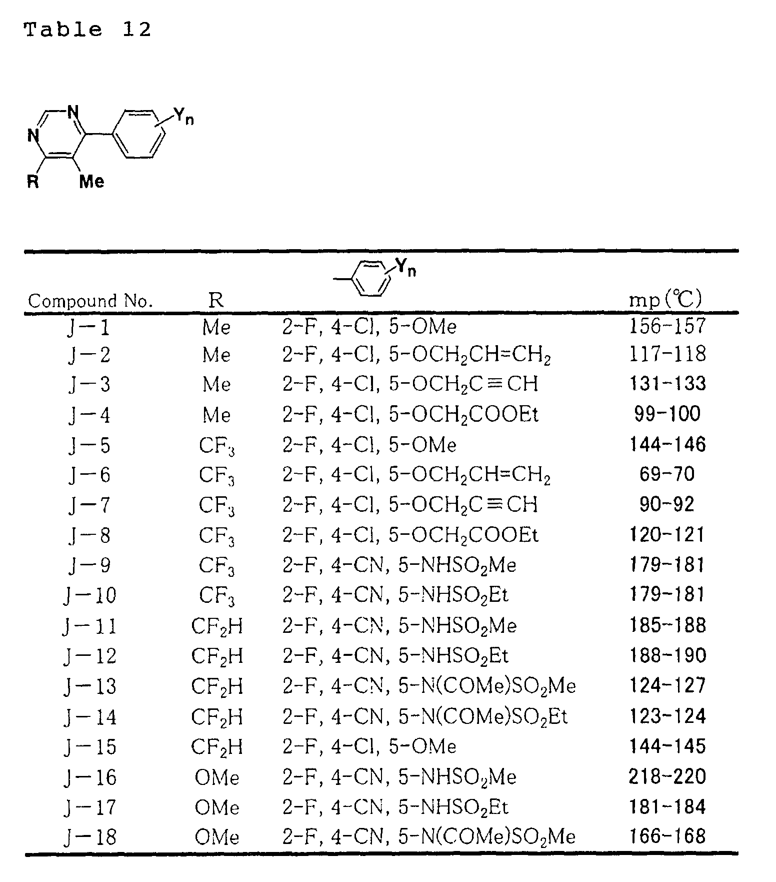

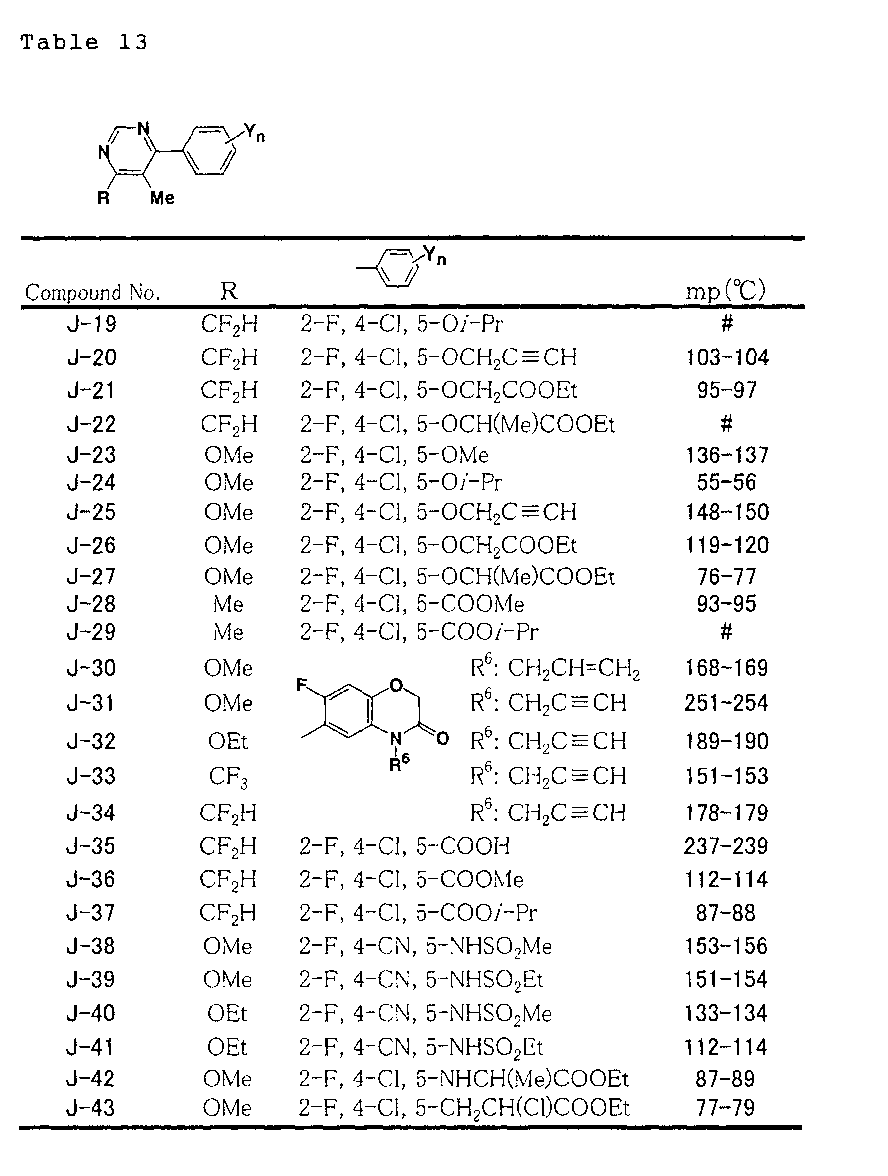

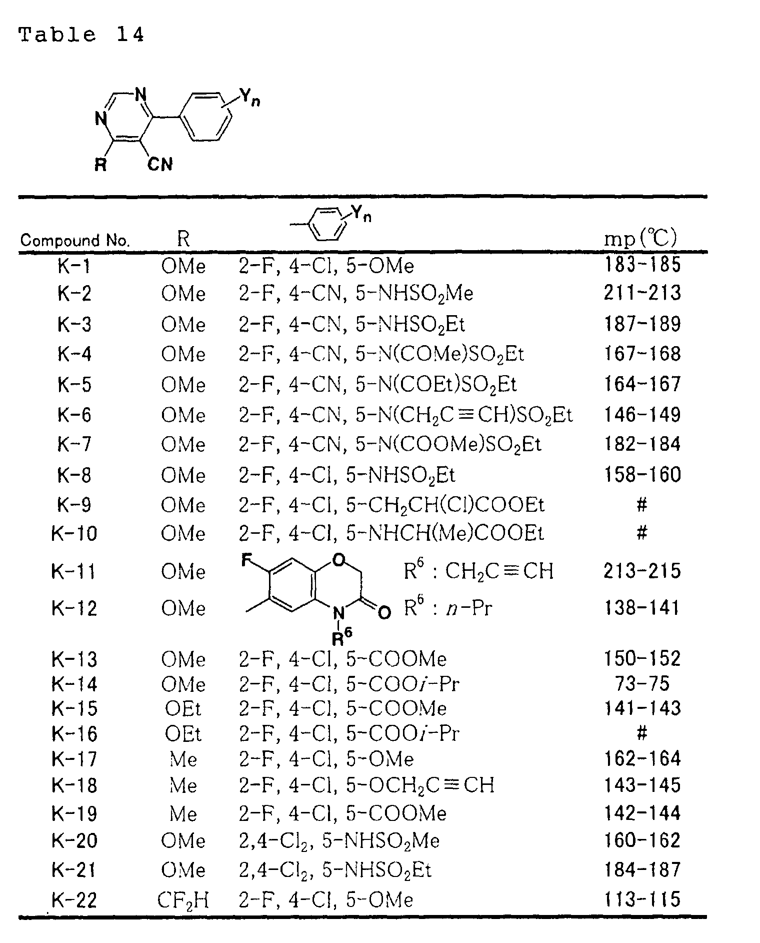

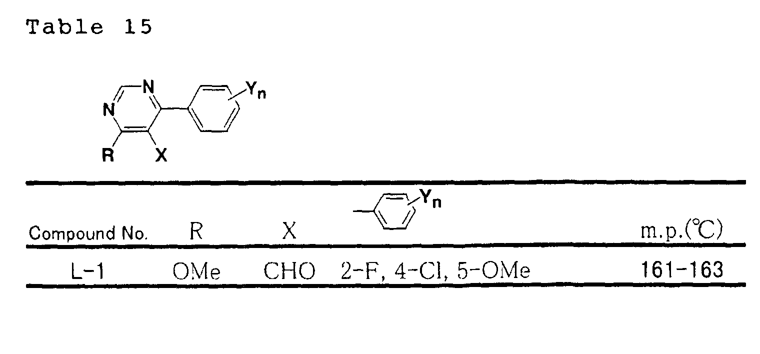

The compounds with the symbol(#) in the column of m.p.(°C) among the compounds which are shown in the above-mentioned Tables 1 to 15 are oily substances, resin-like substances or waxy solids. ¹H-NMR spectrum data for these compounds are shown below.

### Compound No. A-2

(CDCl₃) δ 1.40(6H, d, J=6.1Hz), 4.54(1H, septet, J=6.1Hz), 7.04(1H, d, J=6.2Hz), 7.29(1H, d, J=9.1Hz), 9.27(1H, s).

### Compound No. A-7

(CDCl₃) δ 1.24(3H, t, J=7.2Hz), 1.70(3H, d, J=6.8Hz), 4.22(2H, q, J=7.2Hz), 4.75(1H, q, J=6.8Hz), 7.02(1H, d, J=6.1Hz), 7.31(1H, d, J=9.0Hz), 9.25(1H, s).

### Compound No. A-9

(CDCl₃) δ 1.27(3H, t, J=7.17Hz), 3.25-3.66(2H, m), 4.10-4.32(2H, m), 4.50-4.65(1H, m),7.32(1H, d, J=9.27Hz), 7.46(1H, d, J=7.34Hz), 9.27(1H, S).

### Compound No. A-11

(CDCl₃) δ 1.46(3H, t, J=7.37Hz), 3.19(2H, q, J=7.33Hz), 3.33(3H, s), 7.39(1H, d, J=9.03Hz), 7.72(1H, d, J=6.90Hz), 9.28(1H, s).

### Compound No. A-12

(CDCl₃) δ 1.47(3H, t, J=7.36Hz), 2.36(1H, t, J=2.5Hz), 3.23(2H, q, J=7.37Hz), 4.00-4.90(2H, br s), 7.43(1H, d, J=9.0Hz), 7.79(1H, d, J=7.0Hz), 9.29(1H, s)

### Compound No. A-22

(CDCl₃) δ 1.52(3H, t, J=7.38Hz), 2.42(1H, t, J=2.44Hz), 3.31(2H, q, J=7.40Hz), 4.54(2H, d, J=2.47Hz), 7.62(1H, d, J=8.38Hz), 7.87(1H, d, J=6.17Hz), 9.32(1H, s)

### Compound No. A-27

(CDCl₃) δ 1.43(6H, d, J=6.1Hz), 4.65(1H, septet, J=6.1Hz), 7.34-7.42(2H, m), 7.51(1H, d, J=8.3Hz), 9.23(1H, s).

### Compound No. A-28

(CDCl₃) δ 4.65-4.74(2H, m), 5.30-5.57(2H, m), 6.00-6.20(1H, m), 7.35-7.58(3H, m), 9.23(1H, s).

### Compound No. A-32

(CDCl₃) δ 1.72(3H, d, J=6.79Hz), 3.77(3H, s), 4.86(1H, q, J=6.80Hz), 7.30-7.58(3H, m), 9.22(1H, s).

### Compound No. A-33

(CDCl₃ ) δ 1.25(3H, t, J=7.07Hz), 1.72(3H, d, J=6.80Hz), 4.23(2H, q, J=7.1Hz), 4.83(1H, q), 7.30-7.60(3H, m), 9.21(1H, s).

### Compound No. A-34

(CDCl₃) δ 0.86(3H, t), 1.20-1.50(4H, m), 1.55-1.75(2H, m), 4.20(2H, t, J=6.64Hz), 4.78(2H, s), 7.30-7.60(3H, m), 9.22(1H, s).

### Compound No. A-40

(CDCl₃) δ 1.65(3H, d, J=6.8Hz), 3.75(3H, s), 4.87(1H, q, J=6.8Hz), 5.31(2H, br s), 6.78(1H, d, J=8.1Hz), 6.98-7.06(1H, m), 7.15(2H, d, J=8.9Hz), 7.22-7.30(1H, m), 7.48(1H, d, J=7.5Hz), 7.88(2H, d, J=8.9Hz), 9.17(1H, s).

### Compound No. B-2

(CDCl₃) δ 1.40(6H, d, J=6.1Hz), 4.53(1H, septet, J=6.1Hz), 6.99(1H, d, J=6.4Hz), 7.28(1H, d, J=8.8Hz), 9.29(1H, s).

### Compound No. C-1

(CDCl₃) δ 1.40(6H, d, J=6.1Hz), 2.85(3H, s), 4.53(1H, septet, J=6.1Hz), 7.00(1H, d, J=6.1Hz), 7.27(1H, d, J=9.0Hz).

### Compound No. D-5

(CDCl₃) δ 1.24(3H, d, J=7.09Hz), 1.69(3H, d, J=6.79Hz), 4.22(2H, m), 4.75(1H, q, J=6.79Hz), 6.92(1H, t, J=53.2Hz), 7.02(1H, d, J=6.06Hz), 7.31(1H, d, J=9.09Hz), 9.24(1H,s).

### Compound No. D-10

(CDCl₃) δ 1.40(6H, d, J=6.1Hz), 4.54(1H, septet, J=6.1Hz), 6.94(1H, t, J=53.3Hz), 7.04(1H, d, J=6.2Hz), 7.29(1H, d, J=9.1Hz), 9.26(1H, s)

### Compound No. D-11

(CDCl₃) δ 0.87(3H, t, J=6.6Hz), 1.25-1.32(4H, m), 1.61-1.68(2H, m), 4.20(2H, t, J=6.7Hz), 4.73(2H, s), 6.93(1H, t, J=53.3Hz), 7.01(1H, d, J=5.9Hz), 7.32(1H, d, J=8.9Hz), 9.25(1H, s)

### Compound No. D-22

(CDCl₃) δ 1.97(3H, s), 2.28(1H, t, J=2.4Hz), 4.28, 4,96(2H, dq_{AB}, J_{d}=2.1Hz, J_{AB}=17.5Hz), 6.93(1H, t, J=53.1Hz). 7.69(1H, d, J=6.1Hz), 7.69(1H, d, J=8.4Hz), 9.33(1H, s)

### Compound No. D-23

(CDCl₃) δ 1.26(3H, t, J=7.1Hz), 3.29-3.34(1H, m), 3.52-3.58(1H, m), 4.18-4.27(2H, m), 4.56-4.60(1H, m), 6.92(1H, t, J=53.3Hz), 7.32(1H, d, J=8.2Hz), 7.47(1H, d, J=8.2Hz), 9.26(1H, s)

### Compound No. D-24

(CDCl₃) δ 1.26(3H, t, J=7.1Hz), 1.55(3H, d, J=4.2Hz), 4.11-4.16(1H, m), 4.21(2H, q, J=7.1Hz), 4.82(1H, d, J=7.9Hz), 6.33(1H, d, J=6.1Hz), 6.93(1H, t, J=53.3Hz), 7.22(1H, d, J=6.1Hz), 7.22(1H, d, J=6.1Hz), 9.24(1H, s)

### Compound No. D-36

(CDCl₃) δ 1.52(3H, t, J=7.4Hz), 2.43(1H, t, J=2.2Hz), 3.31(2H, q, J=7.3Hz), 4.53(2H, d, J=2.3Hz), 6.92(1H, t, J=53.3Hz), 7.62(1H, d, J=8.4Hz), 7.87(1H, d, J=6.1Hz), 9.31(1H, s)

### Compound No. D-43

(CDCl₃) δ 1.30(3H, t, J=7.1Hz), 4.26(2H, q, J=7.1Hz), 4.86(2H, s), 6.92(1H, t, J=53.2Hz), 7.42(1H, d, J=9.4Hz), 8.24(1H, d, J=7.4Hz), 9.29(1H, s)

### Compound No. D-44

(CDCl₃) δ 1.29(3H, t, J=7.1Hz), 1.62(3H, d, J=7.0Hz), 4.24(2H, q, J=7.1Hz), 5.34(1H, q, J=7.1Hz), 6.93(1H, t, J=53.2Hz), 7.42(1H, d, J=9.3Hz), 8.19(1H, d, J=7.5Hz), 9.29(1H, s)

### Compound No. D-47

(CDCl₃) δ 2.50(3H, d, J=1.0Hz), 6.33(1H, q, J=1.0Hz), 6.96(1H, t, J=53.3Hz), 7.14(1H, d, J=9.9Hz), 9.30(1H, s)

### Compound No. D-57

¹H-NMR(CDCl₃) δ 1.28(3H, t, J=7.0Hz), 3.81(2H, s), 4.20(2H, q, J=7.1Hz), 6.93(1H, t, J=53.3Hz), 7.32(1H, d, J=9.3Hz), 7.49(1H, d, J=7.5Hz), 9.26(1H, s)

### Compound No. E-6

(CDCl₃) δ 1.23(3H, t, J=7.12Hz), 1.68(3H, d, J=6.79Hz), 2.71(3H, s),4.21(2H, q, J=7.41Hz),4.74(1H, q, J=6.77Hz), 7.00(1H, d, J=6.08Hz), 7.27(1H, d, J=8.93Hz), 8.99(1H, s).

### Compound No. E-7

(CDCl₃) δ 0.86(3H, t), 1.18-1.40(4H, m), 1.55-1.75(2H, m), 2.71(3H, s), 4.18(2H, t, J=6.64Hz), 4.71(2H, s), 6.99(1H, d, J=5.96Hz), 7.28(1H, d), 8.99(1H, s).

### Compound No. E-18

(CDCl₃) δ 1.39(6H, d, J=6.0Hz), 4.53(1H, septet), 7.05(1H, d, J=6.2Hz), 7.18(1H, s), 7.25-7.30(1H, m), 9.26(1H, s)

### Compound No. E-21

(CDCl₃) δ 1.24(3H, t, J=7.1Hz), 1.69(3H, d, J=6.8Hz), 4.15-4.28(2H, m), 4.75(2H, s), 7.03(1H, d, J=6.1Hz), 7.17(1H, s), 7.31(1H, d, J=9.0Hz), 9.24(1H, s)

### Compound No. F-3

(CDCl₃) δ 1.39(6H, d, J=6.07Hz), 2.77(3H, s), 4.53(1H, septet, J=6.06Hz), 7.02(1H, d, J=6.27Hz), 7.25(1H, d, J=9.03Hz), 8.69(1H, s).

### Compound No. F-4

(CDCl₃) δ 1.28(3H, t, J=7.13Hz), 2.76(3H, s), 4.27(2H, q, J=7.12Hz), 4.71(2H, s), 6.99(1H, d, J=6.04Hz),7.28(1H, d), 8.68(1H, s).

### Compound No. G-2

(CDCl₃) δ 1.39(6H, d, J=6.08Hz), 2.75(3H, s), 4.52(1H, septet, J=6.04Hz), 6.98(1H, d, J=7.27Hz), 7.24(1H, d), 8.81(1H, s).

### Compound No. J-19

(CDCl₃) δ 1.37(6H, d, J=6.3Hz), 2.18(3H, d, J=2.5Hz), 2.60(3H, s), 5.27(1H, septet, J=6.3Hz), 7.30(1H, d, J=9.3Hz), 7.99(1H, d, J=7.7Hz), 9.01(1H, s)

### Compound No. J-22

(CDCl₃) δ 1.26(3J, t, J=7.2Hz), 1.69(3H, d, J=6.8Hz), 2.36-2.38(3H, m), 4.19-4.25(2H, m), 4.77(1H, q, J=6.8Hz), 6.69(1H, t, J=54.0Hz), 6.99(1H, d, J=6.2Hz), 7.28(1H, d, J=9.0Hz), 9.15(1H, s)

### Compound No. J-29

(CDCl₃) δ 1.37(6H, d, J=6.3Hz), 2.18(3H, d, J=2.5Hz), 2.60(3H, s), 5.27(1H, septet, J=6.3Hz), 7.30(1H, d, J=9.3Hz), 7.99(1H, d, J=7.7Hz), 9.01(1H, s)

### Compound No. K-9

(CDCl₃) δ 1.25(3H, t, J=7.4Hz), 3.29-3.34(1H, m), 3.52-3.57(1H, m), 4.20-4.25(5H, m), 4.55-4.60(1H, m), 7.33(1H, d, J=9.5Hz), 7.58(1H, J=7.5Hz), 8.96(1H, s)

### Compound No. K-10

(CDCl₃) δ 1.25(3H, t, J=7.2Hz), 1.53(3H, d, J=7.0Hz), 4.19(3H, s), 4.10-4.30(3H, m), 4.83(1H, d, J=7.8Hz), 6.75(1H, d, J=6.2Hz), 7.25(1H, d, J=9.3Hz), 8.93(1H, s)

### Compound No. K-16

(CDCl₃) δ 1.38(6H, d, J=6.2Hz), 1.51(3H, t, J=7.1Hz), 4.65(2H, q, J=7.1Hz), 5.25-5.30(1H, m), 7.39(1H, d, J=9.6Hz), 8.15(1H, d, J=7.6Hz), 8.95(1H, s)

### Formulation Example 1

Wettable powder
Compound No. A-1 (10 wt%),
Tween ZO™ (20 wt%),
white carbon (40 wt%),
clay (30 wt%),
were mixed and ground to produce a wettable powder. (Diluted appropriately with water before use.)

### Formulation Example 2

Wettable powder
Compound No. A-2 (80 wt%).
sodium dodecylbenzenesulfonate (2 wt%),
sodium naphthalenesulfonate (3 wt%),
clay (15 wt%)
were mixed and ground to produce a wettable powder. (Diluted appropriately with water before use.)

### Formulation Example 3

Wettable powder
Compound No. A-24 (5 wt%),
polyoxyethylene glycol nonylphenylether
(NONIPOL 85™) (3 wt%),
sodium lignin sulfonate (5 wt%)
clay (87 wt%)
were mixed and ground to produce a wettable powder. (Diluted appropriately with water before use.)

### Formulation Example 4

Emulsion
Compound No. A-35 (2 wt%),
xylene (75 wt%),
dimethylformamide (18 wt%)
polyoxyethylene glycol nonylphenylether
(NONIPOL 85™) (5 wt%)
were mixed and ground to produce an emulsion. (Diluted appropriately with water before use.)

### Formulation Example 5

Flowable preparation
Compound No. A-57 (2 wt%),
polyoxyethylene allylphenylether-formamide condensation product
(NEW KALGON E-300™) (3 wt%),
polyoxyethylene phenylphenol ether sulfate
(AGRIZOL FL-2017™) (2 wt%),
polyol special polymer
(AGRIZOL FL-104FA™) (15 wt%),
white carbon (2 wt%),
ethylene glycol (10 wt%)
water (66 wt%)
were mixed and wet ground to produce a suspended flowable preparation. (Diluted appropriately with water before use.)

### Test Example 1

### Herbicidal effects against weeds Cultivation of plants to be tested

After filling 300g field soil in jiffypot™ having a diameter of 10 cm, the soil was steam sterilized. As treatment before emergence, seeds of southern crabgrass, green foxtail, velvetleaf, redroot pigweed, tall morningglory, corn and soybeans were scattered onto the surface of the soil, and then they were served for the test after covering with the soil up to be 0.5cm for weeds and 1cm for crops. As treatment after emergence, the above-mentioned seeds were scattered onto the surface of the soil, and cultivated them after covering with the soil up to be 0.5cm for weeds and 1cm for crops, and then they were served for the test when the weed and the crop reached the designated growth stage.

### Preparation of test solutions and method of treatment

The compound to be tested was dissolved in acetone which contains Tween 20™ and diluted with deionized water to prepare the test solutions for 10g/a and 1g/a use. The test solution was sprayed on the pots in a device with a spraygun.

### Method of indicating effect and phytotoxicity

The effect of the compound tested on the weeds and their phytotoxicity on the crops were evaluated two weeks after treating the weeds and crops for post-emergence treatment and three weeks thereafter for pre-emergence treatment as 6 grades from 0 to 5, which were shown in Table 16 and Table 17.

The test results were shown in Tables 18 to 28.

**Table 16**

| Herbicidal effects | | |
|---|---|---|
| Index | Effect | Weed control (%) |
| 0 | no effect | 0 |
| 1 | small | 0.1 to 50.0 |
| 2 | middle | 50.1 to 75.0 |
| 3 | big | 75.1 to 87.5 |
| 4 | maximum | 87.6 to 99.9 |
| 5 | maximum (all dead) | 100 |

**Table 17**

| Phytotoxicity | | |
|---|---|---|
| Index | Effect | Crop injury (%) |
| 0 | no effect | 0 |
| 1 | small | 0.1 to 12.5 |
| 2 | middle | 12.6 to 25.0 |
| 3 | big | 25.1 to 50.0 |
| 4 | maximum | 50.1 to 99.9 |
| 5 | maximum (all dead) | 100 |

**Table 18**

| | | | Evaluation index | | | | | | |
|---|---|---|---|---|---|---|---|---|---|
| Compound No | TreatMent method | g/a | Corn | Soy beans | Southearn crab grass' | Green-foxtail | Tall-morning glory | Velvet leaf | Red-root pigment |
| A-1 | pre | 10 | 0 | 0 | 4 | 4 | 1 | 2 | - |
| | post | 10 | 1 | 4 | 1 | 1 | 4 | 4 | - |
| A-3 | pre | 10(1) | 1(0) | 3(0) | 5(1) | 5(4) | 2(1) | 5(3) | - |
| | post | 10(1) | 4(2) | 4(4) | 4(2) | 5(3) | 5(5) | 5(5) | - |
| A-4 | pre | 10(1) | 2(0) | 2(0) | 5(4) | 5(4) | - | 5(1) | 5(5) |
| | post | 10(1) | 2(2) | 4(4) | 4(2) | 5(3) | - | 5(5) | 5(5) |
| A-5 | pre | 10(1) | 2(0) | 2(0) | 5(2) | 5(4) | - | 3(1) | 5(4) |
| | post | 10(1) | 3(2) | 4(4) | 4(1) | 4(3) | - | 5(4) | 5(5) |
| A-6 | post | 10(1) | 5(4) | 4(4) | 5(4) | 3(1) | 5(5) | 5(5) | - |
| A-7 | pre | 10(1) | 1(0) | 0(0) | 4(1) | 5(3) | - | 1(1) | 5(5) |
| | post | 10(1) | 4(4) | 4(4) | 5(4) | 5(4) | - | 5(5) | 5(5) |
| A-8 | pre | 10(1) | 2(0) | 0(0) | 4(1) | 4(1) | 0(0) | 3(2) | - |
| | post | 10(1) | 4(4) | 4(4) | 5(4) | 4(2) | 5(5) | 5(5) | - |
| A-9 | post | 10(1) | 3(1) | 5(4) | 4(1) | 3(1) | - | 5(5) | 5(5) |
| A-10 | post | 10(1) | 2(2) | 4(4) | 2(2) | 3(1) | 5(5) | 5(5) | - |
| A-11 | post | 10(1) | 2(2) | 4(3) | 3(1) | 3(1) | - | 4(2) | 5(5) |
| A-12 | post | 10(1) | 2(2) | 4(3) | 4(2) | 3(2) | - | 5(4) | 5(5) |
| A-13 | post | 10(1) | 0(0) | 4(3) | 1(1) | 1(1) | - | 4(4) | 5(5) |
| A-14 | post | 10(1) | 3(2) | 4(4) | 1(1) | 2(1) | - | 4(4) | 5(4) |
| A-16 | post | 10(1) | 3(2) | 4(4) | 3(3) | 2(2) | - | 5(5) | 5(5) |
| A-17 | post | 10(1) | 2(2) | 4(4) | 1(1) | 3(1) | - | 4(2) | 5(5) |
| A-22 | post | 10(1) | 3(2) | 4(2) | 3(1) | 2(1) | - | 5(5) | 5(5) |

**Table 19**

| | | | Evaluation index | | | | | | |
|---|---|---|---|---|---|---|---|---|---|
| Compound No. | TreatMent method | g/a | Corn | Soy beans | Southearn crab grass¹ | Green-fox tail | Tall-Morning glory | Velvet leaf | Red-root pigweed |
| A-23 | post | 10(1) | 3(2) | 5(4) | 2(1) | 2(1) | - | 5(5) | 5(5) |
| A-24 | post | 10(1) | 4(4) | 5(5) | 5(4) | 4(2) | - | 5(5) | 5(5) |
| A-25 | pre | 10(1) | 0(0) | 0(0) | 5(1) | 5(3) | - | 5(1) | 5(3) |
| | post | 10(1) | 4(4) | 4(4) | 4(1) | 4(3) | - | 5(5) | 5(5) |
| A-28 | post | 10(1) | 1(0) | 3(3) | 1(1) | 2(1) | - | 5(5) | 5(5) |
| A-29 | pre | 10 | 0 | 0 | 4 | 5 | 0 | 1 | - |
| | post | 10 | 1 | 4 | 2 | 2 | 5 | 5 | - |
| A-30 | pre | 10(1) | 0(0) | 0(0) | 4(1) | 4(1) | - | 1(0) | 4(2) |
| | post | 10(1) | 1(1) | 4(3) | 3(1) | 4(2) | - | 4(4) | 5(5) |
| A-31 | post | 10(1) | 4(3) | 4(3) | 1(1) | 3(1) | 5(4) | 5(4) | - |
| A-32 | post | 10(1) | 4(4) | 4(3) | 4(2) | 3(1) | - | 5(4) | 5(5) |
| A-33 | post | 10(1) | 4(4) | 4(3) | 4(2) | 3(1) | - | 5(4) | 5(5) |
| A-34 | post | 10(1) | 4(4) | 4(4) | 4(2) | 2(1) | - | 5(5) | 5(2) |
| A-37 | pre | 10(1) | 4(0) | 2(0) | 4(0) | 5(0) | - | 1(1) | 5(5) |
| | post | 10(1) | 4(3) | 4(4) | 3(1) | 4(2) | - | 5(5) | 5(5) |
| A-38 | pre | 10(1) | 0(0) | 0(0) | 4(1) | 5(3) | - | 1(1) | 5(5) |
| | post | 10(1) | 3(3) | 4(4) | 4(3) | 4(4) | - | 5(5) | 5(5) |
| A-40 | post | 10(1) | 2(0) | 3(2) | 1(1) | 1(1) | - | 4(1) | 5(5) |
| A-41 | post | 10(1) | 1(1) | 4(4) | 1(1) | 1(1) | - | 5(3) | 5(5) |
| A-42 | post | 10(1) | 2(2) | 4(4) | 1(1) | 1(1) | - | 5(4) | 5(5) |
| A-43 | post | 10(1) | 3(2) | 4(4) | 1(1) | 2(1) | - | 4(4) | 5(5) |
| A-44 | post | 10(1) | 2(1) | 4(4) | 1(1) | 1(1) | - | 5(4) | 5(5) |

**Table 20**

| | | | Evaluation index | | | | | | |
|---|---|---|---|---|---|---|---|---|---|
| Compound No. | Treatment method | g/a | Corn | Soy beans | Southern crabgrass | Green-foxtail | Tall-morning glory | Velvet leaf | Red-root pigment |
| B-1 | post | 10 | 1 | 4 | 2 | 2 | 5 | 5 | - |
| B-2 | pre | 10 | 0 | 0 | 4 | 4 | - | 2 | 5 |
| B-3 | pre | 10(1) | 0(0) | 0(0) | 4(2) | 5(4) | - | 3(1) | 5(5) |
| | post | 10(1) | 3(2) | 4(4) | 4(2) | 4(4) | - | 5(5) | 5(5) |
| B-4 | pre | 10(1) | 3(0) | 0(0) | 3(0) | 4(1) | - | 5(1) | 5(3) |
| | post | 10(1) | 4(4) | 4(4) | 5(4) | 4(3) | - | 5(5) | 5(5) |
| B-5 | post | 10(1) | 4(4) | 4(4) | 4(4) | 4(1) | - | 5(5) | 5(5) |
| B-6 | post | 10(1) | 4(3) | 4(4) | 4(3) | 4(2) | - | 4(3) | 5(5) |
| D-1 | pre | 10(1) | 1(0) | 0(0) | 5(4) | 5(3) | - | 5(1) | 5(5) |
| | post | 10(1) | 3(2) | 4(4) | 4(1) | 5(3) | - | 5(4) | 5(5) |
| D-2 | pre | 10(1) | 0(0) | 0(0) | 4(1) | 5(1) | - | 4(0) | 5(5) |
| | post | 10(1) | 4(3) | 4(4) | 3(1) | 5(2) | - | 5(5) | 5(5) |
| D-3 | pre | 10(1) | 1(0) | 4(2) | 4(1) | 5(3) | - | 4(1) | 5(5) |
| | post | 10(1) | 4(3) | 4(4) | 4(2) | 5(4) | - | 5(5) | 5(5) |
| D-4 | post | 10(1) | 4(4) | 4(4) | 5(3) | 5(3) | - | 5(5) | 5(5) |
| D-5 | pre | 10(1) | 3(1) | 1(0) | 4(0) | 4(1) | - | 4(1) | 5(3) |
| | post | 10(1) | 5(4) | 4(4) | 5(3) | 5(3) | - | 5(5) | 5(5) |
| D-6 | post | 10(1) | 1(0) | 4(2) | 2(1) | 3(1) | - | 5(4) | 5(4) |
| D-7 | pre | 10 | 0 | 0 | 4 | 5 | - | 4 | 5 |
| | post | 10(1) | 2(1) | 4(3) | 3(1) | 4(3) | - | 4(4) | 5(4) |
| D-8 | pre | 10(1) | 3(0) | 0(0) | 5(3) | 5(4) | - | 4(2) | 5(5) |
| | post | 10(1) | 3(2) | 4(4) | 4(2) | 4(4) | - | 5(4) | 5(5) |

**Table 21**

| | | | Evaluation index | | | | | | |
|---|---|---|---|---|---|---|---|---|---|
| Compound No. | Treatment method | g/a | Corn | Soy beans | Southern crabgrass | Green-foxtail | Tall-morning glory | Velvet leaf | Red-root pigment |
| D-9 | pre | 10 | 0 | 0 | 5 | 5 | - | 4 | 5 |
| | post | 10(1) | 3(2) | 4(3) | 4(1) | 4(4) | - | 5(4) | 5(5) |
| D-10 | pre | 10 | 3 | 0 | 5 | 5 | - | 4 | 5 |
| | post | 10(1) | 3(3) | 4(4) | 4(1) | 4(4) | - | 5(4) | 5(5) |
| D-11 | pre | 10(1) | 0(0) | 0(0) | 4(0) | 5(0) | - | 4(1) | 5(5) |
| | post | 10(1) | 5(4) | 4(4) | 5(4) | 5(4) | - | 5(4) | 5(5) |
| D-12 | post | 10(1) | 3(3) | 4(4) | 3(2) | 4(4) | - | 5(4) | 5(5) |
| D-13 | pre | 10(1) | 0(0) | 0(0) | 3(1) | 4(1) | - | 4(0) | 5(5) |
| | post | 10(1) | 3(3) | 4(4) | 3(1) | 4(2) | - | 5(5) | 5(5) |
| D-14 | pre | 10(1) | 1(0) | 0(0) | 2(0) | 3(0) | - | 2(1) | 5(5) |
| | post | 10(1) | 3(3) | 4(4) | 3(2) | 3(2) | - | 4(4) | 5(5) |
| D-15 | pre | 10(1) | 0(0) | 0(0) | 2(1) | 3(1) | - | 1(0) | 5(5) |
| | post | 10(1) | 3(3) | 3(2) | 1(1) | 3(1) | - | 5(3) | 5(5) |
| D-16 | pre | 10(1) | 0(0) | 0(0) | 0(0) | 0(0) | - | 2(0) | 5(5) |
| | post | 10(1) | 3(2) | 3(3) | 1(1) | 2(1) | - | 5(4) | 5(5) |
| D-17 | pre | 10 | 4 | 0 | 4 | 4 | - | 1 | 5 |
| | post | 10(1) | 5(3) | 5(4) | 5(5) | 5(5) | - | 5(5) | 5(5) |
| D-18 | pre | 10(1) | 5(3) | -(-) | 5(4) | 5(5) | - | 2(1) | 5(5) |
| | post | 10(1) | 4(4) | 4(4) | 5(5) | 5(5) | - | 5(5) | 5(5) |
| D-19 | pre | 10 | 0 | 0 | 0 | 0 | - | 1 | 5 |
| | post | 10(1) | 4(2) | 5(4) | 5(5) | 3(3) | - | 5(5) | 5(5) |
| D-22 | post | 10 | 2 | 4 | 1 | 3 | - | 5 | 5 |

**Table 22**

| | | | Evaluation index | | | | | | |
|---|---|---|---|---|---|---|---|---|---|
| Compound No. | TreatMent mothod | g/a | Corn | Soy beans | Southern crabgrass | Green-foxtail | Tall-morning glory | Velvet leaf | Red-root pigment |
| D-25 | pre | 10(1) | 1(0) | 0(0) | 3(0) | 4(0) | - | 5(5) | 5(5) |
| | post | 10(1) | 4(4) | 4(4) | 5(3) | 4(3) | - | 5(5) | 5(5) |
| D-26 | pre | 10(1) | 0(0) | 4(0) | 1(1) | 3(1) | - | 4(1) | 5(5) |
| | post | 10(1) | 4(2) | 4(3) | 4(2) | 2(2) | - | 5(5) | 5(5) |
| D-27 | pre | 10(1) | 2(0) | 0(0) | 4(0) | 2(0) | - | 5(5) | 5(5) |
| | post | 10(1) | 5(4) | 4(4) | 5(5) | 5(4) | - | 5(5) | 5(5) |
| D-28 | pre | 10(1) | 0(0) | 0(0) | 2(1) | 4(1) | - | 5(4) | 5(5) |
| | post | 10(1) | 3(3) | 4(4) | 3(2) | 3(2) | - | 5(5) | 5(5) |
| D-29 | pre | 10(1) | 0(0) | 0(0) | 2(0) | 2(0) | - | 4(1) | 5(5) |
| | post | 10(1) | 4(4) | 4(4) | 5(4) | 5(3) | - | 5(5) | 5(5) |
| D-30 | pre | 10(1) | 0(0) | 0(0) | 2(1) | 3(1) | - | 5(1) | 5(5) |
| | post | 10(1) | 3(1) | 3(3) | 2(1) | 3(1) | - | 5(5) | 5(5) |
| D-31 | pre | 10(1) | 0(0) | 0(0) | 2(0) | 3(0) | - | 2(2) | 5(5) |
| | post | 10(1) | 2(1) | 4(4) | 2(1) | 3(1) | - | 5(5) | 5(5) |
| D-32 | pre | 10(1) | 0(0) | 0(0) | 3(2) | 3(1) | - | -(5) | 5(5) |
| | post | 10(1) | 3(3) | 4(4) | 3(2) | 2(1) | - | 5(5) | 5(5) |
| D-33 | pre | 10(1) | 0(0) | 0(0) | 2(0) | 2(0) | - | 2(0) | 5(5) |
| | post | 10(1) | 4(3) | 4(4) | 4(3) | 4(2) | - | 5(5) | 5(5) |
| D-34 | pre | 10(1) | 1(0) | 0(0) | 4(0) | 4(1) | - | 4(1) | 5(5) |
| | post | 10(1) | 4(3) | 4(4) | 4(2) | 4(3) | - | 5(4) | 5(5) |
| D-35 | post | 10(1) | 1(1) | 4(3) | 1(1) | 3(1) | - | 1(1) | 5(5) |
| D-36 | pre | 10 | 1 | 0 | 4 | 5 | - | 4 | 5 |
| | post | 10(1) | 3(2) | 4(4) | 4(2) | 4(2) | - | 5(5) | 5(5) |

**Table 23**

| | | | Evaluation index | | | | | | |
|---|---|---|---|---|---|---|---|---|---|
| Compound No. | Treatment mothod | g/a | Corn | Soy beans | Southern crabgrass | Green-foxtail | Tall-morning glory | Velvet leaf | Red-root pigment |
| D-37 | post | 10(1) | 2(2) | 3(3) | 1(1) | 1(1) | - | 2(2) | 5(5) |
| D-38 | post | 10(1) | 3(1) | 3(2) | 2(2) | 2(1) | - | 5(4) | 5(5) |
| D-39 | post | 10 | 4 | 4 | 1 | 1 | - | 3 | 5 |
| D-40 | pre | 10 | 4 | 0 | 3 | 3 | - | 4 | 5 |
| | post | 10(1) | 4(3) | 4(4) | 4(1) | 4(1) | - | 5(5) | 5(5) |
| D-41 | pre | 10(1) | 4(0) | 0(0) | 4(1) | 4(1) | - | 4(1) | 5(5) |
| | post | 10(1) | 4(2) | 4(3) | 4(1) | 4(1) | - | 5(4) | 5(5) |
| D-42 | pre | 10(1) | 2(0) | 0(0) | 5(4) | 5(4) | - | 2(1) | 5(5) |
| | post | 10(1) | 4(3) | 4(4) | 5(4) | 4(4) | - | 5(5) | 5(5) |
| D-43 | pre | 10(1) | 3(0) | 1(0) | 2(1) | 2(1) | - | 2(1) | 5(5) |
| | post | 10(1) | 4(2) | 4(3) | 4(1) | 4(1) | - | 5(4) | 5(5) |
| D-44 | pre | 10(1) | 4(0) | 1(0) | 3(0) | 5(2) | - | 2(1) | 5(5) |
| | post | 10(1) | 5(5) | 5(4) | 5(4) | 5(5) | - | 5(5) | 5(5) |
| D-45 | pre | 10 | 4 | 0 | 2 | 3 | - | 1 | 5 |
| | post | 10(1) | 4(4) | 4(4) | 2(1) | 3(2) | - | 5(4) | 5(5) |
| D-46 | pre | 10 | 2 | 0 | 5 | 5 | - | 3 | 5 |
| | post | 10(1) | 4(3) | 4(4) | 4(1) | 4(3) | - | 5(5) | 5(5) |
| D-47 | pre | 10 | 4 | 0 | 2 | 3 | - | 2 | 5 |
| | post | 10(1) | 4(4) | 4(4) | 4(2) | 4(2) | - | 5(5) | 5(5) |
| E-6 | post | 10(1) | 4(3) | 4(4) | 5(3) | 4(3) | - | 5(5) | 5(5) |
| E-7 | post | 10(1) | 4(2) | 4(3) | 2(1) | 2(1) | - | 5(5) | 5(5) |
| E-8 | post | 10(1) | 3(2) | 4(4) | 4(1) | 4(3) | - | 5(4) | 5(5) |

**Table 24**

| | | | Evaluation index | | | | | | |
|---|---|---|---|---|---|---|---|---|---|
| Compound No. | Treatment method | g/a | Corn | Soy beans | southern crabgrass | Green-foxtail | Tall-morning glory | Velvet leaf | Red-root pigment |
| E-9 | pre | 10(1) | 4(0) | 0(0) | 1(0) | 3(1) | - | 5(4) | 5(5) |
| | post | 10(1) | 4(3) | 4(3) | 3(1) | 2(0) | - | 5(5) | 5(5) |
| E-10 | pre | 10(1) | 4(0) | 0(0) | 4(1) | 4(3) | - | 5(5) | 5(5) |
| | post | 10(1) | 4(3) | 4(4) | 4(2) | 4(1) | - | 5(5) | 5(5) |
| E-11 | pre | 10 | 0 | 0 | 3 | 1 | - | 2 | 5 |
| | post | 10(1) | 3(3) | 4(4) | 3(2) | 2(2) | - | 5(5) | 5(5) |
| E-12 | pre | 10 | 0 | 0 | 0 | 0 | - | 3 | 5 |
| | post | 10(1) | 0(0) | 2(2) | 0(0) | 0(0) | - | 5(4) | 5(5) |
| E-13 | pre | 10 | 0 | 0 | 0 | 0 | - | 1 | 5 |
| | post | 10(1) | 0(0) | 2(2) | 0(0) | 0(0) | - | 5(2) | 5(5) |
| E-14 | post | 10 | 2 | 4 | 1 | 2 | - | 5 | 5 |
| E-15 | post | 10 | 3 | 4 | 2 | 2 | - | 5 | 5 |
| E-18 | post | 10 | 2 | 4 | 1 | 1 | - | 5 | 5 |
| E-21 | post | 10 | 4 | 4 | 2 | 2 | - | 5 | 5 |
| E-22 | post | 10(1) | 3(2) | 4(4) | 3(3) | 3(1) | - | 5(5) | 5(5) |
| F-4 | post | 10(1) | 1(0) | 0(0) | 1(0) | 0(0) | 4(1) | 5(2) | - |
| H-1 | pre | 10 | 0 | 0 | 4 | 4 | - | 3 | 5 |
| | post | 10(1) | 3(2) | 4(4) | 3(1) | 4(3) | - | 5(4) | 5(4) |
| H-2 | pre | 10 | 0 | 0 | 2 | 2 | - | 1 | 5 |
| | post | 10(1) | 1(1) | 2(0) | 1(1) | 1(1) | - | 5(5) | 4(1) |
| H-5 | post | 10(1) | 1(1) | 3(2) | 1(1) | 1(1) | - | 5(5) | 1(1) |
| H-6 | post | 10(1) | 1(1) | 3(3) | 1(1) | 1(1) | - | 5(4) | 4(1) |

**Table 25**

| | | | Evaluation index | | | | | | |
|---|---|---|---|---|---|---|---|---|---|
| Compound No. | Treatment mothod | g/a | Corn | Soy beans | Southern crabgrass | Green-foxtail | Tall-morning glory | Velvet leaf | Red-root pigment |
| H-7 | pre | 10 | 0 | 0 | 0 | 0 | - | 4 | 4 |
| | post | 10(1) | 1(1) | 3(3) | 1(1) | 1(1) | - | 5(5) | 4(2) |
| H-8 | pre | 10 | 0 | 0 | 0 | 0 | - | 5 | 5 |
| | post | 10(1) | 0(0) | 1(1) | 0(0) | 0(0) | - | 5(5) | 5(5) |
| H-9 | pre | 10 | 0 | 0 | 3 | 4 | - | 1 | 5 |
| | post | 10(1) | 3(1) | 4(4) | 2(1) | 3(1) | - | 5(5) | 5(5) |
| H-10 | post | 10(1) | 1(1) | 2(2) | 0(0) | 0(0) | - | 5(5) | 1(1) |
| H-11 | post | 10(1) | 2(1) | 3(2) | 0(0) | 1(0) | - | 5(4) | 5(5) |
| H-12 | post | 10(1) | 1(0) | 2(1) | 0(0) | 1(0) | - | 4(4) | 4(2) |
| H-13 | post | 10(1) | 2(1) | 2(2) | 0(0) | 1(0) | - | 4(1) | 5(5) |
| H-14 | post | 10(1) | 2(2) | 4(3) | 1(1) | 1(1) | - | 5(4) | 5(5) |
| H-17 | post | 10(1) | 1(1) | 2(2) | 1(1) | 1(1) | - | 5(5) | 2(1) |
| H-20 | post | 10(1) | 1(1) | 3(2) | 1(0) | 1(0) | - | 5(4) | 5(5) |
| H-22 | post | 10(1) | 1(1) | 2(2) | 0(0) | 0(0) | - | 5(4) | 5(5) |
| H-25 | post | 10(1) | 0(0) | 2(0) | 1(1) | 1(1) | - | 5(2) | 5(5) |
| H-27 | post | 10(1) | 2(2) | 4(3) | 1(1) | 1(1) | - | 5(2) | 5(5) |
| H-28 | post | 10(1) | 2(0) | 2(2) | 1(1) | 1(1) | - | 5(3) | 5(4) |
| J-2 | post | 10(1) | 3(2) | 4(3) | 1(1) | 3(1) | - | 4(1) | 5(4) |
| J-3 | post | 10(1) | 3(2) | 4(4) | 3(1) | 4(1) | · | 4(2) | 5(5) |
| J-4 | post | 10 | 1 | 2 | 1 | 1 | · | 4 | 4 |
| J-5 | pre | 10 | 0 | 0 | 4 | 4 | - | 2 | 5 |
| | post | 10 | 2 | 4 | 1 | 3 | · | 4 | 5 |

**Table 26**

| | | | Evaluation index | | | | | | |
|---|---|---|---|---|---|---|---|---|---|
| Compound No. | Treatment mothod | g/a | Corn | Soy beans | Southern crabgrass | Green-foxtail | Tall-morning glory | Velvet leaf | Red-root pigment |
| J-6 | post | 10 | 4 | 4 | 2 | 4 | - | 5 | 5 |
| J-7 | pre | 10 | 0 | 0 | 5 | 5 | - | 3 | 5 |
| | post | 10(1) | 4(3) | 4(4) | 4(2) | 4(4) | - | 5(4) | 5(4) |
| J-8 | post | 10(1) | 4(4) | 4(4) | 4(3) | 4(4) | - | 5(4) | 5(5) |
| J-9 | pre | 10(1) | 3(0) | 1(0) | 5(2) | 5(3) | - | 3(2) | 5(5) |
| | post | 10(1) | 4(4) | 4(4) | 5(4) | 5(4) | - | 5(4) | 5(5) |
| J-10 | pre | 10(1) | 0(0) | 0(0) | 2(0) | 2(0) | - | 1(1) | 5(5) |
| | post | 10(1) | 4(3) | 4(4) | 5(4) | 4(1) | - | 5(5) | 5(5) |
| J-1 | pre | 10(1) | 4(0) | 0(0) | 1(0) | 2(0) | - | 5(4) | 5(5) |
| | post | 10(1) | 4(3) | 4(3) | 3(1) | 3(1) | - | 5(4) | 5(5) |
| J-12 | pre | 10(1) | 4(0) | 0(0) | 1(0) | 3(0) | - | 5(5) | 5(5) |
| | post | 10(1) | 3(3) | 3(2) | 1(1) | 3(1) | - | 5(5) | 5(5) |
| J-13 | pre | 10(1) | 4(0) | 0(0) | 0(0) | 2(0) | - | 5(4) | 5(5) |
| | post | 10(1) | 4(4) | 2(2) | 1(1) | 3(1) | - | 5(4) | 5(5) |
| J-14 | pre | 10(1) | 4(0) | 0(0) | 1(0) | 4(1) | - | 5(4) | 5(5) |
| | post | 10(1) | 4(3) | 4(2) | 3(1) | 3(1) | - | 5(5) | 5(5) |
| J-15 | post | 10(1) | 3(1) | 4(3) | 1(1) | 2(1) | - | 5(5) | 5(5) |
| J-16 | post | 10(1) | 0(0) | 2(1) | 1(0) | 1(0) | - | 5(5) | 4(1) |
| J-17 | post | 10(1) | 0(0) | 2(2) | 1(0) | 1(0) | - | 5(5) | 5(1) |
| J-18 | post | 10(1) | 0(0) | 1(1) | 0(0) | 1(1) | - | 5(5) | 3(1) |
| J-19 | pre | 10 | 0 | 0 | 4 | 4 | - | 4 | 5 |
| | post | 10 | 3 | 4 | 1 | 3 | - | 4 | 3 |

**Table 27**

| | | | Evaluation index | | | | | | |
|---|---|---|---|---|---|---|---|---|---|
| Compound No. | Treatment method | g/a | Corn | Soy bean s | Southern crabgrass | Green-foxtail | Tall-morning glory | Velvet leaf | Red-root pigmeat |
| J-20 | pre | 10(1) | 4(0) | 0(0) | 5(1) | 5(4) | - | 5(1) | 5(4) |
| | post | 10(1) | 4(2) | 4(4) | 4(1) | 4(3) | - | 5(4) | 5(5) |
| J-21 | post | 10(1) | 4(4) | 1(1) | 3(1) | 4(2) | - | 5(4) | 5(5) |
| J-22 | post | 10(1) | 4(4) | 4(4) | 3(1) | 4(2) | - | 5(4) | 5(5) |
| J-23 | pre | 10 | 2 | 0 | 3 | 4 | - | 5 | 5 |
| | post | 10(1) | 3(2) | 4(3) | 1(1) | 3(1) | - | 5(4) | 5(5) |
| J-24 | post | 10 | 2 | 4 | 1 | 2 | - | 5 | 5 |
| J-25 | post | 10(1) | 2(1) | 4(4) | 4(1) | 4(2) | - | 5(5) | 5(5) |
| J-27 | post | 10(1) | 2(1) | 1(1) | 1(1) | 1(1) | - | 5(5) | 5(5) |
| J-28 | post | 10 | 3 | 4 | 1 | 1 | - | 5 | 5 |
| J-29 | post | 10(1) | 3(2) | 4(3) | 3(1) | 4(1) | - | 4(4) | 5(5) |
| J-30 | post | 10(1) | 3(2) | 4(4) | 1(1) | 1(1) | - | 5(4) | 5(5) |
| J-31 | post | 10 | 1 | 3 | 1 | 1 | - | 4 | 5 |
| J-32 | post | 10(1) | 2(1) | 4(4) | 1(1) | 1(1) | - | 5(5) | 5(5) |
| K-1 | pre | 10(1) | 2(0) | 0(0) | 4(2) | 4(2) | - | 2(1) | 5(5) |
| | post | 10(1) | 2(1) | 4(4) | 4(2) | 4(3) | - | 5(4) | 5(5) |
| K-2 | pre | 10 | 0 | 0 | 0 | 0 | - | 5 | 5 |
| | post | 10(1) | 1(0) | 2(1) | 0(0) | 1(1) | - | 5(5) | 4(1) |
| K-3 | pre | 10 | 0 | 0 | 0 | 0 | - | 5 | 5 |
| | post | 10(1) | 0(0) | 3(2) | 1(1) | 1(1) | - | 5(5) | 5(3) |
| K-4 | pre | 10 | 0 | 0 | 0 | 1 | - | 5 | 5 |
| | post | 10(1) | 1(1) | 3(2) | 1(1) | 1(1) | - | 5(5) | 5(5) |

**Table 28**

| | | | Evaluation index | | | | | | |
|---|---|---|---|---|---|---|---|---|---|
| Compound No. | Treatment mothod | g/a | Corn | Soy beans | Southern crabgrass | Green-foxtail | Tall-morning glory | Velvet leaf | Red-root pigment |
| K-5 | pre | 10 | 0 | 0 | 0 | 1 | - | 4 | 5 |
| | post | 10(1) | 0(0) | 3(3) | 1(1) | 1(1) | - | 5(5) | 5(5) |
| K-6 | pre | 10 | 1 | 0 | 3 | 5 | - | 5 | 5 |
| | post | 10(1) | 3(3) | 4(4) | 3(1) | 4(3) | - | 5(5) | 5(4) |
| K-7 | pre | 10(1) | 3(0) | 2(0) | 3(1) | 4(3) | - | 5(4) | 5(4) |
| | post | 10(1) | 3(3) | 3(2) | 2(1) | 3(2) | - | 5(5) | 5(3) |
| K-8 | pre | 10 | 0 | 0 | 1 | 1 | - | 4 | 5 |
| | post | 10(1) | 3(2) | 3(3) | 1(1) | 1(1) | - | 5(5) | 5(5) |
| K-9 | post | 10(1) | 4(4) | 4(4) | 1(1) | 1(1) | - | 5(5) | 5(5) |
| K-17 | pre | 10 | 2 | 0 | 4 | 5 | - | 5 | 5 |
| | post | 10 | 3 | 3 | 2 | 4 | - | 4 | 5 |
| K-18 | pre | 10 | 1 | 0 | 4 | 5 | - | 2 | 5 |
| | post | 10(1) | 3(3) | 4(4) | 4(2) | 4(4) | - | 5(5) | 5(5) |

### INDUSTRIAL APPLICABILITY

A compound or a salt thereof has excellent herbicidal activity against a wide variety of weeds, for example, weeds in paddy field or in plow lands with a low dose. Moreover, it has little pytotoxicity on cultivated plants, for example, rice, wheat, barley, corn beans, cotton, and so on, and shows an excellent selective herbicidal effect. Also, the selective herbicidal effect can be maintained for a long time. It has little toxicity on mammals and fish and shellfish, without polluting environment, so it can be used extremely safely as a herbidical composition for paddy fields, plow lands, orchards or non-agricultural lands and so on.

## Claims

1. A pyrimidine derivative or a salt thereof represented by Formula (I) : 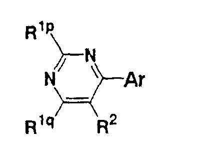 [wherein each of R^{1p} and R^{1q} may be the same or differently (1) hydrogen, (2) halogen, (3) a C₁₋₆alkyl group which may be substituted by a substituent or substituents selected from the group consisting of halogen, a C₁₋₆alkoxy group, a C₂₋₆alkenyloxy group, a C₂₋₆alkynyloxy group, C₁₋₆alkylthio group, C₂₋₆alkenylthio group, a C₂₋₆alkynylthio group, a C₁₋₆haloalkoxy group, a C₂₋₆haloalkenyloxy group, a C₁₋₆haloalkylthio group, a C₂₋₆haloalkenylthio group, hydroxy group, mercapto group and cyano group, the number of the substituent being a number falling within substitutable number, preferably from 1 to 6, (4) a C₁₋₆alkoxy group, (5) a C₂₋₆alkenyloxy group, (6) a C₂₋₆alkynyloxy group, (7) a C₁₋₆haloalkoxy group, (8) a C₂₋₆haloalkenyloxy group, (9) a C₁₋₆alkylthio group, (10) a C₂₋₆alkenylthio group, (11) a C₂₋₆alkynylthio group, (12) a C₁₋₆haloalkylthio group, (13) a C₂₋₆haloalkenylthio group, (14) an amino group which may be mono- or di-substituted by a substituent or substituents selected from a C₁₋₆alkyl group, a C₂₋₆alkenyl group, a C₂₋₆alkynyl group, a C₁₋₇ alkanoyl group and a cyclic amino which may contain one or two atoms selected from oxygen atom, sulfur atom and nitrogen atom, (15) a C₂₋₇alkoxycarbonyl group, (16) a C₃₋₇alkenyloxycarbonyl group, (17) a C₃₋₇ haloalkenyloxycarbonyl group, (18) a C₃₋₇alkynyloxycarbonyl group, (19) a carbamoyl group which may be substituted on its nitrogen atom by one or two C₁₋₄alkyl groups, (20) thiocarbamoyl group, (21) cyano group, or (22) formyl group, R² is halogen, a C₁₋₆alkyl group, a carbamoyl group which may be substituted on its nitrogen atom by one or two C₁₋₄alkyl groups, thiocarbamoyl group, cyano group or formyl group,
Ar is represented by one of the formulas: 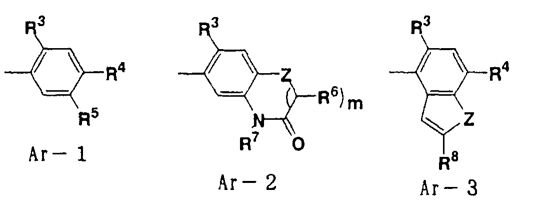 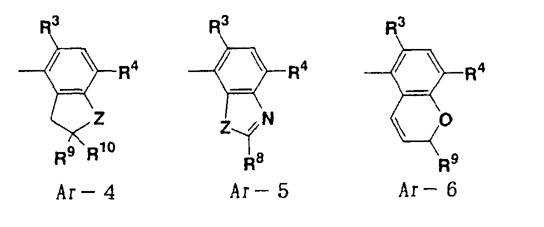 (wherein R³ is hydrogen, halogen, a C₁₋₆alkyl group, a C₁₋₆haloalkyl group, a C₁₋₆alkoxy group or a C₁₋₆haloalkoxy group,
R⁴ is (1) halogen, (2) nitro group, (3) cyano group, (4) carbamoyl group, (5) thiocarbamoyl group, (6) a hydroxyC₁₋₄alkyl group, (7) a C₁₋₄haloalkyl group, (8) a C₁₋₆alkoxy-C₁₋₄alkyl group, (9) a C₂₋₇alkoxycarbonyl group, (10) a C₃₋₇alkenyloxycarbonyl group, (11) a C₃₋₇alkynyloxycarbonyl group, (12) a C₁₋₆alkoxy group, (13) a C₂₋₆alkenyloxy group, (14) a C₂₋₆alkynyloxy group or (15) a C₇₋₁₂aralkyloxy group which may be substituted by halogen, a C₁₋₆alkyl group, a C₁₋₆haloalkyl group, a C₁₋₆alkoxy group, a C₁₋₆haloalkoxy group, a C₂₋₆alkenyloxy group, a C₂₋₆alkynyloxy group, a C₂₋₇alkoxycarbonyl group, a C₃₋₇alkenyloxycarbonyl group, a C₃₋₇alkynyloxycarbonyl group, a C₂₋₇alkoxycarbonyl-C₁₋₄alkoxy group, a C₃₋₇alkenyloxycarbonyl-C₁₋₄alkoxy group or a C₃₋₇alkynyloxycarbonyl-C₁₋₄alkoxy group, the number of the substituents being from one to the maximum substitutable number,
R⁵ is hydrogen, cyano group, a C₁₋₆alkyl group, a C₃₋₆cycloalkyl group, hydroxy group, mercapto group, a C₁₋₆alkoxy group, a C₁₋₆alkoxy-C₁₋₄alkoxy group, a C₃₋₆cycloalkyloxy group, a C₁₋₆haloalkoxy group, a C₂₋₆alkenyloxy group, a C₂₋₆haloalkenyloxy group, a C₂₋₆alkynyloxy group, a C₆₋₁₀aryloxy group, a C₇₋₁₂aralkyloxy group, a C₁₋₆alkythio group, a C₁₋₆alkoxyC₁₋₄alkylthio group, a C₃₋₆cycloalkylthio group, a C₁₋₆haloalkylthio group, a C₂₋₆alkenylthio group, a C₂₋₆haloalkenylthio group, a C₂₋₆alkynylthio group, a C₆₋₁₀arylthio group, a C₇₋₁₂aralkylthio group, a C₁₋₆alkylsulfonyl group, a C₃₋₆cycloalkylsulfonyl group, a C₁₋₆haloalkylsulfonyl group, a C₂₋₆alkenylsulfonyl group, a C₂₋₆haloalkenylsulfonyl group, a C₂₋₆alkynylsulfonyl group, or a cyclic amino group which may contain one or two oxygen atoms, sulfur atoms and nitrogen atoms, or alternatively
R⁵ is one of the groups represented by formulas: 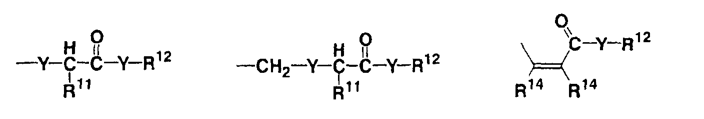 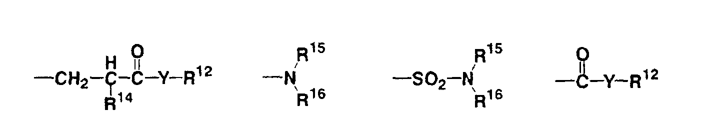 (wherein Y is oxygen, sulfur or -N-R¹³,
R¹¹ is hydrogen or a C₁₋₆alkyl group,
R¹² is hydrogen, a C₁₋₆alkyl group, a C₁₋₆haloalkyl group, a C₃₋₆cycloalkyl group, a C₂₋₆alkenyl group, C₂₋₆haloalkenyl group, a C₂₋₆alkynyl group, a C₆₋₁₀aryl group, a C₇₋₁₂aralkyl group, a C₁₋₆alkoxy-C₁₋₄alkyl group, a C₂₋₆alkenyloxy-C₁₋₄alkyl group, a C₂₋₆ alkynyloxy-C₁₋₄alkyl group, a C₃₋₆cycloalkoxy-C₁₋₄alkyl group, a C₂₋₇alkoxycarbonyl-C₁₋₄alkyl group, a C₃₋₇alkenyoxycarbonyl-C₁₋₄alkyl group, a C₃₋₇alkynyloxycarbonyl-C₁₋₄alkyl group, a C₄₋₇cycloalkoxycarbonyl-C₁₋₄alkyl group, a C₂₋₇haloalkoxycarbonyl-C₁₋₄alkyl group, a C₃₋₇haloalkenyloxycarbonyl-C₁₋₄alkyl group or a C₇₋₁₂aralkyloxycarbonyl-C₁₋₄alkyl group,
R¹³ is hydrogen, a C₁₋₆alkyl group, a C₁₋₆alkoxy-C₁₋₄alkyl group, a C₁₋₇alkanoyl group, a C₇₋₁₁arylcarbonyl group, a C₂₋₇alkoxycarbonyl group, a C₂₋₇haloalkoxycarbonyl group, or a C₃₋₇haloalkenyloxycarbonyl group,
R¹⁴ is hydrogen, halogen or a C₁₋₆alkyl group,
each of R¹⁵ and R¹⁶ is same or differently hydrogen, a C₁₋₆alkyl group, a C₂₋₆alkenyl group, a C₂₋₆alkynyl group, a C₃₋₆cycloalkyl group, a C₁₋₆alkoxy-C₁₋₄alkyl group, a C₆₋₁₂aralkyl group, a C₁₋₄alkyl group substituted by a 5-or 6-membered heterocyclic ring which may contain one or two atoms selected from nitrogen, oxygen and sulfur, a C₁₋₇alkanoyl group, a C₆₋₁₂arylcarbonyl group, a C₂₋₇haloalkylcarbonyl group, a C₂₋₇alkoxycarbonyl group, a C₃₋₇alkenyloxycarbonyl group, a C₃₋₇alkynyloxycarbonyl group, a C₄₋₇cycloalkyloxycarbonyl group, a C₂₋₇haloalkoxycarbonyl group, a C₁₋₆alkylsulfonyl group, a C₂₋₆alkenylsulfonyl group, a C₂₋₆haloalkenylsulfonyl group, a C₂₋₆alkynylsulfonyl group, a C₃₋₆cycloalkylsulfonyl group, a C₁₋₆haloalkylsulfonyl group, a C₆₋₁₀arylsulfonyl group, a C₇₋₁₂aralkylsulfonyl group or alternatively
each of R¹⁵ and R¹⁶ is same or differently a group represented by formula: 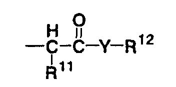 (wherein each of R¹¹ and R¹² has the same meaning as mentioned above)),
R⁶ is hydrogen or a C₁₋₆alkyl group, and m represents 0 or 1,
R⁷ is hydrogen, a C₁₋₆alkyl group, a C₁₋₆haloalkyl group, a C₂₋₆alkenyl group, a C₂₋₆alkynyl group, a C₁₋₆alkoxy-C₁₋₄alkyl group, a C₁₋₇alkanoyl group, a C₇₋₁₁arylcarbonyl group, a C₂₋₇alkoxycarbonyl group, a C₃₋₇alkenyloxycarbonyl group, a C₃₋₇alkynyloxycarbonyl group, a C₄₋₇cycloalkyloxycarbonyl group, a C₂₋₇alkoxycarbonyl-C₁₋₄alkyl group, a C₃₋₇alkenyloxycarbonyl-C₁₋₄alkyl group, a C₃₋₇haloalkenyoxycarbonyl-C₁₋₄alkyl group or a C₃₋₇alkynyloxycarbonyl-C₁₋₄alkyl group,
R⁸ is hydrogen, halogen, a C₁₋₆alkyl group, a C₁₋₆alkoxy group, a C₁₋₆haloalkoxy group, a C₂₋₆alkenyloxy group, a C₂₋₆alkynyloxy group, a C₁₋₆alkylthio group, a C₁₋₆haloalkylthio group, a C₂₋₆alkenylthio group, a C₂₋₆alkynylthio group, a C₁₋₆haloalkyl group, a C₁₋₆alkoxy -C₁₋₄alkyl group, a C₁₋₆alkylthio-C₁₋₄alkyl group or a C₁₋₆alkylsulfonyl-C₁₋₄alkyl group,
R⁹ is hydrogen or a C₁₋₆alkyl group,
R¹⁰ is a C₁₋₆alkyl group,
Z is oxygen atom or sulfur atom)].

2. A pyrimidine derivative or a salt thereof as claimed in claim 1, which is represented by formula (Ia): 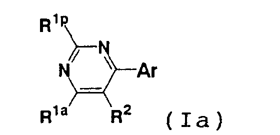 (wherein R^{1p}, R² and Ar have each the same meaning as described above, and R^{1a} is a C₁₋₆alkyl group which is substituted by the substituent or the substituents described above).

3. A pyrimidine derivative or a salt thereof as claimed in claim 1, which is represented by formula (Ia): 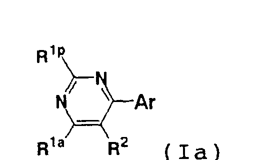 (wherein R^{1p}, R² and Ar have each the same meaning as described above, and R^{1a} is a C₁₋₆alkyl group).

4. A pyrimidine derivative or a salt thereof as claimed in any one of claims 1 to 3, wherein Ar is the group represented by formula Ar-1 in the formula (I).

5. A pyrimidine derivative or a salt thereof as claimed in any one of claims 1 to 3, wherein Ar is the group represented by formula Ar-2 in the formula (I).

6. A pyrimidine derivative or a salt thereof as claimed in any one of claims 1 to 3, wherein Ar is the group represented by formula Ar-3 in the formula (I).

7. A pyrimidine derivative or a salt thereof as claimed in any one of claims 1 to 3, wherein Ar is the group represented by formula Ar-4 in the formula (I).

8. A pyrimidine derivative or a salt thereof as claimed in any one of claims 1 to 3, wherein Ar is the group represented by formula Ar-5 in the formula (I).

9. A pyrimidine derivatives or a salt thereof as claimed in any one of claims 1 to 3, wherein Ar is the group represented by formula Ar-6 in the formula (I).

10. A herbicide which comprises a pyrimidine derivative or a salt thereof as claimed in any one of claims 1 to 9.
